# EUROPEAN PATENT SPECIFICATION

(11) **EP 1 226 266 B1**
(45) Date of publication and mention of the grant of the patent: **18.04.2007**
(21) Application number: 00979601.2
(22) Date of filing: 03.11.2000
(51) Int. Cl.: C12P 17/18, C12P 35/00, C12P 37/00, C12N 9/12, C12N 9/14, C07K 14/385, C12N 15/31

(54) **METHOD FOR ENHANCING SECRETION OF BETA-LACTAM COMPOUNDS**
VERFAHREN ZUR ERHÖHUNG DER AUSSCHEIDUNG VON BETA-LACTAM VERBINDUNGEN
PROCEDE PERMETTANT D'AMELIORER LA SECRETION DE COMPOSES A BASE DE $g(b)-LACTAME

(30) Priority: 03.11.1999 EP 99203693; 03.11.1999 EP 99203685; 03.11.1999 EP 99203687; 03.11.1999 EP 99203690; 03.11.1999 EP 99203684; 03.11.1999 EP 99203689; 03.11.1999 EP 99203694; 03.11.1999 EP 99203688; 03.11.1999 EP 99203695; 03.11.1999 EP 99203696; 03.11.1999 EP 99203691; 03.11.1999 EP 99203692
(43) Date of publication of application: 31.07.2002
(73) Proprietor: DSM IP Assets B.V., 6411 TE Heerlen (NL)
(72) Inventor: BERG, VAN DEN, Marco, Alexander, NL-2565 SH Den Haag (NL); BOVENBERG, Roelof, Ary, Lans, NL-3062 DA Rotterdam (NL); DRIESSEN, Arnold, Jacob, Mattieu, NL-9727 DA Groningen (NL); KONINGS, Wilhelmus, Nicolaas, NL-9752 CJ Haren (NL); SCHUURS, Theo, Auke, NL-9713 HW Groningen (NL); NIEBOER, Maarten, NL-2645 GZ Delfgauw (NL); WESTERLAKEN, Ilja, NL-3032 GA Rotterdam (NL)
(74) Representative: Elkenbracht, Johan Christiaan
(86) International application number: PCT/EP2000/011489
(87) International publication number: WO 2001/032904

(56) References cited:
- TAUCH A. ET AL.: "The tetAB genes of the Corynebacterium striatum R-plasmid pTP10 encode an ABC transporter and confer tetracycline, oxytetracycline and oxacillin resistance in Corynebacterium glutamicum" FEMS MICROBIOLOGY LETTERS, vol. 173, 1 April 1999 (1999-04-01), pages 203-209, XP000926286 AMSTERDAM NL
- NAKAUNE RYOJI ET AL: "A novel ATP-binding cassette transporter involved in multidrug resistance in the phytopathogenic fungus Penicillium digitatum." APPLIED AND ENVIRONMENTAL MICROBIOLOGY, vol. 64, no. 10, October 1998 (1998-10), pages 3983-3988, XP002169426 ISSN: 0099-2240
- BOZDECH ZBYNEK ET AL: "Cloning and sequence analysis of a novel member of the ATP-binding cassette (ABC) protein gene family from Plasmodium falciparum." MOLECULAR AND BIOCHEMICAL PARASITOLOGY, vol. 81, no. 1, 1996, pages 41-51, XP002169427 ISSN: 0166-6851
- DE WAARD MAARTEN A: "Significance of ABC transporters in fungicide sensitivity and resistance." PESTICIDE SCIENCE, vol. 51, no. 3, November 1997 (1997-11), pages 271-275, XP000926418 ISSN: 0031-613X
- GUILFOILE P G ET AL: "A BACTERIAL ANALOG OF THE MDR GENE OF MAMMALIAN TUMOR CELLS IS PRESENT IN STREPTOMYCES-PEUCETIUS THE PRODUCER OF DAUNORUBICIN AND DOXORUBICIN" PROCEEDINGS OF THE NATIONAL ACADEMY OF SCIENCES OF THE UNITED STATES, vol. 88, no. 19, 1991, pages 8553-8557, XP000993100 1991 ISSN: 0027-8424
- DATABASE WPI Section Ch, Week 199954 Derwent Publications Ltd., London, GB; Class B04, AN 1999-626936 XP002169428 & JP 11 276172 A (TANABE SEIYAKU CO), 12 October 1999 (1999-10-12)
- KAMP VAN DE M ET AL: "COMPARTMENTALIZATION AND TRANSPORT IN BETA-LACTAM ANTIBIOTIC BIOSYNTHESIS BY FILAMENTOUS FUNGI" ANTONIE VAN LEEUWENHOEK,NL,DORDRECHT, vol. 75, no. 1/02, 1999, pages 41-78, XP000853787
- ANDRADE, A.C. ET AL.: "The role of ABC transporters from Aspergillus nidulans in protection against cytotoxic agents and in antibiotic production" MOLECULAR AND GENERAL GENETICS., vol. 263, July 2000 (2000-07), pages 966-977, XP000992817 SPRINGER VERLAG, BERLIN., DE ISSN: 0026-8925

## Description

Historically, β-lactam antibiotics constitute the most important group of antibiotic compounds, having a long and successful record of clinical use. The prominent members among this group are the penicillins and cephalosporins. These compounds are naturally produced by filamentous fungi, such as *Penicillium chrysogenum* and *Acremonium chrysogenum,* and by filamentous bacteria.

As a result of classical strain improvement techniques, the production levels of these antibiotics in *P. chrysogenum* and *A. chrysogenum* have increased dramatically over the past decades. Increased understanding of the biosynthetic pathways leading to penicillins and cephalosporins has prompted the advent of recombinant DNA technology, providing new tools for the improvement of β-lactam production strains and for the in vivo derivatisation of β-lactam compounds.

Most enzymes involved in β-lactam biosynthesis have been identified (Ingolia and Queener, Med. Res. Rev. **9**: 245-264, 1989), and their corresponding genes have been cloned (Aharonowitz, et al., (Ann. Rev. Microbiol. **46**: 461-495, 1992).

The first two steps in the biosynthesis of penicillin in *P. chrysogenum* are the condensation of the three amino acids L-5-amino-5-carboxypentanoic acid (L-α-aminoadipic acid) (A), L-cysteine (C) and L-valine (V) into the tripeptide LLD-ACV, followed by cyclisation of this tripeptide to form isopenicillin N. The isopenicillin N intermediate compound contains the β-lactam structure characteristic of penicillin. These first two steps in the biosynthesis of penicillins are common in penicillin-producing, cephamycin-producing and cephalosporin-producing fungi and bacteria.

The third synthesis step involves the exchange of the hydrophilic α-aminoadipic acid side chain derived from L-5-amino-5-carboxypentanoic acid with a hydrophobic side chain, via the action of the enzyme acyltransferase (AT). The enzymatic exchange reaction mediated by AT takes place inside a cellular organelle, the microbody, as has been described in EP-A-0448180.

Industrial fermented penicillins like-penicillin V and penicillin G, are produced by adding to the culture broth phenoxyacetic acid or phenylacetic acid, respectively, during fermentation of *P. chrysogenum,* thereby replacing the side chains of the natural β-lactams with phenoxyacetic acid or phenylacetic acid. In order to replace the α-aminoadipic acid side chain in the acyltransferase-catalysed reaction, the carboxylic acid group of the new side chain first must be activated, as the AT enzyme requires the side chain in an activated form. The activated form is a coenzyme A (CoA) derivative, synthesised by a CoA ligase.

Two CoA ligases have been cloned from *P. chrysogenum.* The gene encoding an acetyl-coenzyme A synthetase has been characterised by Van Hartingsveldt et al. (WO92/07079) Gouka et al. (Appl. Microbiol. Biotechnol., **38**:514-519 (1993)) and Martinez-Blanco et al. (Gene, **130**:265-270 (1993); J. Biol. Chem. **267**:5474-5481 (1992)). The enzyme not only accepts acetic acid but also phenylacetic acid as a substrate in the synthesis of the corresponding acyl-coenzyme A ester, although the specificity for phenylacetic acid is very low as compared to the specificity for acetic acid. More recently, a gene encoding a homologue of the *Pseudomonas putida* phenylacetic acid CoA ligase has been cloned; the *pcl* gene (WO 97/02349). The protein was shown to be active with both phenylacetic and phenoxyacetic acid.

In novel fermentation processes for the production of cephalosporin intermediates, *P. chrysogenum* strains carrying heterologous cephalosporin biosynthetic genes, such as the expandase gene of *S. clavuligerus* or the expandase/hydroxylase gene of *A. chrysogenum,* are used for the production of the corresponding adipoyl-cephalosporins by feeding adipic acid to these recombinant strains. Cephalosporin intermediates produced in this fashion include 7-aminodeacetoxycephalosporanic acid (7-ADCA), 7-aminodeacetylcephalosporanic acid (7-ADAC) and 7-aminocephalosporanic acid (7-ACA). Subsequent removal of the adipoyl side chain from the adipoyl-cephalosporins can be achieved by using an amidase (EP 532341 and Crawford et al., Bio/Technology **13**: 58-62 (1995)).

During the latter stages of industrial penicillin fermentation processes extremely high β-lactam titers are found, using *Penicillium chrysogenum* as the production host (estimated to be 120 mM by Hillenga (1999) PhD thesis, University of Groningen, The Netherlands). From literature it is known that the ergosterol content of *P. chrysogenum* plasma membranes is around 50% (Hillenga, 1999). At this concentration the membrane is almost impermeable for passive diffusion of penicillins. Therefore, it remains unclear how the fungus can export such an amount of β-lactam antibiotics. Especially at the end of fermentation this must be an efficient system to export against a high external concentration.

Recently, organic cation/peptide transporters were suggested to be involved in the transport of β-lactam antibiotics in humans (Ganapathy et al., J. Biol. Chem (2000) 275:1699-1707). Until now no such proteins are reported in filamentous fungi.

Industrial strains of the filamentous fungus *Penicillium chrysogenum* have been selected for their capacity to produce and secrete large quantities of a variety of β-lactam compounds into culture medium. Various lines of evidence indicate that β-lactam secretion is an active process, possibly via transport proteins. During the latter stages of such fermentation processes extremely high β-lactam titers are found in fermentations on an industrial scale. However, despite traditional genetic selection of fungal strains for β-tactam output and evidence of how β-lactam compounds are secreted, micro-organisms have yet to be optimised for maximal secretion of β-lactam compounds. Moreover, the transport process might be optimised for each organism and each antibiotic. For example, the production rate of heterologous cephalosporins produced in *P. chrysogenum* will be limited due to the fact that the penicillin transporter will have a low affinity for the product. This could be overcome either by cloning one or more homologue β-lactam transporters from natural cephalosporin producers like *A. chrysogenum* with the aid of the transporter subject of this invention or, alternatively, by mutagenesing the penicillin transporter according to the present invention into a derivative more apt to transporting cephalosporins.

EP-A-0908516 discloses a *Staphylococcus* ATP-binding cassette (ABC) transporter polypeptide, an ABC transporter polynucleotide and materials and methods for their production. The transporter polypeptide of EP-A-0908516 may be used to determine the presence of drug resistant bacteria in a mammalian host. The polypeptide of EP-A-0908516 may be obtained from other organisms, but has no bearing on transport of β-lactam compounds.

Certain transporters have been described in eukaryotic yeast and moulds, but none appear related to improving β-lactam output from industrial strains of fungi. For example, a histidine transporter (Tanaka and Fink, Gene **38:** 205-214 (1985)) and a proline transporter (Vandenbol et al., Gene **83:** 153-159 (1989)) have been identified in *Saccharomyces cerevisiae.* Over-expression of the yeast multi-drug-resistance gene CDR1 in *Candida albicans* can confer resistance to cycloheximide, chloramphenicol, and miconazole (Prasad et al., Curr. Genet., **27** (4): 320-329 (1995)). An ATP-binding cassette transporter p-glycoprotein encoded by a *Penicillium* multi-drug resistance gene is over-expressed in strains of *Penicillium digitatum* resistant to fungicide, suggesting the toxicant efflux system of the fungus is directly involved with fungicide resistance (Nakaune et al., Appl. Environ. Microbiol. **64**:3983-3988 (1998)). However, such protein carriers are not associated with β-lactam secretion from fungi.

Thus, a need exists for improving the secreted amounts of β-lactam compounds recoverable from industrial strains of micro-organisms. The invention solves the above-mentioned problem by manipulation of the ABC-transport protein system, providing a means for enhancing β-lactam secretion from the micro-organisms in which these compounds are or can be synthesized.

Present invention therefore provides a method for producing β-lactam compounds comprising the step of enhancing ABC-transporter activity of a micro-organism and subsequently culturing said micro-organism under conditions allowing the production of said β-lactam compounds. In another embodiment of the present invention a method is provided for enhancing the secretion of a β-lactam compound from a micro-organism comprising the step of enhancing an ABC transporter activity of said micro-organism.

The present invention further provides an isolated polynucleotide encoding an ABC transporter polypeptide selected from the group consisting of SEQ ID NO. 10, 13, 16, 20, 2,3, 28, 31, 36, 39, and 48 as incorporated in the present application, or a sequence which shows a percentage of similarity of at least 95% with these sequences.

The present invention also provides for isolated ABC transporters encoded by an ABC transporter gene comprising the nucleotide sequence according to SEQ ID NO. selected from the group consisting of SEQ ID NO. 7, 8, 9, 11, 12, 14, 15, 18, 19, 21, 22, 24, 25, 26, 27, 29, 30, 34, 35, 37, 38, 40, 46 and 47 or a polynucleotide sequence which shows a similarity of at least 95% with these nucleotide sequences.

An ABC-transport protein utilises ATP to drive transport processes of a diverse range of substrates including various antibiotics, oligopeptides, polysaccharides and unrelated toxic drugs (reviewed by Schneider *et al* (Schneider, E. and Hunke, S. FEMS Microbiol. Rev. **22:** 1-20, 1998); Méndez *et al* (Méndez, C. and Salas, J.A. FEMS Microbiol. Lett. **158 :** 1-8, 1998) ; van Veen *et al* (Veen, H.W. van and Konings, W.N. Biochim.Biophys. Acta **1365:** 31-36, 1998)).

An embodiment of the invention provides a method for enhancing the secretion of a β-lactam compound from a micro-organism which method comprises the step of enhancing an ABC transporter activity as defined on page 5 of said micro-organism. Secretion according to the invention comprises any step involved in transport of β-lactam compounds or intermediates thereof occurring inside the micro-organism and from inside to outside the micro-organism. β-lactam compounds according to the invention comprise Penicillins, Cephalosporins, Amoxycillins, Calvulanic acid and intermediates thereof such as PenV, PenG, 6-APA, 7-ADCA, 7-ACA, 7-ADCA.

In a preferred embodiment of the invention, the step of enhancing the ABC transporter acitivity comprises the step of transforming said micro-organism with at least one vector comprising a polynucleotide encoding a homologous or heterologous ABC transporter protein as defined on page 5. According to another embodiment of the invention the method comprises the use of polynucleotides encoding an ABC transporter polypeptide selected from the group consisting of SEQ ID NO. 10 13, 16, 20, 23, 28, 31, 36, 39, and 48 or a polypeptide showing at least 60% similarity with these polypeptide sequences. The micro-organism useful as a source for a polynucleotide according to the present invention, may be micro-organisms capable of producing β-lactam compounds either naturally or upon induction or mutation according to methods known in the art. In a preferred embodiment of the invention, micro-organisms are β-lactam-producing fungi, filamentous fungi or filamentous bacteria, more preferably *Penicillium, Acremonium, Saccharomyces, Hansenula, Pichia* and *Aspergillus* strains. Most preferably *Penicillium chrysogenum, Saccharomyces cerevisiae, Hansenula polymorpha, Pichia pastoris, Acremonium chrysogenum* or *Aspergillus nidulans,* or Actinomycetes like *Streptomyces clavuligerus, Nocardia lactamdurans, Flavobacterium sp.*

The isolated polynucleotide according to the invention comprises a nucleotide sequence encoding an ABC transporter polypeptide may suitably be of genomic of of cDNA origin, for instance obtained by preparing a genomic or a cDNA library and screening for DNA sequences coding for all or part of the polypeptide by hybridisation using at least part of the novel nucleotide sequence as disclosed herein as a probe applying standard techniques (cf. Sambrook *et al.,* Molecular Cloning: A Laboratory Manual, 2nd. Ed., Cold Spring Harbor Laboratory, Cold Spring Harbor, New York, 1989). The isolated polynucleotides of the invention comprising the gene or cDNA encoding a ABC transporter polypeptide may also be prepared by polymerase chain reaction using specific primers advantageously primer derived from the nucleotide sequence as disclosed herein. To this end use can be made, for instance of the method described in US 4,683,202 or by Saiki *et al.* (Science, **239** (1988): 487-491).

The isolated polynucleotide according to the invention may be selected from the group consisting of polynucleotides as defined on page 5.

A homologous ABC transporter polynucleotide comprises a polynucleotide that is obtained from a micro-organisms belonging to the same species as the micro -organism to which said polynucleotide is transformed, whereas a heterologous ABC transporter comprises a polynucleotide that is obtained from a micro-organisms belonging to another species as the micro -organism to which said polynucleotide is transformed.

Polynucleotide and polypeptide sequences that are substantially homologous to the polynucleotide or polypeptide sequences can be determined using the method described in experiment 2. In this experiment it is described how the percentage of sequence identity and percentage of similarity can be determined. Basically, a comparison of the sequences of two polynucleotides (or more than two) may be restricted to a "comparison window" in order to determine sequence similarity in a local region of the two polynucleotides. A "comparison window" is defined to be a continuous series of at least 20 nucleotides in a first polynucleotide sequence, which when aligned with a reference sequence of at least 20 contiguous nucleotides, enables a comparison of the first polynucleotide and reference sequences. The segment of contiguous nucleotides in the "comparison window" of the first polynucleotide may comprise additions and/or deletions such as, for example, gaps, of up to 20% of the reference sequence. Local homology or sequence identity within the "comparison window" of the first polynucleotide and reference polynucleotide may be achieved via alignment of the sequences according to the algorithm of Smith and Waterman (Appl. Math. 2:482 (1981), or by a another suitable alignment method or algorithm (Needleman and Wunsch, J. Mol. Bio. 48:443 (1970); Pearson and Lipman, Proc. Natl. Acad. Sci. (USA) 85:2444 (1988)).

Sequence identity is established when the first polynucleotide and reference polynucleotide exhibit complete sequence similarity on a nucleotide by nucleotide basis over the span of the "comparison window". However, polynucleotides having substantial identity with a reference polynucleotide can be contemplated, wherein a polynucleotide having substantial identity with the reference polynucleotide exhibits the same function as the reference polynucleotide. By "substantial identity" is meant that a polynucleotide has nearly identical nucleic acid bases in a "comparison window" located in positions corresponding to those of the reference polynucleotide. The number of matched base positions divided by the number of total positions compared, multiplied by 100 percent, yields what is known as the percentage sequence identity (also known as percentage sequence homology) between compared polynucleotides. A reference sequence having a "comparison window" of at least 20 nucleotides (preferably 20-50 nucleotides) is contemplated. The reference sequence is obtained via a PCR reaction on a homologous boxes in the so-called ATP Binding Cassette (ABC-) proteins: the Walker A and B domains. Although the similarities of these sequences to known ABC-transporters can be quite high, this is concentrated in these conserved regions. The variability in sequence identity within the known sequences is due to the existence of two sequence subclasses, determined via clustal sequence alignment. The above outlined procedure is applicable to all the sequences which are part of this invention.

For the purposes of this invention, the BlastP method and Blast N method (Version 2.0.6, release date September 1998, running under the software of Biomax Bioinformatics GmbH, Martinsried, GE) is preferred for determining the degree of identity between two polypeptide sequences or polynucleotide sequences, using the following parameters:

| | |
|---|---|
| Expectation value (E) | : 10 |
| Cost to open a gap | : 11 |
| Cost to extend a gap | : 1 |
| Matrix | : Blosum62 |

Polynucleotide and polypeptide sequences which are substantially homologous may show a percentage of similarity of at least 60% , preferably at least 80 %, more preferably 90 % ,and more preferred at least 95%, 96%, 97%, 98%, 99% with the polynucleotide or polypeptide sequence represented by SEQ ID NO. 7-48 as incorporated in the present application.

Functional equivalents of the ABC transporter protein include nucleotide sequences encoding a ABC transporter polypeptide which may have one or more amino acid substitutions, deletions or additions as compared to their natural counterparts and still resulting in a functional ABC transporter polypeptide.

Fragments of the ABC transporter protein may include biologically active fragments of the polypeptide comprising amino acid sequences sufficiently identical to or derived from the amino acid sequence of the ABC transporter protein, which include fewer amino acids than the full length protein. Typically, biologically active fragments comprise a domain or motif with at least one activity of the ABC transporter protein. Amino acid substitutions may encompass changes made on the basis of similarity in polarity, charge, solubility, hydrophobicity, hydrophilicity, and/or the amphipathic nature of the residues involved. These changes are preferably of a minor nature (that is conservative amino acid substitutions that do not adversely affect the folding or activity of the protein), small deletions (typically of one to about 30 amino acids), small amino- or carboxyl-terminal extensions (such as an amino-terminal methionine residue), a small linker peptide of up to about 20-25 residues, or a small extension that facilitates purification (such as a poly-histidine tract, an antigenic epitope or a binding domain). See in general Ford *et al*. (Protein Expression and Purification, **2** (1991), 95-107,).
Examples of conservative substitutions are substitutions within the respective groups of
◆ basic amino acids (such as arginine, lysine, histidine),
◆ acidic amino acids (such as glutamic acid and aspartic acid),
◆ polar amino acids (such as glutamine and asparagine),
◆ hydrophobic amino acids (such as leucine, isoleucine, valine),
◆ aromatic amino acids (such as phenylalanine, tryptophan, tyrosine) and
◆ small amino acids (such as glycine, alanine, serine, threonine, methionine).

It will be apparent to persons skilled in the art that such substitutions can be made outside the regions critical to the function of the molecule and still have to result in an active polypeptide. A test which can be applied to determine the ABC transporter activity of a polypeptide is described in example 12.
Amino acids essential to the activity of the ABC transporter polypeptide of the invention, and therefore preferably not subject to substitution, may be identified according to procedures known in the art, such as site-directed mutagenesis or alanine-scanning mutagenesis (Cunningham and Wells, Science, **244** (1989): 1081-1085). In the latter technique mutations are introduced at every residue in the molecule, and the resultant mutant molecules are tested for biological activity to identify amino acid residues that are critical to the activity of the molecule. Sites of ligand-receptor interaction can also be determined by analysis of crystal structure as determined by such techniques as nuclear magnetic resonance, crystallography or photo-affinity labelling. See, for example, de Vos *et al.* (Science, **255** (1992): 306-312), Smith *et al.* (J. Mol. Biol., **224** (1992): 899-904) and Wlodaver *et al.* (FEBS Lett., **309** (1992): 59-64).

Another embodiment of the invention provides for a micro-organism transformed with at least one vector comprising at least one polynucleotide encoding a microbial ABC-transporter as defined on page 5. Said polynucleotide might be operably linked to a regulatory sequence, such as a heterologous or homologous promoter, or a constitutive or regulatable promoter. The term "operably linked" indicates that the sequence encoding at least part of a microbial ABC transporter and the regulatory sequence are arranged so that they function in concert for their intended purposes, e.g. transcription initiates in a promoter and proceeds through the DNA sequence coding for the polypeptide.

An overview of fungal promoters can be found in, for instance, Applied Molecular Genetics of filamentous fungi (Kinghorn, Turner (eds.), Blackie, Glasgow, UK, 1992). Suitable promoters for use in filamentous fungus host cells are, for instance, the *ADH3* promoter (McKnight *et al.,* EMBO J., **4** (1985): 2093-2099,) or the *tpiA* promoter. Examples of other useful promoters are those derived from the gene encoding *A. oryzae* TAKA amylase, *Rhizomucor miehei* aspartic proteinase, *A. niger* neutral β-amylase, *A. niger* acid stable a-amylase, *A. niger* or *A. awamori* glucoamylase (gluA), *Rhizomucor miehei* lipase, *A. oryzae* alkaline protease, *A. oryzae* triose phosphate isomerase, *A. nidulans* acetamidase, *P. chrysogenum* ACV synthetase, *P. chrysogenum* isopenicillin N synthase, *P. chrysogenum* acyltransferase, *P. chrysogenum* phosphoglycerate kinase, *P. chrysogenum* gene Y. Preferred are the A. *niger* glucoamylase or *P. chrysogenum* promoters.

The DNA sequence of the invention encoding the ABC-transporter polypeptide according to the invention may also, if necessary, be operably connected to a suitable terminator.

The vector may conveniently be subjected to recombinant DNA procedures, and the choice of the vector will often depend on the host cell into which it is to be introduced. Thus, the vector may be an autonomously replicating vector, i.e. a vector which exists as an extra-chromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

The recombinant vector may also comprise a selectable marker, e.g. a gene the product of which complements a defect in the host cell, or one which confers resistance to a drug. Examples of the latter are phleomycin or hygromycin. For filamentous fungi, additional selectable markers include *amdS, pyrG, argB, niaD, facA,* and sC (Applied Molecular Genetics of Filamentous fungi (*ibid.*)*,* Biotechnology of Filamentous fungi, Finkelstein, Ball (eds.), Butterworth-Heinemann, Boston, 1992).

It is also possible to introduce the vector comprising the DNA sequence of the invention encoding a ABC transporter polypeptide into a host cell on a DNA fragment separate from the vector comprising a selectable marker, by a so-called co-transformation process.

In another embodiment the invention relates to the transformed host cell comprising the vector that contain the polynucleotide according to SEQ ID NO selected from the group consisting of: 7, 8, 9, 11, 12, 14, 15, 18, 19, 21, 22, 24, 25, 26, 29, 30, 34, 35, 37, 38, 46 and 47 or a polynucleotide having at least 95% similarity thereto.

A preferred embodiment of the invention is a process for enhancing ABC transporter activity comprising expression from the host cell under conditions sufficient for the production of the polypeptide according to SEQ ID. 10, 13, 16, 20, 23, 28, 31, 36, 39, or 48 or a polypeptide having at least 60 % similarity thereto.

Another embodiment is the use of an isolated polynucleotide according to the invention or a vector comprising said polynucleotide in a method for the production of β-lactam compounds in a micro-organism.

Another embodiment of the invention provides a method for producing beta-lactam compounds in a miro-organism comprising the step of enhancing ABC-transporter activity of said micro-organism and subsequently culturing said micro-organism under conditions allowing the production of said β-lactam compounds.

The invention also provides an isolated ABC transporter encoded by an ABC transporter gene comprising at least part of the nucleotide sequence as defined on page 5.

The invention also provides a method for producing a β-lactam compound from a cultured micro-organism comprising transforming a β-lactam-producing micro-organism with an isolated polynucleotide encoding the microbial ABC-transporter according to the invention under the control of a suitable promoter, and subsequent culturing the β-lactam-producing micro-organism under conditions which permit
(i) synthesis of a β-lactam compound,
(ii) expression of the isolated polynucleotide encoding the microbial ABC-transporter under the control of a suitable promoter, and
(iii) ABC-transporter-mediated secretion of the β-lactam compound by the micro-organism into culture media, and thereafter, recovery of the secreted β-lactam compound from the culture media.

Another embodiment is the use of an isolated polynucleotide encoding the microbial ABC-transport protein according to the invention under the control of a suitable promoter in the production of a secreted microbial β-lactam compound.

### EXAMPLE 1

### INTRACELLULAR ACCUMULATION OF β-LACTAM ANTIBIOTICS AFTER INHIBITION OF ABC-TRANSPORTERS

*P. chrysogenum* was cultivated in 100 ml shake flasks with 25 ml of defined medium containing lactose as the carbon source to ensure non-repressing conditions for penicillin production (280 rpm at 25 degrees Celsius).

**Table I: Intracellular accumulation β-lactam antibiotics as a result of the inhibition of ABC transporters.**

| | Inhibitor¹ | Intracellular penicillinG conc.² | Intracellular isopenicillinN conc.² |
|---|---|---|---|
| Control without phenyl acetic acid | - | 0 | 5.2 |
| Control with phenyl acetic acid | - | 0.4 | 0.1 |
| Addition of inhibitor at the start of penicillin production | BeF | 0.4 | 0.1 |
| | Verapamil | 1.2 | 0 |
| | Cyclosporin A | 0.6 | 0 |
| | | | |
| Control with 100mM penicillinG incubation | - | 1.8 | 0.1 |
| Addition of inhibitor at the end of the fermentation, after 1 hour followed by the 100mM penicillinG incubation | BeF | 3.1 | 0.1 |
| | Verapamil | 5.3 | 0 |
| | Cyclosporin A | 6.1 | 0 |

| | | | |
|---|---|---|---|
| ¹ Berilliumfluoride was added in the a final concentration of 400µM BeSO₄ and 2mM NaF; together with 400µM ATP, 4mM MgSO₄ and 0.1 mM EDTA. Verapamil and cyclosporinA were added in a final concentration of 20µM and 2µM, respectively; together with 40µM vanadate, 4 mM MgCl₂ and 4mM ATP. ² In arbritary units | | | |

The precursor for penicillinG, phenyl acetic acid, was added as potassium salt 48 hours after inoculation. The ABC transporter inhibitors were added either at the start of penicillin production (48 hours) or at the end of the fermentation (144 hours). In the latter case 100 mM penicillinG was added after the cultures were incubated for 1 hour with the inhibitors. Exactly 1 hour later all cultures were harvested by filtration and washed three times with cold physiological salt (0.9%).
These washing steps are very critical; they must be done thoroughly. The mycelial pellets were frozen in liquid nitrogen, freeze-dried and the internal penicillinG concentration was determined by NMR.

There was a clear increase in intracellular penicillinG when the organism was incubated with ABC transporter inhibitors (see Table I). The best results were obtained with verapamil and cyclosporinA, where a two-to-three fold increase in intracellular penicillinG was found.

This is a clear indication that ABC transporters are directly involved in the export of β-lactam antibiotics as penicillinG.

### EXAMPLE 2

### DESCRIPTION OF THE METHODS USED IN CLONING ABC TRANSPORTER FRAGMENTS

### PCR oligonucleotides

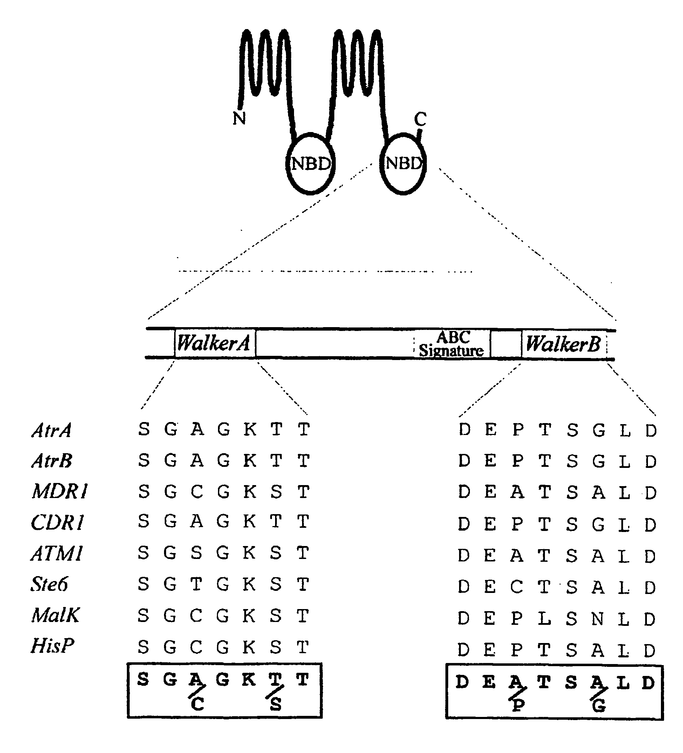

Degenerate oligonucleotides complementary to the rather conserved C-terminal *WalkerA* and *WalkerB* motifs (present in the nucleotide binding domain of ABC-transporters) were designed (see Figure 1). As a result, the forward primers (complementary to the *WalkerA* motif; SGA/CGKT/ST), 5'-TCI GGI G/TG/CI GGI AAA/G A/TCI AC-3' (SEQ ID NO. 3) and 5'-TCI GGI G/TG/CI GGI AAA/G AGC/T AC-3' (SEQ ID NO. 4) and the reverse primers (complementary to the *WalkerB* motif; DEA/PTSA/GLD), 5'-TCI AA/GI G/CCI GAI GTI GG/CC/T TCA/G TC-3' (SEQ ID NO. 5) and 5'-TCI AA/GI G/CCA/G CTI GTI GG/CC/T TCA/G TC-3' (SEQ ID NO. 6)were applied.

### RT-PCR

Total RNA was isolated from a high penicillin producing strain of *P. chrysogenum* (e.g. Panlabs P2) under penicillin producing conditions to enlarge the presence of putative penicillin transporter transcripts in the total RNA pool. RT-PCR was performed on these total RNA isolates using the above mentioned degenerate primers. cDNA was synthesized with AMV reverse transcriptase (Boehringer Mannheim) using the reverse (*WalkerB*) primers (incubation for 1 hr at 50°C). This cDNA material was subsequently used in a PCR procedure using the following program. As the standard PCR programs (with one standard cycle) gave no satisfactory results, we applied a special "staircase" program to obtain good PCR products A 'hot start' was made for 3 min at 95°C after which 10 cycles of 0.15 min at 95°C, 0.30 min at 55°C and 0.40 min at 72°C were done. This was followed by 20 cycles in which the last step at 72°C was extended each cycle with 20 sec., followed by 5 min at 72°C.

### Cloning of PCR fragments

PCR reaction products were analyzed by electrophoresis on agarose gels containing ethidium bromide. PCR products of proper size (about 400 bp) were isolated from gel, purified and cloned in the pGEM-T Easy vector (Promega). This vector contains 3' protruding thymidine residues which makes ligation with PCR-fragments that contain 3' protruding adenosine residues (when generated with Taq-polymerase, for example), possible.

### AFLP analysis

PCR techniques as described above restrict ones choice to one set of genes. As another approach to obtain genes involved in the export of β-lactams a DNA fingerprinting technique called AFLP (selective restriction fragment amplification) is employed.

The AFLP technique is based on the selective PCR amplification of restriction fragments from a total digest of genomic DNA.

The technique involves three steps:
(i) restriction of the DNA and ligation of oligonucleotide adapters,
(ii) selective amplification of sets of restriction fragments, and
(iii) gel analysis of the amplified fragments.

PCR amplification of restriction fragments is achieved by using the adapter and restriction site sequence as target sites for primer annealing. The selective amplification is achieved by the use of primers that extend into the restriction fragments, amplifying only those fragments in which the primer extensions match the nucleotides flanking the restriction sites. Using this method, sets of restriction fragments may be visualized by PCR without knowledge of their nucleotide sequence. The method allows the specific co-amplification of high numbers of restriction fragments.

The number of fragments that can be analyzed simultaneously, however, is dependent on the resolution of the detection system. Typically 50-100 restriction fragments are amplified and detected on denaturing polyacrylamide gels.
The AFLP technique provides a novel and very powerful DNA fingerprinting technique for DNAs of any origin or complexity. It can also be used in combination with RNA and thereby applied to pinpoint differences in mRNA levels between two strains or two conditions. This is called: Differential Display. We applied this technique to identify genes which are expressed higher with increased penicillin titers.

### Sequence analysis

After determining the sequences of the PCR-fragments, homology searches and sequence comparisons were executed as following. The terms used herein to describe the sequence relationships between two or more polynucleotides are well known in the art, as exemplified in U.S. Patent Nos. 5,912,120 and 5,914,393.

Polynucleotide sequences discussed herein refer to single or doublestranded polymers of deoxyribonucleotide or ribonucleotide bases as read from their respective 5' to 3'ends. A reference sequence in this regard is defined as a polynucleotide sequence, at least 20 nucleotides in length, used as a basis for a comparison between sequences. The reference sequence comprise a full-length cDNA or gene sequence in a sequence listing, or the reference sequence comprise a portion of a full-length cDNA or gene sequence.

A comparison of the sequences of two polynucleotides (or more than two) may be restricted to a "comparison window" in order to determine sequence similarity in a local region of the two polynucleotides. A "comparison window" is defined to be a continuous series of at least 20 nucleotides in a first polynucleotide sequence, which when aligned with a reference sequence of at least 20 contiguous nucleotides, enables a comparison of the first polynucleotide and reference sequences. The segment of contiguous nucleotides in the "comparison window" of the first polynucleotide may comprise additions and/or deletions such as, for example, gaps, of up to 20% of the reference sequence. Local homology or sequence identity within the "comparison window" of the first polynucleotide and reference polynucleotide may be achieved via alignment of the sequences according to the algorithm of Smith and Waterman (Appl. Math. 2:482 (1981), or by a another suitable alignment method or algorithm (Needleman and Wunsch, J. Mol. Bio. 48:443 (1970); Pearson and Lipman, Proc. Natl. Acad. Sci. (USA) 85:2444 (1988)).

Sequence identity is established when the first polynucleotide and reference polynucleotide exhibit complete sequence similarity on a nucleotide by nucleotide basis over the span of the "comparison window". However, polynucleotides having substantial identity with a reference polynucleotide may be contemplated wherein a polynucleotide having substantial identity with the reference polynucleotide exhibits the same function as the reference polynucleotide. By "substantial identity" is meant that a polynucleotide has nearly identical nucleic acid bases in a "comparison window" located in positions corresponding to those of the reference polynucleotide. The number of matched base positions divided by the number of total positions compared, multiplied by 100 percent, yields what is known as the percentage sequence identity (also known as percentage sequence homology) between compared polynucleotides.

The reference sequence is obtained via a PCR reaction on a homologous boxes in the so-called ATP Binding Cassette (ABC-) proteins: the Walker A and B domains. Although the similarities of these sequences to known ABC-transporters can be quite high, this is concentrated in these conserved regions. The variability in sequence identity within the known sequences is due to the existence of two sequence subclasses, determined via clustal sequence alignment. Yeast ABC-transporters have been analyzed for sequence and function (substrate) relationship to other known ABC-transporters, and classified as MDR-like ABC-transporters and ABC-transporters involved in the secretion of substances (Decottignies and Goffeau, Nature Gen. 15: 137-145 (1998)). The substrate specificity of these proteins is in part conferred to the regions outside the conserved domains.

The above outlined procedure is applicable to all the sequences which are part of this invention.

For the purposes of this invention, the BlastP or BlastN method (Version 2.0.6, release date September 1998, running under the software of Biomax Bioinformatics GmbH, Martinsried, GE) is preferred for determining the degree of identity between two amino acid sequences, using the following parameters:

| | |
|---|---|
| Expectation value (E) : | 10 |
| Cost to open a gap : | 11 |
| Cost to extend a gap : | 1 |
| Matrix : | Blosum62 |

### EXAMPLE 3

### PCR AMPLIFICATION OF ABC TRANSPORTER FRAGMENTS

Degenerate oligonucleotides complementary to the rather conserved *Walker A* and *Walker B* motifs, present in the nucleotide binding domain (NBD) of ABC-transporters, were used in RT-PCR reactions (Reverse Transcriptase Polymerase Chain Reaction) on total RNA isolated from *P. chrysogenum* strains (e.g. Wisconsin 54-1255 and Panlabs P2). To enlarge the presence of putative penicillin transporter transcripts in the total RNA pools, the above mentioned strains were grown under penicillin producing conditions. The RT-PCR resulted in the formation of a DNA fragment of about 400 bp, which is the expected size. The fragment was cloned in the vector pGEM-T Easy (Promega) and 32 individual clones were analyzed. Ten different sequences were obtained (due two multiple clones containing identical sequences). The sequence of these fragments (see SEQ ID NO. 7, 11, 14, 17, 21, 24, 29, 32, 37 and 40) showed high sequence identity to several ABC transporter genes from other organisms.

### EXAMPLE 4

### AFLP ANALYSIS IDENTIFYING ABC TRANSPORTER FRAGMENTS

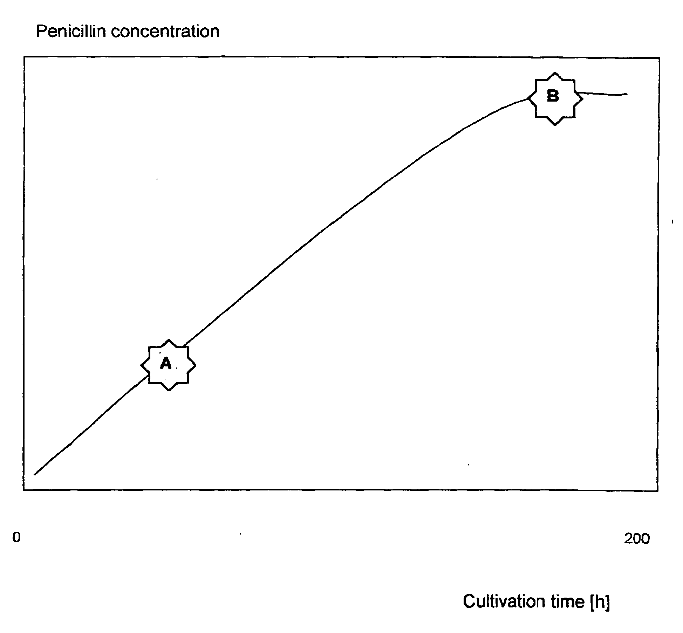

Samples of a β-lactam producing fed-batch culture of *Penicillium chrysogenum* were used to isolate RNA. An AFLP analysis as described above between the samples A and B (see Figure 2) was performed. Two transcripts were found which were approximately 8-10 times increased during the fermentation. Surprisingly, these two polynucleotide sequences (see SEQ ID NO. 43 and 46) showed a high sequence identity to several ABC transporter genes from other organisms.

This indicates that there is need for high ABC transporter activity when the penicillinG concentration is high, which confirms the direct role for these transporters in the export of penicillinG.

### EXAMPLE 5

### NORTHERN ANALYSIS: TRANSCRIPTION OF THE ISOLATED PENICILLIUM RT-PCR FRAGMENTS ENCODING ABC-TRANSPORTERS

The isolated *Penicillium chrysogenum* ABC transporter clones were subjected to an expression study to determine the conditions under which the transporter genes are regulated. Northern analysis was conducted on RNA samples obtained from the organism subjected to two different growth conditions. Total RNA was isolated from a high yielding strain grown on either glucose or under conditions optimal for penicillinV production. RNA electrophoresis, blotting and hybridizations were performed as described by Schuurs *et al.,* 1997 (Genetics 147:589-596)

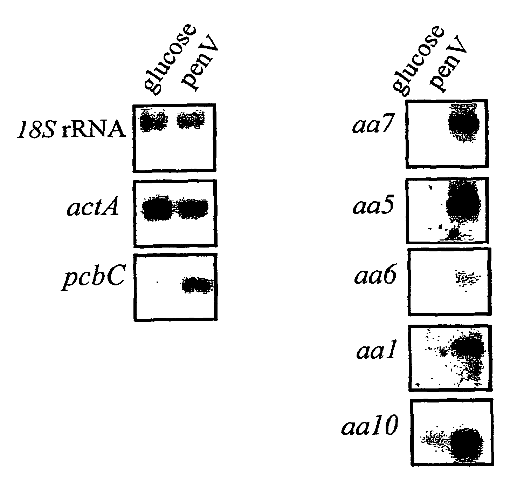

Northern analysis showed a remarkable elevated expression of the 5 tested ABC transporter genes (*aa1, aa5,* aa6, *aa7* and *aa10*) during penicillinV fermentation conditions (see Figure 3). Thus, during penicillin fermentation, *P. chrysogenum* specifically expresses ABC-transporter genes whose protein product is shown to be a transporter of β-tactam.

### EXAMPLE 6

### DNA ARRAY HYBRIDISATION: TRANSCRIPTION OF THE ISOLATED PENICILLIUM RT-PCR FRAGMENTS ENCODING ABC-TRANSPORTERS

All PCR-isolated *Penicillium chrysogenum* ABC transporter clones were subjected to an expression study using a DNA array. Opposite to Northern analysis here the DNA is linked to a filter and hybridized with labeled mRNA. Moreover, we applied the technique in such a way that it is possible to omit the RT-PCR step normally applied in DNA experiments and directly labeled the mRNA.

The filter was prepared by spotting 12µg of denatured pGEM-T Easy vector containing the PCR amplified fragment (as described in example 3) on a Hybond-N⁺ filter (Amersham Pharmacia Biotech); this means that approximately 1µg of each ABC transporter fragment is on the filter.

The RNA samples were obtained from the strain Wisconsin54-1255 subjected to two different growth conditions. Total RNA was isolated from the fungus grown on either glucose or under conditions optimal for penicillinG production. RNA was prepared by mortaring the washed and frozen fungal pellet with trizol. The continuing steps were essentially as described in laboratory manuals (e.g. Sambrook et al (1989) Molecular Cloning: a laboratory manual (2^{nd} ed), Cold Spring Harbor Press, CSH, NY). mRNA was isolated from 3µg of total RNA with the Oligotex mRNA Midi Kit (Qiagen). Prior to hybridization 1µg of mRNA was labeled according to the ECL kit (Pharmacia Amersham Biotech):
- 1 µg/20 µl mRNA was denatured by boiling for 5 minutes in a water bath
- after 5 minutes the mRNA was directly placed on ice for 5 minutes
- 20 µl labeling reagent was added and gently mixed
- 20 µl glutaraldehyde solution was added and mixed
- the mixture was incubated for 15 minutes at 37 °C
The blot was first pre-hybridized for 30-45 minutes with hybridizationbuffer (500 ml ECL hybridizationbuffer+ 25 mg ECL blockingreagent + 14 g NaCL) at 42 °C in a tube in a rotisserie oven. The labeled probe was added to 50 ml hybridizationbuffer after pre-hybridization. Hybridization was carried out overnight at 42 °C in a tube in a rotisserie oven. Afterwards, the blot was washed 2 times for 20 minutes at 42 °C with primary washbuffer (6 M ureum, 0.4% SDS, 0.2 x SSC) in a tube in a rotisserie oven and washed 2 times for 5 minutes at roomtemperature with secondary washbuffer (2 x SSC) in a container with gentle agitation. Detection was started by mixing 10 ml ECL detection reagent 1 with 10 ml ECL detection reagent 2. The washed blots were placed in the detection mixture for one minute, wrapped in plastic bags and placed in a cassette with a photographic film.

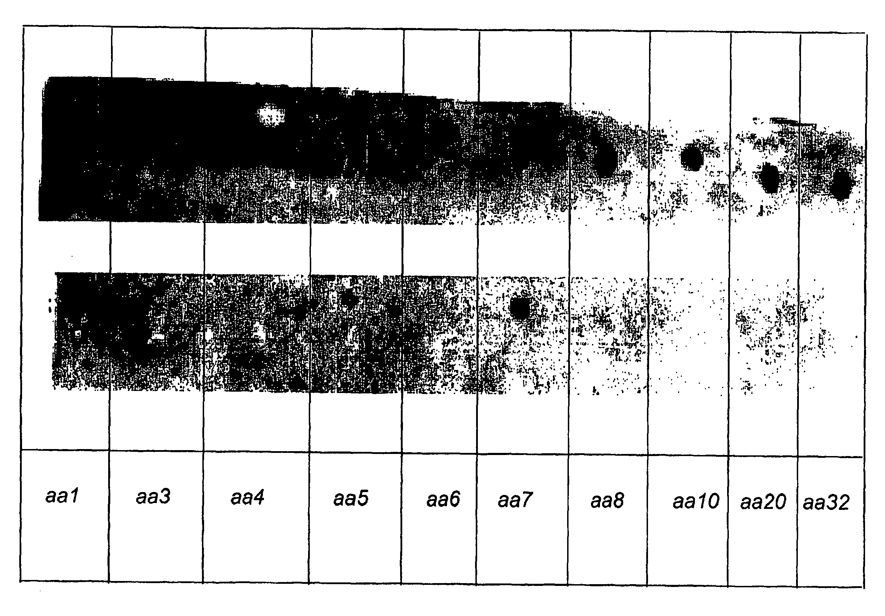

As depicted in Figure 4 all 10 ABC transporter genes clearly show elevated expression during penicillinG fermentation conditions.

### EXAMPLE 7

### INDUCTION OF TRANSCRIPTION OF THE ISOLATED PENICILLIUM RT-PCR FRAGMENTS, ENCODING ABC-TRANSPORTERS, BY β-LACTAM ANTIBIOTICS

*Penicillium chrysogenum* was pre-grown overnight on a rich medium (e.g. YEPD) and then diluted into a defined medium containing lactose as the sole carbon source to ensure derepressing conditions. After 24 hours the culture was washed and resuspended in fresh medium containing 100mM penicillinV, 10mM 7-amino deacetoxycephalosporanic acid (7-ADCA) and 100mM adipoyl-7-ADCA, respectively. At several time-points samples were taken for RNA isolation with trizol. This material was used as a template for a semi-quantitative RT-PCR, using RT-PCR beads from Amersham/Pharmacia. The PCR program was as follows: 30 minutes at 42°C (the RT-step); 5 minutes at 95°C; followed by 20 cycles of 30 seconds at 95°C, 30 seconds at 59°C and 45 seconds at 72°C; followed by a final step of 7 minutes at 72°C and cooling at 4°C. The primers used were: *aa7* forward primer (SEQ ID. 49): 5'-ATT GGC TGA GAC AGC AAA TCT CGC-3'. *aa7* reverse primer (SEQ ID. 50) 5'-AGG ATC GGA AAT GAG GGC GC-3'. *aa10* forward primer (SEQ ID. 51): 5'-AGC GGT TCT ATC TCC CAG TCG GAG G-3'. *aa10* reverse primer (SEQ ID. 52): 5'-CAC GAG CGA TGG CAA TAC GTT GC-3'. *dd062* forward primer (SEQ ID. 53) 5'-TGG AGT GCC CGA TGA TGT ATG-3'. *dd062* reverseprimer (SEQ ID. 54): 5'-CGC GAG GTT GCT ACC TGG AGA G-3'.

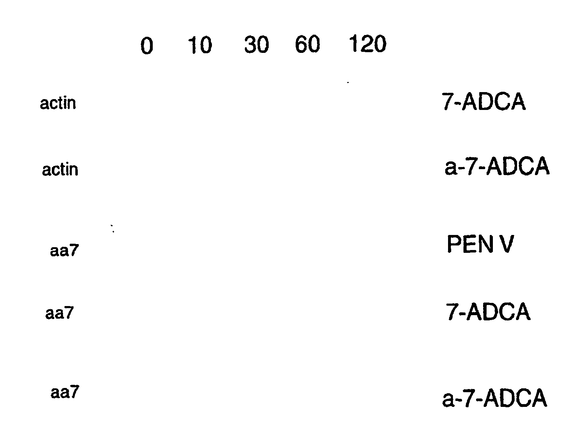

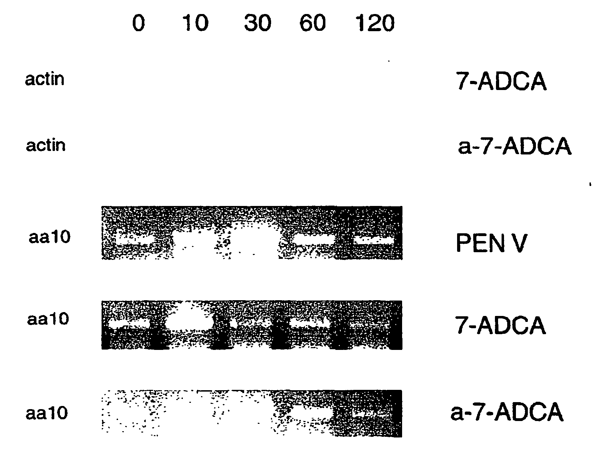

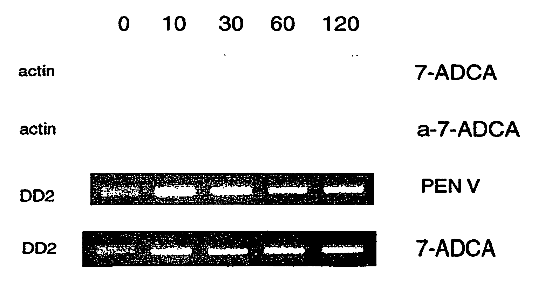

Of the three ABC transporters tested (*aa7, aa10 and DD062*) all responded positively to the addition of β-lactams (see respectively Figure 5, 6 and 7).

### EXAMPLE 8

### INDUCTION OF TRANSCRIPTION OF AA5, ENCODING AN ABC-TRANSPORTER, BY PENICILLING

*Penicillium chrysogenum* was pre-grown to mid-logarithmic phase on a rich medium (e.g. YEPD) when 100mM penicillinG was added to the culture. At several time-points samples were taken for RNA isolation with trizol. This material was analyzed with a Northern blot (as described in example 5) using the PCR-fragment cloned in the pGEM-T easy vector (as described in example 3) as a probe.

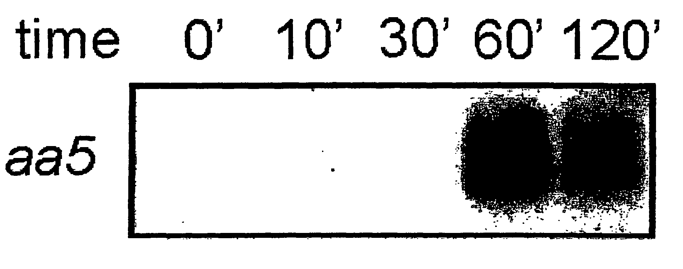

Theinduction of penicillinG on the transcription of the ABC transporter gene takes place in the presence of high glucose concentrations and subsequently the absence of penicillin production (see Figure 8).

### EXAMPLE 9

### CLONING OF THE COMPLETE GENES

### Aa7 and aa10

A phage lambda genomic *P. chrysogenum* DNA library was successfully screened using the 400 bp fragments from the pGEM-T Easy vector (see example 3) as probes. Hybridization was carried out overnight in: 1 mM EDTA; 0.5 M Na2HPO4, pH 7.2; 7% SDS; 1% Blocking agent (Cat. No. 1096176, Roche Molecular Biochemicals). Washing was done 2X 30 minutes in: 1 mM EDTA; 40 mM Na2HPO4, pH 7.2; 5% SDS, followed by 2X 30 minutes in: 1 mM EDTA; 40 mM Na2HPO4, pH 7.2; 1% SDS. All steps were performed at the stringent temperature of 65°C. The probes were labeled with 32P via PCR (25 microCi 32P dCTP, standard protocol Oligolabeling Kit (27-9250-01, Pharmacia Biotech.). DNA from positive clones was excised *in vivo* by picking the core phage of the plaque of interest; transfer it into 500µl buffer and 20µl chloroform; the subsequent incubation of 2 hours at room temperature releases the phage particles. 200µl of XL1-Blue cells (grown in LB-medium to an OD600=1) were mixed with 200µl of the phage particles and 10µl R408 helper phage (Predigested Lambda ZAP II/*Eco*RI Cloning Kit; Catalog#236211, Stratagene). This was incubated for 15 minutes at 37°C, and afterwards 5 ml 2XTrypton medium was added for another incubation of 3 hours at 37°C. The temperature was increased to 70°C for 20 min, the mixture was spun down for 5 minutes at 4000 g and transformed to XL1-Blue host cells.

These clones were sequenced (see SEQ ID NO. 25 and 34). The gene *aa7* contains one putative intron, while *aa10* contains no introns (see SEQ ID NO. 26 and 35).

### Aa1 and aa5

These clones were isolated directly from the phage lambda library. The methods employed were the same as described for *aa7* and *aa10*. The genomic sequences of *aa1* and *aa5* are depicted in SEQ ID NO. 8 and 18. As presented in SEQ ID NO. 9 and 19 these genes contain a lot more introns: 8 and 4, respectively.

### Aa3, aa4, aa6, aa8, aa20, aa32, dd034 and dd062

Chromosomal DNA of a high-production strain of *Pencillium chrysogenum* (e.g. Panlabs P2) was digested with 5 different restriction enzymes; *Eco*RI, *Hin*dIII, *Sal*I*, Bam*HI and *Pst*I*.* 3 µg of the digested chromosomal DNA was separated on an 0.4 % agarose gel with ethidium bromide and the DNA was transferred on a hybond-N+ filter (Pharmacia Amersham Biotech) by vacuum blotting and afterwards cross-linked by UV light.

As probes we amplified the approximately 400 bp fragments of the ABC-transporter genes by PCR from the pGEM-T easy vectors (see example 3).The PCR products were purified from gel with the Gel Extraction Kit (Qiagen). The hybridization was essentially the same as described in example 6. Hybridization signals were obtained for all probes.

### EXAMPLE 10

### SIMILARITY/IDENTITY SEARCHES USING BLASTP

The longest protein sequence derived from each ABC transporter gene described in this invention were analyzed using the blastP software with settings as described in example 2.

| ABC trans porter gene | DNA translation depicted in SEQ ID NO: | Protein sequence depicted inSEQ ID NO: | Similari ty score (%) | Identity score (%) | Closest homologue |
|---|---|---|---|---|---|
| *aa1* | 9 | 10 | 71 | 54 | TREMBL\|AF071410 1 gene: *"atrC";* product: "ATP-binding cassette multidrug transport protein ATRC"; *Emericella nidulans* |
| *aa3* | 12 | 13 | 67 | 46 | TREMBL\|AC004411_16 gene: *"At2g47000";* product: "putative ABC transporter"; *Arabidopsis thaliana* |
| *aa4* | 15 | 16 | 89 | 83 | PIR\|T30566 ATP-binding cassette multidrug transport protein - *Emericella nidulans* |
| *aa5* | 19 | 20 | 79 | 60 | TREMBL\|ANPGP 2_1 product: "ATP-binding cassette multidrug transporter"; *A. nidulans* |
| *aa6* | 22 | 23 | 92 | 87 | PIR\|T3O567 ATP-binding cassette multidrug transport protein- *Emericella nidulans* |
| *aa7* | 26 | 28 | 66 | 47 | TREMBL\|AF173826_1 gene: *"abcD";* product: "ABC-transporter"; *Emericella nidulans* |
| *aa8* | 30 | 31 | 90 | 86 | TREMBL\|MGR243113_1 gene: *"atr2";* product: "putative ABC transporter"; *Mycosphaerella graminicola* |
| *aa10* | 35 | 36 | 85 | 75 | TREMBL\|U62934_1 gene: *"mdrl";* product: "multidrug resistance protein 1"; *Aspergillus fumigatus* |
| *aa20* | 38 | 39 | 85 | 77 | P\|R\| T43022 ATP-binding multidrug cassette transport protein - *Botryotinia fuckeliana* |
| *aa32* | 41 | 42 | 78 | 64 | PATENTPROT Y21815 A. *nidulans atrC* polypeptide |
| *dd034* | 44 | 45 | 89 | 69 | TREMBL\|BFU6217_1 gene: *"atrB";* product: "ABC transporter"; *Botryotinia fuckeliana* |
| *dd062* | 47 | 48 | 56 | 38 | PIR\|T43551 multidrug resistance protein fnx1 fission yeast (*Schizosaccharomyces pombe*) |

### EXAMPLE 11

### TRANSFECTION OF THE ABC TRANSPORT PROTEIN GENE INTO A β-LACTAM-PRODUCING MICRO-ORGANISM

The above described full genomic gene was used as template DNA for a PCR reaction with two homologous primers, which insert a *Nde*I restriction site just 5' of the start codon and a *Nsi*I restriction site just 3' of the stop codon. The resulting fragment was cloned into an expression vector. For this the vector pISEWA, as described in PCT/EP99/03455, was digested with *Nde*I and *Nsi*I. The *pcbC* promoter was replaced by one of the suitable promoters mentioned in the text. The resulting plasmids were transformed to *Penicillium chrysogenum* (using co-transformation with the *A. nidulans amdS* gene under control of *the A. nidulans gpdA* promoter) and the penicillin production was tested as described above and found to be enhanced.

### EXAMPLE 12

### DISRUPTION OF THE ABC TRANSPORT GENE.

The above described full genomic genes of aa7 and aa 10 were disrupted by inserting a heterologous selectionmarker like the *Aspergillus nidulans amdS* gene under control of the *A. nidulans gpdA* promoter. Transformants growing on agar plates with acetamide as the sole nitrogen source were selected. Colony PCR was employed to select the homologous integrants from the random transformants. Southern analysis confirmed the disruption of the aa7 and aa10 gene described in this invention.

### DESCRIPTIONS OF FIGURES

Figure 1.
   Schematic representation of the C-terminal nucleotide binding domain (NBD) found in ABC-transporters. Examples of some WalkerA and WalkerB sequences found in a variety of ABC-tranporters are given. The boxed amino acid sequences were used to develop degenerate primers. (*AtrA* and *AtrB* of *A. nidulans; MDR1* of human; *CDR1* of *C. albicans; ATM1* and *STE6* of *S. cerevisiae; MalK* and *HisP* of *E. coli*).
Figure 2.
   Schematic representation of a typical penicillin production curve with *Penicillium chrysogenum.* Samples at indicated timepoints A and B were used for mRNA isolation and subsequent AFLP analysis.
Figure 3.
   Northern analyses showing expression of several isolated ABC-transporter gene fragments, from a high yielding strain (e.g. Panlabs P2 or Wisconsin54-1255) grown on glucose-containing medium or under conditions optimal for penicillin V production. Expression of the pcbC gene (encoding the penicillin biosynthetic enzyme isopenicillin N synthase) was used as a positive control; specific expression under penicillin producing conditions. 18S rRNA and the *actA* gene (actin gene, isolated from a cDNA library by PCR) were used to check for equal gel loading.
Figure 4.
   DNA filter analysis showing expression of several isolated ABC-transporter gene fragments. mRNA *was isolated* from Wisconsin54-1255 grown on glucose-containing medium or under conditions optimal for penicillinG production. All genes show elevated expression when producing penicillin G. Top-panel: Wisconsin54-1255 producing penicillinG Bottom-panel: Wisconsin54-1255 grown on glucose (not producing penicillinG).
Figure 5.
   Time-course experiments after addition of β-lactam antibiotics to growing cultures of *Penicillium chrysogenum* aa7 mutants. In the experiment the actin gene was used a a control for equal amounts of starting material. The time is indicated in minutes.
Figure 6.
   As in figure 5, but now for *Penicillium chrysogenum* aa 10 mutants.
Fig ure 7.
   As in figure 5, but now for *Penicillium chrysogenum* DD062 mutants.
Figure 8.
   Time-course analysis of aa5 transcription after addition of penicillinG to a growing culture of *Penicillium chrysogenum.*

(SEQ ID NO. 3) Degenerate forward primer I (complementary to Walker A motif) :
   5'-TCI GGI KSI GGI AAR WCI AC-3'
(SEQ ID NO. 4) Degenerate forward primer II (complementary to Walker A motif):
   5'-TCI GGI KSI GGI AAR AGY AC-3'
(SEQ ID NO. 5) Degenerate reverse primer I (complementary to Walker B motif) :
   5'-TCI ARI SCI GAI GTI GSY TCR TC-3'
(SEQ ID NO. 6) Degenerate reverse primer II (complementary to Walker B motif) :
   5'-TCI ARI SCR CTI GTI GSY TCR TC-3'
(SEQ ID NO. 7) Partial cDNA sequence of *Penicillium chrysogenum* ABC transporter *aa1* (Shown in bold are the sequences complementary to the Walker-motif primers used for the PCR):
(SEQ ID NO. 8) Complete genomic DNA sequence of *Penicillium chrysogenum* ABC transporter *aa1* (Shown in bold are the sequences complementary to the Walker-motifs; the cDNA sequence from (SEQ ID NO. 7) is underlined) :
(SEQ ID NO. 9)Translation of the complete genomic DNA sequence of *Penicillium chrysogenum* ABC transporter aa1 (The DNA sequence is shown above the dashed line; shown in bold are the putative intron sequences which are spliced out during RNA processing; the derived amino acid sequence is shown below the dashed line; start- and stopcodon are shown in italic):
(SEQ ID NO. 10) Protein sequence of the *Penicillium chrysogenum* ABC transporter *aa1* (derived from the DNA sequence as shown in (SEQ ID NO. 8)):
(SEQ ID NO. 11) Partial cDNA sequence of *Penicillium chrysogenum* ABC transporter *aa3* (Shown in bold are the sequences complementary to the Walker-motif primers used for the PCR):
(SEQ ID NO. 12)Translation of the cDNA sequence of *Penicillium chrysogenum* ABC transporter *aa3* from SEQ ID NO. 11:
(SEQ ID NO. 13)Protein sequence of the *Penicillium chrysogenum* ABC transporter *aa3* (derived from the DNA sequence as shown in SEQ ID NO. 11):
(SEQ ID NO. 14)Partial cDNA sequence of *Penicillium chrysogenum* ABC transporter *aa4* (Shown in bold are the sequences complementary to the Walker-motif primers used for the PCR):
(SEQ ID NO. 15)Translation of the cDNA sequence of *Penicillium chrysogenum* ABC transporter *aa4* from SEQ ID NO. 14:
(SEQ ID NO. 16) Protein sequence of the *Penicillium chrysogenum* ABC transporter *aa4* (derived from the DNA sequence as shown in SEQ ID NO. 14):
(SEQ ID NO. 17)Partial cDNA sequence of *Penicillium chrysogenum* ABC transporter *aa5* (Shown in bold are the sequences complementary to the Walker-motif primers used for the PCR) :
(SEQ ID NO. 18) Complete genomic DNA sequence of *Penicillium chrysogenum* ABC transporter *aa5:*
(SEQ ID NO. 19)Translation of the complete genomic DNA sequence of *Penicillium chrysogenum* ABC transporter *aa5* (The DNA sequence is shown above the dashed line; shown in bold are the putative intron sequences which are spliced out during RNA processing; the derived amino acid sequence is shown below the dashed line; start- and stopcodon are shown in italic) :
(SEQ ID NO. 20) Protein sequence of the *Penicillium chrysogenum* ABC transporter *aa5* (derived from the DNA sequence as shown in SEQ ID NO. 18) :
(SEQ ID NO. 21)Partial cDNA sequence of *Penicillium chrysogenum* ABC transporter *aa6* (Shown in bold are the sequences complementary to the Walker-motif primers used for the PCR):
(SEQ ID NO. 22)Translation of the cDNA sequence of *Penicillium chrysogenum* ABC transporter *aa6* from SEQ ID NO. 21:
(SEQ ID NO. 23) Protein sequence of the *Penicillium chrysogenum* ABC transporter *aa6* (derived from the DNA sequence as shown in SEQ ID NO. 21):
(SEQ ID NO. 24)Partial cDNA sequence of *Penicillium chrysogenum* ABC transporter *aa7* (Shown in bold are the sequences complementary to the Walker-motif primers used for the PCR) :
(SEQ ID NO. 24)Large, partial cDNA sequence of *Penicillium chrysogenum* ABC transporter *aa7* (Shown in bold are the sequences complementary to the Walker-motifs; the smaller cDNA sequence of SEQ ID NO. 23 is underlined) :
(SEQ ID NO. 25)Complete genomic DNA sequence of *Penicillium chrysogenum* ABC transporter *aa7* (Shown in bold are the sequences complementary to the Walker-motifs; the cDNA sequence of figure 23 is underlined;the cDNA sequence of SEQ ID NO. 24 is in italic):
(SEQ ID NO. 26)Translation of the complete genomic DNA sequence of *Penicillium chrysogenum* ABC transporter *aa7* (The DNA sequence is shown above the dashed line; shown in bold are the putative intron sequences which are spliced out during RNA processing; the derived amino acid sequence is shown below the dashed line; start- and stopcodon are shown in italic):
(SEQ ID NO. 28) Protein sequence of the *Penicillium chrysogenum* ABC transporter *aa7* (derived from the DNA sequence as shown in SEQ ID NO. 26):
(SEQ ID NO. 29)Partial cDNA sequence of *Penicillium chrysogenum* ABC transporter *aa8* (Shown in bold are the sequences complementary to the Walker-motif primers used for the PCR):
(SEQ ID NO. 30)Translation of the cDNA sequence of *Penicillium chrysogenum* ABC transporter *aa8* from SEQ ID NO. 29:
(SEQ ID NO. 31)Protein sequence of the *Penicillium chrysogenum* ABC transporter *aa8* (derived from the DNA sequence as shown in SEQ ID NO. 29):
(SEQ ID NO. 32)Partial cDNA sequence of *Penicillium chrysogenum* ABC transporter *aa10* (Shown in bold are the sequences complementary to the Walker-motif primers used for the PCR):
(SEQ ID NO. 33)Large, partial cDNA sequence of *Penicillium chrysogenum* ABC transporter *aa10* (Shown in bold are the sequences complementary to the Walker-motifs; the smaller cDNA sequence of SEQ ID NO. 32 is underlined)
(SEQ ID NO. 34)Complete genomic DNA sequence of *Penicillium chrysogenum* ABC transporter *aa10* (Shown in bold are the sequences complementary to the Walker-motifs; the cDNA sequence of SEQ ID NO. 32 is underlined;the cDNA sequence of SEQ ID NO. 33 is in italic):
(SEQ ID NO. 35)Translation of the complete genomic DNA sequence of *Penicillium chrysogenum* ABC transporter *aa10* (The DNA sequence is shown above the dashed line; the derived amino acid sequence is shown below the dashed line; start- and stopcodon are shown in italic):
(SEQ ID NO. 36) Protein sequence of the *Penicillium chrysogenum* ABC transporter *aa10* (derived from the DNA sequence as shown in SEQ ID NO. 34):
(SEQ ID NO. 37)Partial cDNA sequence of *Penicillium chrysogenum* ABC transporter *aa20* (Shown in bold are the sequences complementary to the Walker-motif primers used for the PCR):
(SEQ ID NO. 38)Translation of the cDNA sequence of *Penicillium chrysogenum* ABC transporter *aa20* from SEQ ID NO. 37:
(SEQ ID NO. 39)Protein sequence of the *Penicillium chrysogenum* ABC transporter *aa20* (derived from the DNA sequence as shown in SEQ ID NO. 37):
(SEQ ID NO. 40) Partial cDNA sequence of *Penicillium chrysogenum* ABC transporter *aa32* (Shown in bold are the sequences complementary to the Walker-motif primers used for the PCR):
(SEQ ID NO. 41)Translation of the cDNA sequence of *Penicillium chrysogenum* ABC transporter *aa32* from SEQ ID NO. 40:
(SEQ ID NO. 42)Protein sequence of the *Penicillium chrysogenum* ABC transporter *aa32* (derived from the DNA sequence as shown in SEQ ID NO. 42):
(SEQ ID NO. 43)Partial cDNA sequence of *Penicillium chrysogenum* ABC transporter *dd034* (Sequence is derived from AFLP experiment)
(SEQ ID NO. 44)Translation of the cDNA sequence of *Penicillium chrysogenum* ABC transporter *dd034* from SEQ ID NO. 43:
(SEQ ID NO. 45) Protein sequence of the Penicillium *chrysogenum* ABC transporter *dd034* (derived from the DNA sequence as shown in SEQ ID NO. 43):
(SEQ ID NO.46)Partial cDNA sequence of *Penicillium chrysogenum* ABC transporter *dd062* (Sequence is derived from AFLP experiment) :
(SEQ ID NO. 47)Translation of the cDNA sequence of *Penicillium chrysogenum* ABC transporter *dd062* from SEQ ID NO. 46:
(SEQ ID NO. 48)Protein sequence of the *Penicillium chrysogenum* ABC transporter *dd062* (derived from the DNA sequence as shown in SEQ ID NO. 46):

### SEQUENCE LISTING

<110> DSM NV
<120> METHOD FOR ENHANCING SECRETION OF β-LACTAM COMPOUNDS
<130> WO 2958
<140>
   <141>
<160> 52
<210> 1
   <400> 1
   0000
<210> 2
   <400> 2
   0000
<210> 3
   <211> 20
   <212> DNA
   <213> Artificial Sequence
<220>
   <223> Degenerate forward primer I
<400> 3
   tcnggnksng gnaarwcnac 20
<210> 4
   <211> 20
   <212> DNA
   <213> Artificial Sequence
<220>
   <223> Degenerate forward primer II
<400> 4
   tcnggnksng gnaaragyac 20
<210> 5
   <211> 23
   <212> DNA
   <213> Artificial Sequence
<220>
   <223> Degenerate reverse primer I
<400> 5
   tcnarnscng angtngsytc rtc 23
<210> 6
<211> 23
   <212> DNA
   <213> Artificial Sequence
<220>
   <223> Degenerate reverse primer II
<400> 6
   tcnarnscrc tngtngsytc rtc 23
<210> 7
   <211> 429
   <212> DNA
   <213> Penicillium chrysogenum
<220>
   <223> Partial cDNA of Penicillium chrysogenum ABC transporter aa1
<400> 7
   tcgggggcgg ggaagagtac catcgtcggg ctgatcgaac gttggtacga tcctttgtcg 60
   ggaacgatca agcttgacgg ccagactatc gagacgttca acctcaaatg gctacgccag 120
   caggttagac ttgttcaaca ggagcccgtt ttgttcgctg gaacagttgc tgagaatatc 180
   gccaatggcc ttgtgggcac accgtgggag aatgaatccg cagccgagaa gcaggcccgg 240
   atacaggacg ctgctaaaat tgctttcgcc catgatttca tcacggaact tcccaatgga 300
   tatgatacag tcattggaga gagaggtggg ctattatccg gtggtcaaaa acagcgggtt 360
   gcaattgctc gcagtattgt ttcccagccc cgaattcttc ttctggacga acccaccagc 420
   gccctcgaa                                                         429
<210> 8
   <211> 7744
   <212> DNA
   <213> Penicillium chrysogenum
<220>
   <223> Complete genomic DNA sequence of Penicillium chrysogenum ABC transporter aa1
<400> 8
   gatcttgtta agcgcatgtc cgacatggag gtcgccattt gcatatggcg gaccatcgtg 60
   caggacaaat ggttggtcgg cgggcctttc acggcgttgc caggcataaa gatcatcggt 120
   gcatcgtttt aggtatttcg tccggtcgac aggcgataca cgtggaggga atgatgactt 180
   aggaagtgtc aatgtcgagg tccatgagcg ggggattgac cgcgccagtt ccggcattgt 240
   ccagcatatc cctagaaagt gataaggtgg gaaggtcagg aagagaagag tcttggcatt 300
   ttacaaaaac atgcctgcca gaaagggttt agactccgcc agtattttta aatatttaaa 360
   gacactagta gtactataag cccatccgac tacaacatac attaatccat cattcctgct 420
   gtacaaatgt gctaaagaaa agtgtgcact tcattacata tataaatata ctaagtaaag 480
   aaaggggaat gggaagaaga ggagagattg gacaagaggg gagcctttgt ctcctccgat 540
   acatgtaaac agtgcaaaca gtcggtgtgg agtggcagaa cccttgatcc tatatctgaa 600
   atatcagcag cccaggtctc aacgctaatg ttgtactcgc ggatcacgct ttcgtcgatg 660
   tatgatcgaa cgagtttcaa cacatcccgg taacgaggtg taaagcacat cgccattttg 720
   tgagaaacgg tccactcaat ggatgtattg aactgtgaag actccacatg agtagtatcc 780
   gggcgtccag aagaggcggg tcgggcgtac gagcaaagtt agcaaagcct cctctcgcat 840
   ctattggggc aaggccatca agatattgaa tgaacttcag ctggctatgg gtaataattg 900
   taataatcca tatttatcaa aggacaaata aggaataagg aatactaatt tctagcccgt 960
   cattccctca catattatct gacgtagttc gttattactt actactcagt aacttggtat 1020
   gttgatggaa taacatacgg caagattcca ctaggccacg aaaaccaagg ttctcgttga 1080
   gggcttaggg aaactcatat cccagtttgg catatgtaaa tacgttgcga gggatcgggc 1140
   cccgcggaac cccgagagga ttttcggcca ggccaagaca cccaaaccat cagcagtgca 1200
   gcccgcggac cccggatagc cgcatgacca cccgtggaat ccaagtctaa aacagccgca 1260
   tatctcgatg gctaggaatt atcgttatgt cctccccaac acagtgtttg gatcgtacat 1320
   cgtcaaaata gagcaaagtc gaccctatgc tcgcctaacg ggggttcgcc tgaacatcta 1380
   caatggagaa agaatccgtg aaatatagat cagatggaca gggagctgag gaacaaacaa 1440
   atgcatttaa tggttacctg gtagtatcac cccctcccat cgcaggctta aaggctaaca 1500
   tcaggaagag gatctttcag tatggctcgt ccaaggtgta taccatgcag ggcattgcca 1560
   ctgcctgcgc actggggtcc ggcgtcggca tggctcttgt taatctagtg tttggccagt 1620
   ttatcacagt catcactgac tacacgtcgg gtcgtataga tcctgcgagg ttccgaggcg 1680
   aagcagggag actcaggtat gtgccctgcg ttcaatccag atcagcagaa gaaattaata 1740
   taaagatagt ctctacttct ttgtcattgg cctttgccga ttctttttaa cttatggata 1800
   cagcacactg tttacaattg ccgcgtaccg gatcacgcga aatattcgcc gcctgtatct 1860
   gaaagcaggt ctgacccagg aaattgcctt ctttgattct ggcgctagtg gatcgattgc 1920
   cgtgcaagct acctcgaacg gacgattgat ccagagtgga atatcggaaa agctgggcct 1980
   tgttgtccag ggcctgtcta cttttatatc cgccttcgtg ctggcatttg tcactcattg 2040
   gaagttgact ctcatttgct gttgtattgc cccagcaatg cttctcgccc taggaatcag 2100
   ctctgccatt gaagcttcta ttgaaacaaa agttctcaaa gtccacgcgc aggctggagc 2160
   tttcgcagaa agtattttgt caagctctcg gactgtacag gcgttcggac ttcggaaaca 2220
   actgctacgc gactttgaca gattcttgca agcttcaagg aatctcggaa acaagaaaag 2280
   ccctcttttt ggctgtctat ttgccacgga gtacagcatc atctttgcag ggtttggctt 2340
   gtgcttttgg cagggtataa aaatggtatc gactggggag gtcgcgcaat caggagatat 2400
   tttcacgttt gttcagtctc agtcatttta cagctggcga tgctaatcat ggtaattgca 2460
   gtgtgctcat gtctgtcgtt gtggcagcta caagcttgac atctatactg ccttacgtga 2520
   ttgattttgt ccgcgctgcc tctgcagcgg cagagctatt ccagctcatg gaccggacgt 2580
   ctaggattga tccgtttgat gagtccggag aaagaccgac agacgttacg ggatatctcg 2640
   attttggtgg catttcgttt gcctacccta cgcgccctga cgtcaaggtg ttgaatgatt 2700
   tctcattgca tatccctgcc gggaagacaa ctgcgctcgt cggagcaagc gggtctggta 2760
   aaagcaccat cgtcgggctg atcgaacgtt ggtacgatcc tttgtcggga acgatcaagc 2820
   ttgacggcca gactatcgag acgttcaacc tcaaatggct acgccagcag gttagacttg 2880
   ttcaacagga gcccgttttg ttcgctggaa cagttgctga gaatatcgcc aatggccttg 2940
   tgggcacacc gtgggagaat gaatccgcag ccgagaagca ggcccggata caggacgctg 3000
   ctaaaattgc tttcgcccat gatttcatca cggaacttcc caatggatat gatacagtca 3060
   ttggagagag aggtgggcta ttatccggtg gtcaaaaaca gcgggttgca attgctcgca 3120
   gtattgtttc ccagccccga attcttcttc tggatgaagc aacgagtgca cttgatcctc 3180
   atgccgagga agttgtgcaa cgggcactaa acaatgtttc gaagggacgc acaacgatca 3240
   ccatcgcaca caagctagcc accataagag atgctcataa tattgtcgtc atggaaaatg 3300
   gccgtattct cgaacagggc actcataaat cgcttttgga ttcgaatggc gcctacgcac 3360
   gcctagtgca agcccaggat ttgtctgtgg ccgcgcagaa ccccgaagat gtgtcgaata 3420
   gctcggatga aacagataag gaagaccatg tcacactgac tggtgaattg acccggtgct 3480
   caactatgac gagatcagct ttggagaaac ggttgagtcg ggatgatttt gacaactgga 3540
   aaagattggg gcttctccac acagtctgga gattggtgaa atcgacccct gagctaaatt 3600
   ggacttttgc gattctgatt ctgtcttgtc tcgctggagg tgaggctagc atttttaagc 3660
   ccaattcccg cgaactgaga ctgaccaaat tgtagctgcg tctttccctg gccaagctat 3720
   tttgatgtcc aggtttatcg aagtatttca gttcacaggt gcagctatgc ggacgaaagg 3780
   gaactttttc gctctcatgt tcttggttct gggacttgga gctttcgtcg tctacttcgt 3840
   tgttgggtgg acatcaaatg ttgtggcaca ggtgagtctc ttccagtgat gtatgagtat 3900
   atcttggctt gactaactca tccagacaat gaaccataaa taccggaaac aagtcgttaa 3960
   tgacatgatg aggcaagacc tgcaattctt tgatcgcgcc gagaacacca caggggcact 4020
   aaccagccgt gccgactctt atccacaggc tgtgtttgag cttatggggt ttaatgttgc 4080
   tctgatatgg tagcaacggt cggtgtattg tcatgctccg tcctagcact ggcatatgca 4140
   tggaagctcg gtcttgttat tatcttagca ggacttccac ccatgctcct ttctggttac 4200
   gctcgcatca gaatggaggg tgcaatggac cacaaaattt cgaagatgtt ttgcggaagc 4260
   gcatccattg cctcagaagc cgtgacggct atccggacgg tgtcctcctt ggcgattgag 4320
   acgtcagttt tggagcgata caccaaggag cttgaccgag cgaatgcgac atctacgaag 4380
   ccagtgcttc tcatcatgct ccctttcgca ttcactcaga ctgttgaata ctctttcctg 4440
   gctctcggtt tctggtatgt ttgtcgtata tgttccatat gctcgaagtg gcttgttctc 4500
   ggtttgatga caggatgcta accaccggat cgtctctagg tatggatgcc gtcttgtctc 4560
   atttggagat gtgagcatgg tgaacttttt catcagtttc ttgagtgtgt ttttttctgg 4620
   ccagcaagct acaatccttt ttggcttctc aagcagtaag atgcattcca aacctgtatg 4680
   ggaggcgaaa gtgagttgct aatagttata caggcatgac aaaggcaaca aatgctgcca 4740
   attacatatt ttggctggag gagctccagc ctaccattcg agagaccgac gaaaaccgtg 4800
   acattggccc tggcgacctt aagtctctgc acttggaaag cctgcaattt tcttacccaa 4860
   tgagacctca cgctcgtgta ctacgtggca ttgaccttca cgtatgtcag cccaacttgt 4920
   cctcttttca atcctgtcct gtggtctacc cacttgacgc cataaacctg tccttgttcg 4980
   ctgctaatat attgtataga tcgaaaaaga acaatttgtc gcattcgtcg gcgcatctgg 5040
   ctgtggaaaa agtacgatga ttagcatgct cgaacgcttt tacgatccag tagccggtca 5100
   cattaaaatc gacgcactta cattggatag cttgaatccg tggctgtatc gaggtcaagt 5160
   ggcacttgtg cagcaggaac ccaccctcta tcccggcacc attcgcgaaa acgtcgccat 5220
   ggggacaccg actgatgacg gatcaacggt tcctgacagt gatattgaag ctgcatgtcg 5280
   ggctgccaac gcgtgggaat ttatctcttc tctgcccgat ggccttatga ctctctgtgg 5340
   ggcgaatggg acacagctgt ctggtggcca acgtcagcgc atcgccattg ccagggcgct 5400
   actcagaaat ccacgtttac tcctacttga tgaagcaaca agtgcgttgg atactcagtc 5460
   ggagagaatt gtccaagatg cgctgaatga ggctgcttcc catggggata gaattacgat 5520
   cgcggttgcc catcgcttgt ctactattcg ccatgccgat atgatttgcg ttttcgacgg 5580
   gggcaggatc gctgagtctg gaactcatga ggaattactg atgaaaggcc gtctttatcc 5640
   gaaaatgtgc gaggctcaga acctcggcac ttaatgaagc atcctatctt ctgctccttc 5700
   caaagccatg agtttgaata tgttatatat gtacgtactt ggaataatcg aacgcgatga 5760
   tggtctgatt agtagatcag cgcagataga taatgtaatt taactgaagc gaatatatac 5820
   tcaaagcctt tcgactattc ccccatatac aatgtccaaa agccccatgt aaaataacgt 5880
   cctcctcccc gggttcgctg ccgtctttcg ggtcagcgag ccagctcagc ggcaattgca 5940
   tacaaccatt gaaggggcaa atccttgatc cgataaagtt cgggatggac tccgtttctc 6000
   gtccaactat caattcttat gagccagtct tcttagactc atcgggtata gaggggcctc 6060
   ggacaacccc aagattccgc gtcagatcgg gtagtagcta caagcctcgc gaacgaaatg 6120
   ccacagacta aagatatgaa ggcgtctaca agccgtcaga tggccttaat atagtaatcg 6180
   ataaggctgt tgagggatat agtaagtatc tgagggtagg gcagtaagtc tggacagaag 6240
   ccctcgaaac cttcatctcg cctgttctcg ctttccaaaa agccgataat ttgattctgc 6300
   tgagacaagc tatcatccga ttcctggatt gtcaggcata gtaaccaggt cacttctcca 6360
   gggctccagg gctagcgtgt atttcagttg cttctactgg tactcggatt acaatgtagc 6420
   agcttcctgt accgggctca gccactgtgt agggcccaaa gatattgtct ccttccctca 6480
   ttgaccgact tcggtatcgg tggaacgggt gatgaagggc gatttggatc tctaatatgt 6540
   ctgaatttgc tcataggtag ccagcattgc ccaagttacc acaatactcc gccccgtatt 6600
   gattcaaaac ggagagagct tctttaagat gactttgcaa catgtcctct cggacttcga 6660
   tcccgttgcg agcaagttca gatagtgtgg atccagcaac ctctaacaaa ataagcccag 6720
   gaattccgtt gagcgatcct tcactaaaag gctgcgaaat gactgttcct tgaaggtcct 6780
   ccggtgtctt gtagacttct ttctggcgct gaaattcggg gacccatccg tccttctgtt 6840
   gcttaataaa tgatcgactt cacattgtga ggtagagcat tgtgacatgt ggatggccca 6900
   gtctcaaggc ggagtacagt gggacgtcgt tgatctagag ccttgagcgt tcgcacggcg 6960
   acgcgctcgc catgtagata aacaatctaa tccctattaa gcttgataac ttgtcaggtc 7020
   tagaagatat agcttacact cgaaagatat gattcaggca taggcgcgtt ggggatggaa 7080
   ggcagaaaaa gagaaacaaa caaggggaca taaaaagcct gcggagttcc cagacctcca 7140
   agggccaggg ctccgtctcc ccatggtctc cctaggtagg taggggcccg gggtaggcgg 7200
   ctgattacta aggcaggaat agtgctggcc agccctgggg cattatcatg attcataatg 7260
   cattgaaata tgattaaaaa gtgctggcaa tagtctcaaa tcagtacact gtcaagcaac 7320
   tcacggcgcc attcttgagg acttcaaata aaagccccgg gaacccaaaa agctcggtgt 7380
   tgcccaaccc cccatcctgc ccatctgcac caccccgagg acctttggcc gtggtctcct 7440
   cccagccacc aagaaactca acgctactct cagggctttc tccccagcca cggttcagca 7500
   ccattggccg ttgcagagca cgctcctcgg cggggctggc ataagatcct ttccaagtag 7560
   tacgcagccg ttctgagact tcatcatagg gcgattccga agtcagacag tcgccagatt 7620
   catccaaggg aattagccag cgacttcgcc ggtgacgatt aaatggataa gagcccggca 7680
   cgttcccatg gagaataatc ttggtcactg tcagctggga agagggtggc tgaggcgatg 7740
   gatc 7744
<210> 9
   <211> 7744
   <212> DNA
   <213> Penicillium chrysogenum
<220>
   <221> CDS
   <222> (1383)..(1699)
<220>
   <221> CDS
   <222> (1748)..(2904)
<220>
   <221> CDS
   <222> (2460)..(3638)
<220>
   <221> CDS
   <222> (3695)..(3871)
<220>
   <221> CDS
   <222> (3926)..(4027)
<220>
   <221> CDS
   <222> (4093)..(4452)
<220>
   <221> CDS
   <222> (9538)..(4690)
<220>
   <221> CDS
   <222> (4770)..(4901)
<220>
   <221> CDS
   <222> (5000)..(5671)
<220>
   <223> Translation of the complete genomic sequence of Penicillium chrysogenum ABC transporter aa1
<400> 9
   gatcttgtta agcgcatgtc cgacatggag gtcgccattt gcatatggcg gaccatcgtg 60
   caggacaaat ggttggtcgg cgggcctttc acggcgttgc caggcataaa gatcatcggt 120
   gcatcgtttt aggtatttcg tccggtcgac aggcgataca cgtggaggga atgatgactt 180
   aggaagtgtc aatgtcgagg tccatgagcg ggggattgac cgcgccagtt ccggcattgt 240
   ccagcatatc cctagaaagt gataaggtgg gaaggtcagg aagagaagag tcttggcatt 300
   ttacaaaaac atgcctgcca gaaagggttt agactccgcc agtattttta aatatttaaa 360
   gacactagta gtactataag cccatccgac tacaacatac attaatccat cattcctgct 420
   gtacaaatgt gctaaagaaa agtgtgcact tcattacata tataaatata ctaagtaaag 480
   aaaggggaat gggaagaaga ggagagattg gacaagaggg gagcctttgt ctcctccgat 540
   acatgtaaac agtgcaaaca gtcggtgtgg agtggcagaa cccttgatcc tatatctgaa 600
   atatcagcag cccaggtctc aacgctaatg ttgtactcgc ggatcacgct ttcgtcgatg 660
   tatgatcgaa cgagtttcaa cacatcccgg taacgaggtg taaagcacat cgccattttg 720
   tgagaaacgg tccactcaat ggatgtattg aactgtgaag actccacatg agtagtatcc 780
   gggcgtccag aagaggcggg tcgggcgtac gagcaaagtt agcaaagcct cctctcgcat 840
   ctattggggc aaggccatca agatattgaa tgaacttcag ctggctatgg gtaataattg 900
   taataatcca tatttatcaa aggacaaata aggaataagg aatactaatt tctagcccgt 960
   cattccctca catattatct gacgtagttc gttattactt actactcagt aacttggtat 1020
   gttgatggaa taacatacgg caagattcca ctaggccacg aaaaccaagg ttctcgttga 1080
   gggcttaggg aaactcatat cccagtttgg catatgtaaa tacgttgcga gggatcgggc 1140
   cccgcggaac cccgagagga ttttcggcca ggccaagaca cccaaaccat cagcagtgca 1200
   gcccgcggac cccggatagc cgcatgacca cccgtggaat ccaagtctaa aacagccgca 1260
   tatctcgatg gctaggaatt atcgttatgt cctccccaac acagtgtttg gatcgtacat 1320
   cgtcaaaata gagcaaagtc gaccctatgc tcgcctaacg ggggttcgcc tgaacatcta 1380
   ca atg gag aaa gaa tcc gtg aaa tat aga tca gat gga cag gga gct    1427
      Met Glu Lys Glu Ser Val Lys Tyr Arg Ser Asp Gly Gln Gly Ala
       1               5                  10                  15
   gag gaa caa aca aat gca ttt aat ggt tac ctg gta gta tca ccc cct    1475
   Glu Glu Gln Thr Asn Ala Phe Asn Gly Tyr Leu Val Val Ser Pro Pro
                   20                  25                  30
   ccc atc gca ggc tta aag gct aac atc agg aag agg atc ttt cag tat    1523
   Pro Ile Ala Gly Leu Lys Ala Asn Ile Arg Lys Arg Ile Phe Gln Tyr
               35                  40                  45
   ggc tcg tcc aag gtg tat acc atg cag ggc att gcc act gcc tgc gca    1571
   Gly Ser Ser Lys Val Tyr Thr Met Gln Gly Ile Ala Thr Ala Cys Ala
           50                  55                  60
   ctg ggg tcc ggc gtc ggc atg gct ctt gtt aat cta gtg ttt ggc cag    1619
   Leu Gly Ser Gly Val Gly Met Ala Leu Val Asn Leu Val Phe Gly Gln
       65                  70                  75
   ttt atc aca gtc atc act gac tac acg tcg ggt cgt ata gat cct gcg    1667
   Phe Ile Thr Val Ile Thr Asp Tyr Thr Ser Gly Arg Ile Asp Pro Ala
   80                  85                  90                  95
   agg ttc cga ggc gaa gca ggg aga ctc aggtatgtgc cctgcgttca          1714
   Arg Phe Arg Gly Glu Ala Gly Arg Leu
                   100
   atccagatca gcagaagaaa ttaatataaa gat agt ctc tac ttc ttt gtc att   1768
                                        Ser Leu Tyr Phe Phe Val Ile
                                        105                 110
   ggc ctt tgc cga ttc ttt tta act tat gga tac agc aca ctg ttt aca    1816
   Gly Leu Cys Arg Phe Phe Leu Thr Tyr Gly Tyr Ser Thr Leu Phe Thr
               115                 120                 125
   att gcc gcg tac cgg atc acg cga aat att cgc cgc ctg tat ctg aaa    1864
   Ile Ala Ala Tyr Arg Ile Thr Arg Asn Ile Arg Arg Leu Tyr Leu Lys
           130                 135                 140
   gca ggt ctg acc cag gaa att gcc ttc ttt gat tct ggc gct agt gga    1912
   Ala Gly Leu Thr Gln Glu Ile Ala Phe Phe Asp Ser Gly Ala Ser Gly
       145                 150                 155
   tcg att gcc gtg caa gct acc tcg aac gga cga ttg atc cag agt gga    1960
   Ser Ile Ala Val Gln Ala Thr Ser Asn Gly Arg Leu Ile Gln Ser Gly
   160                 165                 170                 175
   ata tcg gaa aag ctg ggc ctt gtt gtc cag ggc ctg tct act ttt ata    2008
   Ile Ser Glu Lys Leu Gly Leu Val Val Gln Gly Leu Ser Thr Phe Ile
                   180                 185                 190
   tcc gcc ttc gtg ctg gca ttt gtc act cat tgg aag ttg act ctc att    2056
   Ser Ala Phe Val Leu Ala Phe Val Thr His Trp Lys Leu Thr Leu Ile
               195                 200                 205
   tgc tgt tgt att gcc cca gca atg ctt ctc gcc cta gga atc agc tct    2104
   Cys Cys Cys Ile Ala Pro Ala Met Leu Leu Ala Leu Gly Ile Ser Ser
           210                 215                 220
   gcc att gaa gct tct att gaa aca aaa gtt ctc aaa gtc cac gcg cag    2152
   Ala Ile Glu Ala Ser Ile Glu Thr Lys Val Leu Lys Val His Ala Gln
       225                 230                 235
   gct gga gct ttc gca gaa agt att ttg tca agc tct cgg act gta cag    2200
   Ala Gly Ala Phe Ala Glu Ser Ile Leu Ser Ser Ser Arg Thr Val Gln
   240                 245                 250                 255
   gcg ttc gga ctt cgg aaa caa ctg cta cgc gac ttt gac aga ttc ttg    2248
   Ala Phe Gly Leu Arg Lys Gln Leu Leu Arg Asp Phe Asp Arg Phe Leu
                   260                 265                 270
   caa gct tca agg aat ctc gga aac aag aaa agc cct ctt ttt ggc tgt    2296
   Gln Ala Ser Arg Asn Leu Gly Asn Lys Lys Ser Pro Leu Phe Gly Cys
               275                 280                 285
   cta ttt gcc acg gag tac agc atc atc ttt gca ggg ttt ggc ttg tgc    2344
   Leu Phe Ala Thr Glu Tyr Ser Ile Ile Phe Ala Gly Phe Gly Leu Cys
           290                 295                 300
   ttt tgg cag ggt ata aaa atg gta tcg act ggg gag gtc gcg caa tca    2392
   Phe Trp Gln Gly Ile Lys Met Val Ser Thr Gly Glu Val Ala Gln Ser
       305                 310                 315
   gga gat att ttc acgtttgttc agtctcagtc attttacagc tggcgatgct        2444
   Gly Asp Ile Phe
   320
   aatcatggta attgc agt gtg ctc atg tct gtc gtt gtg gca gct aca agc   2495
                    Thr Val Leu Met Ser Val Val Val Ala Ala Thr Ser
                        325                 330                 335
   ttg aca tct ata ctg cct tac gtg att gat ttt gtc cgc gct gcc tct    2543
   Leu Thr Ser Ile Leu Pro Tyr Val Ile Asp Phe Val Arg Ala Ala Ser
                   340                 345                 350
   gca gcg gca gag cta ttc cag ctc atg gac cgg acg tct agg att gat    2591
   Ala Ala Ala Glu Leu Phe Gln Leu Met Asp Arg Thr Ser Arg Ile Asp
               355                 360                 365
   ccg ttt gat gag tcc gga gaa aga ccg aca gac gtt acg gga tat ctc    2639
   Pro Phe Asp Glu Ser Gly Glu Arg Pro Thr Asp Val Thr Gly Tyr Leu
                   370                 375                 380
   gat ttt ggt ggc att tcg ttt gcc tac cct acg cgc cct gac gtc aag     2687
   Asp Phe Gly Gly Ile Ser Phe Ala Tyr Pro Thr Arg Pro Asp Val Lys
       385                 390                 395
   gtg ttg aat gat ttc tca ttg cat atc cct gcc ggg aag aca act gcg     2735
   Val Leu Asn Asp Phe Ser Leu His Ile Pro Ala Gly Lys Thr Thr Ala
   400                 405                 410                 415
   ctc gtc gga gca agc ggg tct ggt aaa agc acc atc gtc ggg ctg atc     2783
   Leu Val Gly Ala Ser Gly Ser Gly Lys Ser Thr Ile Val Gly Leu Ile
                   420                 425                 430
   gaa cgt tgg tac gat cct ttg tcg gga acg atc aag ctt gac ggc cag     2831
   Glu Arg Trp Tyr Asp Pro Leu Ser Gly Thr Ile Lys Leu Asp Gly Gln
               435                 440                 445
   act atc gag acg ttc aac ctc aaa tgg cta cgc cag cag gtt aga ctt     2879
   Thr Ile Glu Thr Phe Asn Leu Lys Trp Leu Arg Gln Gln Val Arg Leu
           450                 455                 460
   gtt caa cag gag ccc gtt ttg ttc gct gga aca gtt gct gag aat atc     2927
   Val Gln Gln Glu Pro Val Leu Phe Ala Gly Thr Val Ala Glu Asn Ile
       465                 470                 475
   gcc aat ggc ctt gtg ggc aca ccg tgg gag aat gaa tcc gca gcc gag     2975
   Ala Asn Gly Leu Val Gly Thr Pro Trp Glu Asn Glu Ser Ala Ala Glu
   480                 485                 490                 495
   aag cag gcc cgg ata cag gac gct gct aaa att gct ttc gcc cat gat     3023
   Lys Gln Ala Arg Ile Gln Asp Ala Ala Lys Ile Ala Phe Ala His Asp
                   500                 505                 510
   ttc atc acg gaa ctt ccc aat gga tat gat aca gtc att gga gag aga     3071
   Phe Ile Thr Glu Leu Pro Asn Gly Tyr Asp Thr Val Ile Gly Glu Arg
               515                 520                 525
   ggt ggg cta tta tcc ggt ggt caa aaa cag cgg gtt gca att gct cgc     3119
   Gly Gly Leu Leu Ser Gly Gly Gln Lys Gln Arg Val Ala Ile Ala Arg
           530                 535                 540
   agt att gtt tcc cag ccc cga att ctt ctt ctg gat gaa gca acg agt     3167
   Ser Ile Val Ser Gln Pro Arg Ile Leu Leu Leu Asp Glu Ala Thr Ser
       545                 550                 555
   gca ctt gat cct cat gcc gag gaa gtt gtg caa cgg gca cta aac aat     3215
   Ala Leu Asp Pro His Ala Glu Glu Val Val Gln Arg Ala Leu Asn Asn
   560                 565                 570                 575
   gtt tcg aag gga cgc aca acg atc acc atc gca cac aag cta gcc acc     3263
   Val Ser Lys Gly Arg Thr Thr Ile Thr Ile Ala His Lys Leu Ala Thr
                   580                 585                 590
   ata aga gat gct cat aat att gtc gtc atg gaa aat ggc cgt att ctc     3311
   Ile Arg Asp Ala His Asn Ile Val Val Met Glu Asn Gly Arg Ile Leu
               595                 600                 605
   gaa cag ggc act cat aaa tcg ctt ttg gat tcg aat ggc gcc tac gca     3359
   Glu Gln Gly Thr His Lys Ser Leu Leu Asp Ser Asn Gly Ala Tyr Ala
           610                 615                 620
   cgc cta gtg caa gcc cag gat ttg tct gtg gcc gcg cag aac ccc gaa    3407
   Arg Leu Val Gln Ala Gln Asp Leu Ser Val Ala Ala Gln Asn Pro Glu
       625                 630                 635
   gat gtg tcg aat agc tcg gat gaa aca gat aag gaa gac cat gtc aca    3455
   Asp Val Ser Asn Ser Ser Asp Glu Thr Asp Lys Glu Asp His Val Thr
   640                 645                 650                 655
   ctg act ggt gaa ttg acc cgg tgc tca act atg acg aga tca gct ttg    3503
   Leu Thr Gly Glu Leu Thr Arg Cys Ser Thr Met Thr Arg Ser Ala Leu
                   660                 665                 670
   gag aaa cgg ttg agt cgg gat gat ttt gac aac tgg aaa aga ttg ggg    3551
   Glu Lys Arg Leu Ser Arg Asp Asp Phe Asp Asn Trp Lys Arg Leu Gly
               675                 680                 685
   ctt ctc cac aca gtc tgg aga ttg gtg aaa tcg acc cct gag cta aat    3599
   Leu Leu His Thr Val Trp Arg Leu Val Lys Ser Thr Pro Glu Leu Asn
           690                 695                 700
   tgg act ttt gcg att ctg att ctg tct tgt ctc gct gga ggtgaggcta     3648
   Trp Thr Phe Ala Ile Leu Ile Leu Ser Cys Leu Ala Gly
       705                 710                 715
   gcatttttaa gcccaattcc cgcgaactga gactgaccaa attgta gct gcg tct     3703
                                                      Ala Ala Ser
   ttc cct ggc caa gct att ttg atg tcc agg ttt atc gaa gta ttt cag    3751
   Phe Pro Gly Gln Ala Ile Leu Met Ser Arg Phe Ile Glu Val Phe Gln
   720                 725                 730                 735
   ttc aca ggt gca gct atg cgg acg aaa ggg aac ttt ttc gct ctc atg    3799
   Phe Thr Gly Ala Ala Met Arg Thr Lys Gly Asn Phe Phe Ala Leu Met
                   740                 745                 750
   ttc ttg gtt ctg gga ctt gga gct ttc gtc gtc tac ttc gtt gtt ggg    3847
   Phe Leu Val Leu Gly Leu Gly Ala Phe Val Val Tyr Phe Val Val Gly
               755                 760                 765
   tgg aca tca aat gtt gtg gca cag gtgagtctct tccagtgatg tatgagtata   3901
   Trp Thr Ser Asn Val Val Ala Gln
           770                 775
   tcttggcttg actaactcat ccag aca atg aac cat aaa tac cgg aaa caa     3952
                              Thr Met Asn His Lys Tyr Arg Lys Gln
                                                  780
   gtc gtt aat gac atg atg agg caa gac ctg caa ttc ttt gat cgc gcc    4000
   Val Val Asn Asp Met Met Arg Gln Asp Leu Gln Phe Phe Asp Arg Ala
   785                 790                 795                 800
   gag aac acc aca ggg gca cta acc agc cgtgccgact cttatccaca          4047
   Glu Asn Thr Thr Gly Ala Leu Thr Ser
                   805
   ggctgtgttt gagcttatgg ggtttaatgt tgctctgata tggta gca acg gtc ggt 4104
                                                     Pro Thr Val Gly
                                                     810
   gta ttg tca tgc tcc gtc cta gca ctg gca tat gca tgg aag ctc ggt    4152
   Val Leu Ser Cys Ser Val Leu Ala Leu Ala Tyr Ala Trp Lys Leu Gly
       815                 820                 825
   ctt gtt att atc tta gca gga ctt cca ccc atg ctc ctt tct ggt tac    4200
   Leu Val Ile Ile Leu Ala Gly Leu Pro Pro Met Leu Leu Ser Gly Tyr
   830                 835                 840                 845
   gct cgc atc aga atg gag ggt gca atg gac cac aaa att tcg aag atg    4248
   Ala Arg Ile Arg Met Glu Gly Ala Met Asp His Lys Ile Ser Lys Met
                   850                 855                 860
   ttt tgc gga agc gca tcc att gcc tca gaa gcc gtg acg gct atc cgg    4296
   Phe Cys Gly Ser Ala Ser Ile Ala Ser Glu Ala Val Thr Ala Ile Arg
               865                 870                 875
   acg gtg tcc tcc ttg gcg att gag acg tca gtt ttg gag cga tac acc    4344
   Thr Val Ser Ser Leu Ala Ile Glu Thr Ser Val Leu Glu Arg Tyr Thr
           880                 885                 890
   aag gag ctt gac cga gcg aat gcg aca tct acg aag cca gtg ctt ctc    4392
   Lys Glu Leu Asp Arg Ala Asn Ala Thr Ser Thr Lys Pro Val Leu Leu
       895                 900                 905
   atc atg ctc cct ttc gca ttc act cag act gtt gaa tac tct ttc ctg    4440
   Ile Met Leu Pro Phe Ala Phe Thr Gln Thr Val Glu Tyr Ser Phe Leu
   910                 915                 920                 925
   gct ctc ggt ttc tggtatgttt gtcgtatatg ttccatatgc tcgaagtggc        4492
   Ala Leu Gly Phe
   ttgttctcgg tttgatgaca ggatgctaac caccggatcg tctct agg tat gga tgc 4549
                                                     Trp Tyr Gly Cys
                                                     930
   cgt ctt gtc tca ttt gga gat gtg agc atg gtg aac ttt ttc atc agt    4597
   Arg Leu Val Ser Phe Gly Asp Val Ser Met Val Asn Phe Phe Ile Ser
       935                 940                 945
   ttc ttg agt gtg ttt ttt tct ggc cag caa gct aca atc ctt ttt ggc    4645
   Phe Leu Ser Val Phe Phe Ser Gly Gln Gln Ala Thr Ile Leu Phe Gly
   950                 955                 960                 965
   ttc tca agc agt aag atg cat tcc aaa cct gta tgg gag gcg aaa        4690
   Phe Ser Ser Ser Lys Met His Ser Lys Pro Val Trp Glu Ala Lys
                   970                 975                 980
   gtgagttgct aatagttata caggcatgac aaaggcaaca aatgctgcca attacatatt 4750
   ttggctggag gagctccag cct acc att cga gag acc gac gaa aac cgt gac   4802
                        Pro Thr Ile Arg Glu Thr Asp Glu Asn Arg Asp
                                        985                 990
   att ggc cct ggc gac ctt aag tct ctg cac ttg gaa agc ctg caa ttt    4850
   Ile Gly Pro Gly Asp Leu Lys Ser Leu His Leu Glu Ser Leu Gln Phe
               995                 1000                1005
   tct tac cca atg aga cct cac gct cgt gta cta cgt ggc att gac ctt    4898
   Ser Tyr Pro Met Arg Pro His Ala Arg Val Leu Arg Gly Ile Asp Leu
           1010                1015                1020
   cac gtatgtcagc ccaacttgtc ctcttttcaa tcctgtcctg tggtctaccc         4951
   His
   acttgacgcc ataaacctgt ccttgttcgc tgctaatata ttgtatag atc gaa aaa  5008
                                                       Ile Glu Lys
                                                       1025
   gaa caa ttt gtc gca ttc gtc ggc gca tct ggc tgt gga aaa agt acg   5056
   Glu Gln Phe Val Ala Phe Val Gly Ala Ser Gly Cys Gly Lys Ser Thr
           1030                1035                1040
   atg att agc atg ctc gaa cgc ttt tac gat cca gta gcc ggt cac att   5104
   Met Ile Ser Met Leu Glu Arg Phe Tyr Asp Pro Val Ala Gly His Ile
       1045                1050                1055
   aaa atc gac gca ctt aca ttg gat agc ttg aat ccg tgg ctg tat cga   5152
   Lys Ile Asp Ala Leu Thr Leu Asp Ser Leu Asn Pro Trp Leu Tyr Arg
   1060                1065                1070                1075
   ggt caa gtg gca ctt gtg cag cag gaa ccc acc ctc tat ccc ggc acc   5200
   Gly Gln Val Ala Leu Val Gln Gln Glu Pro Thr Leu Tyr Pro Gly Thr
                   1080                1085                1090
   att cgc gaa aac gtc gcc atg ggg aca ccg act gat gac gga tca acg   5248
   Ile Arg Glu Asn Val Ala Met Gly Thr Pro Thr Asp Asp Gly Ser Thr
               1095                1100                1105
   gtt cct gac agt gat att gaa gct gca tgt cgg gct gcc aac gcg tgg   5296
   Val Pro Asp Ser Asp Ile Glu Ala Ala Cys Arg Ala Ala Asn Ala Trp
           1110                1115                1120
   gaa ttt atc tct tct ctg ccc gat ggc ctt atg act ctc tgt ggg gcg   5344
   Glu Phe Ile Ser Ser Leu Pro Asp Gly Leu Met Thr Leu Cys Gly Ala
       1125                1130                1135
   aat ggg aca cag ctg tct ggt ggg caa cgt cag cgc atc gcc att gcc   5392
   Asn Gly Thr Gln Leu Ser Gly Gly Gln Arg Gln Arg Ile Ala Ile Ala
   1140                1145                1150                1155
   agg gcg cta ctc aga aat cca cgt tta ctc cta ctt gat gaa gca aca   5440
   Arg Ala Leu Leu Arg Asn Pro Arg Leu Leu Leu Leu Asp Glu Ala Thr
                   1160                1165                1170
   agt gcg ttg gat act cag tcg gag aga att gtc caa gat gcg ctg aat   5488
   Ser Ala Leu Asp Thr Gln Ser Glu Arg Ile Val Gln Asp Ala Leu Asn
               1175                1180                1185
   gag gct gct tcc cat ggg gat aga att acg atc gcg gtt gcc cat cgc   5536
   Glu Ala Ala Ser His Gly Asp Arg Ile Thr Ile Ala Val Ala His Arg
           1190                1195                1200
   ttg tct act att cgc cat gcc gat atg att tgc gtt ttc gac ggg ggc   5584
   Leu Ser Thr Ile Arg His Ala Asp Met Ile Cys Val Phe Asp Gly Gly
       1205                1210                1215
   agg atc gct gag tct gga act cat gag gaa tta ctg atg aaa ggc cgt   5632
   Arg Ile Ala Glu Ser Gly Thr His Glu Glu Leu Leu Met Lys Gly Arg
   1220                1225                1230                1235
   ctt tat ccg aaa atg tgc gag gct cag aac ctc ggc act taatgaagca    5681
   Leu Tyr Pro Lys Met Cys Glu Ala Gln Asn Leu Gly Thr
                   1240                1245
   tcctatcttc tgctccttcc aaagccatga gtttgaatat gttatatatg tacgtacttg 5741
   gaataatcga acgcgatgat ggtctgatta gtagatcagc gcagatagat aatgtaattt 5801
   aactgaagcg aatatatact caaagccttt cgactattcc cccatataca atgtccaaaa 5861
   gccccatgta aaataacgtc ctcctccccg ggttcgctgc cgtctttcgg gtcagcgagc 5921
   cagctcagcg gcaattgcat acaaccattg aaggggcaaa tccttgatcc gataaagttc 5981
   gggatggact ccgtttctcg tccaactatc aattcttatg agccagtctt cttagactca 6041
   tcgggtatag aggggcctcg gacaacccca agattccgcg tcagatcggg tagtagctac 6101
   aagcctcgcg aacgaaatgc cacagactaa agatatgaag gcgtctacaa gccgtcagat 6161
   ggccttaata tagtaatcga taaggctgtt gagggatata gtaagtatct gagggtaggg 6221
   cagtaagtct ggacagaagc cctcgaaacc ttcatctcgc ctgttctcgc tttccaaaaa 6281
   gccgataatt tgattctgct gagacaagct atcatccgat tcctggattg tcaggcatag 6341
   taaccaggtc acttctccag ggctccaggg ctagcgtgta tttcagttgc ttctactggt 6401
   actcggatta caatgtagca gcttcctgta ccgggctcag ccactgtgta gggcccaaag 6461
   atattgtctc cttccctcat tgaccgactt cggtatcggt ggaacgggtg atgaagggcg 6521
   atttggatct ctaatatgtc tgaatttgct cataggtagc cagcattgcc caagttacca 6581
   caatactccg ccccgtattg attcaaaacg gagagagctt ctttaagatg actttgcaac 6641
   atgtcctctc ggacttcgat cccgttgcga gcaagttcag atagtgtgga tccagcaacc 6701
   tctaacaaaa taagcccagg aattccgttg agcgatcctt cactaaaagg ctgcgaaatg 6761
   actgttcctt gaaggtcctc cggtgtcttg tagacttctt tctggcgctg aaattcgggg 6821
   acccatccgt ccttctgttg cttaataaat gatcgacttc acattgtgag gtagagcatt 6881
   gtgacatgtg gatggcccag tctcaaggcg gagtacagtg ggacgtcgtt gatctagagc 6941
   cttgagcgtt cgcacggcga cgcgctcgcc atgtagataa acaatctaat ccctattaag 7001
   cttgataact tgtcaggtct agaagatata gcttacactc gaaagatatg attcaggcat 7061
   aggcgcgttg gggatggaag gcagaaaaag agaaacaaac aaggggacat aaaaagcctg 7121
   cggagttccc agacctccaa gggccagggc tccgtctccc catggtctcc ctaggtaggt 7181
   aggggcccgg ggtaggcggc tgattactaa ggcaggaata gtgctggcca gccctggggc 7241
   attatcatga ttcataatgc attgaaatat gattaaaaag tgctggcaat agtctcaaat 7301
   cagtacactg tcaagcaact cacggcgcca ttcttgagga cttcaaataa aagccccggg 7361
   aacccaaaaa gctcggtgtt gcccaacccc ccatcctgcc catctgcacc accccgagga 7421
   cctttggccg tggtctcctc ccagccacca agaaactcaa cgctactctc agggctttct 7481
   ccccagccac ggttcagcac cattggccgt tgcagagcac gctcctcggc ggggctggca 7541
   taagatcctt tccaagtagt acgcagccgt tctgagactt catcataggg cgattccgaa 7601
   gtcagacagt cgccagattc atccaaggga attagccagc gacttcgccg gtgacgatta 7661
   aatggataag agcccggcac gttcccatgg agaataatct tggtcactgt cagctgggaa 7721
   gagggtggct gaggcgatgg atc 7744
<210> 10
   <211> 1248
   <212> PRT
   <213> Penicillium chrysogenum
   <223> Translation of the complete genomic sequence of Penicillium chrysogenum ABC transporter aa1
<400> 10
   Met Glu Lys Glu Ser Val Lys Tyr Arg Ser Asp Gly Gln Gly Ala Glu
   1 5 10 15
   Glu Gln Thr Asn Ala Phe Asn Gly Tyr Leu Val Val Ser Pro Pro Pro
   20 25 30
   Ile Ala Gly Leu Lys Ala Asn Ile Arg Lys Arg Ile Phe Gln Tyr Gly
   35 40 45
   Ser Ser Lys Val Tyr Thr Met Gln Gly Ile Ala Thr Ala Cys Ala Leu
   50 55 60
   Gly Ser Gly Val Gly Met Ala Leu Val Asn Leu Val Phe Gly Gln Phe
   65 70 75 80
   Ile Thr Val Ile Thr Asp Tyr Thr Ser Gly Arg Ile Asp Pro Ala Arg
   85 90 95
   Phe Arg Gly Glu Ala Gly Arg Leu Ser Leu Tyr Phe Phe Val Ile Gly
   100 105 110
   Leu Cys Arg Phe Phe Leu Thr Tyr Gly Tyr Ser Thr Leu Phe Thr Ile
   115 120 125
   Ala Ala Tyr Arg Ile Thr Arg Asn Ile Arg Arg Leu Tyr Leu Lys Ala
   130 135 140
   Gly Leu Thr Gln Glu Ile Ala Phe Phe Asp Ser Gly Ala Ser Gly Ser
   145 150 155 160
   Ile Ala Val Gln Ala Thr Ser Asn Gly Arg Leu Ile Gln Ser Gly Ile
   165 170 175
   Ser Glu Lys Leu Gly Leu Val Val Gln Gly Leu Ser Thr Phe Ile Ser
   180 185 190
   Ala Phe Val Leu Ala Phe Val Thr His Trp Lys Leu Thr Leu Ile Cys
   195 200 205
   Cys Cys Ile Ala Pro Ala Met Leu Leu Ala Leu Gly Ile Ser Ser Ala
   210 215 220
   Ile Glu Ala Ser Ile Glu Thr Lys Val Leu Lys Val His Ala Gln Ala
   225                 230                 235                 240
   Gly Ala Phe Ala Glu Ser Ile Leu Ser Ser Ser Arg Thr Val Gln Ala
                   245                 250                 255
   Phe Gly Leu Arg Lys Gln Leu Leu Arg Asp Phe Asp Arg Phe Leu Gln
               260                 265                 270
   Ala Ser Arg Asn Leu Gly Asn Lys Lys Ser Pro Leu Phe Gly Cys Leu
           275                 280                 285
   Phe Ala Thr Glu Tyr Ser Ile Ile Phe Ala Gly Phe Gly Leu Cys Phe
       290                 295                 300
   Trp Gln Gly Ile Lys Met Val Ser Thr Gly Glu Val Ala Gln Ser Gly
   305                 310                 315                 320
   Asp Ile Phe Thr Val Leu Met Ser Val Val Val Ala Ala Thr Ser Leu
                   325                 330                 335
   Thr Ser Ile Leu Pro Tyr Val Ile Asp Phe Val Arg Ala Ala Ser Ala
               340                 345                 350
   Ala Ala Glu Leu Phe Gln Leu Met Asp Arg Thr Ser Arg Ile Asp Pro
           355                 360                 365
   Phe Asp Glu Ser Gly Glu Arg Pro Thr Asp Val Thr Gly Tyr Leu Asp
       370                 375                 380
   Phe Gly Gly Ile Ser Phe Ala Tyr Pro Thr Arg Pro Asp Val Lys Val
   385                 390                 395                 400
   Leu Asn Asp Phe Ser Leu His Ile Pro Ala Gly Lys Thr Thr Ala Leu
                   405                 410                 415
   Val Gly Ala Ser Gly Ser Gly Lys Ser Thr Ile Val Gly Leu Ile Glu
               420                 425                 430
   Arg Trp Tyr Asp Pro Leu Ser Gly Thr Ile Lys Leu Asp Gly Gln Thr
           435                 440                 445
   Ile Glu Thr Phe Asn Leu Lys Trp Leu Arg Gln Gln Val Arg Leu Val
       450                 455                 460
   Gln Gln Glu Pro Val Leu Phe Ala Gly Thr Val Ala Glu Asn Ile Ala
   465                 470                 475                 480
   Asn Gly Leu Val Gly Thr Pro Trp Glu Asn Glu Ser Ala Ala Glu Lys
                   485                 490                 495
   Gln Ala Arg Ile Gln Asp Ala Ala Lys Ile Ala Phe Ala His Asp Phe
               500                 505                 510
   Ile Thr Glu Leu Pro Asn Gly Tyr Asp Thr Val Ile Gly Glu Arg Gly
           515                 520                 525
   Gly Leu Leu Ser Gly Gly Gln Lys Gln Arg Val Ala Ile Ala Arg Ser
       530                 535                 540
   Ile Val Ser Gln Pro Arg Ile Leu Leu Leu Asp Glu Ala Thr Ser Ala
   545 550 555 560
   Leu Asp Pro His Ala Glu Glu Val Val Gln Arg Ala Leu Asn Asn Val
   565 570 575
   Ser Lys Gly Arg Thr Thr Ile Thr Ile Ala His Lys Leu Ala Thr Ile
   580 585 590
   Arg Asp Ala His Asn Ile Val Val Met Glu Asn Gly Arg Ile Leu Glu
   595 600 605
   Gln Gly Thr His Lys Ser Leu Leu Asp Ser Asn Gly Ala Tyr Ala Arg
   610 615 620
   Leu Val Gln Ala Gln Asp Leu Ser Val Ala Ala Gln Asn Pro Glu Asp
   625 630 635 640
   Val Ser Asn Ser Ser Asp Glu Thr Asp Lys Glu Asp His Val Thr Leu
   645 650 655
   Thr Gly Glu Leu Thr Arg Cys Ser Thr Met Thr Arg Ser Ala Leu Glu
   660 665 670
   Lys Arg Leu Ser Arg Asp Asp Phe Asp Asn Trp Lys Arg Leu Gly Leu
   675 680 685
   Leu His Thr Val Trp Arg Leu Val Lys Ser Thr Pro Glu Leu Asn Trp
   690 695 700
   Thr Phe Ala Ile Leu Ile Leu Ser Cys Leu Ala Gly Ala Ala Ser Phe
   705 710 715 720
   Pro Gly Gln Ala Ile Leu Met Ser Arg Phe Ile Glu Val Phe Gln Phe
   725 730 735
   Thr Gly Ala Ala Met Arg Thr Lys Gly Asn Phe Phe Ala Leu Met Phe
   740 745 750
   Leu Val Leu Gly Leu Gly Ala Phe Val Val Tyr Phe Val Val Gly Trp
   755 760 765
   Thr Ser Asn Val Val Ala Gln Thr Met Asn His Lys Tyr Arg Lys Gln
   770 775 780
   Val Val Asn Asp Met Met Arg Gln Asp Leu Gln Phe Phe Asp Arg Ala
   785 790 795 800
   Glu Asn Thr Thr Gly Ala Leu Thr Ser Ala Thr Val Gly Val Leu Ser
   805 810 815
   Cys Ser Val Leu Ala Leu Ala Tyr Ala Trp Lys Leu Gly Leu Val Ile
   820 825 830
   Ile Leu Ala Gly Leu Pro Pro Met Leu Leu Ser Gly Tyr Ala Arg Ile
   835 840 845
   Arg Met Glu Gly Ala Met Asp His Lys Ile Ser Lys Met Phe Cys Gly
   850 855 860
   Ser Ala Ser Ile Ala Ser Glu Ala Val Thr Ala Ile Arg Thr Val Ser
   865 870 875 880
   Ser Leu Ala Ile Glu Thr Ser Val Leu Glu Arg Tyr Thr Lys Glu Leu
   885 890 895
   Asp Arg Ala Asn Ala Thr Ser Thr Lys Pro Val Leu Leu Ile Met Leu
   900 905 910
   Pro Phe Ala Phe Thr Gln Thr Val Glu Tyr Ser Phe Leu Ala Leu Gly
   915 920 925
   Phe Arg Tyr Gly Cys Arg Leu Val Ser Phe Gly Asp Val Ser Met Val
   930 935 940
   Asn Phe Phe Ile Ser Phe Leu Ser Val Phe Phe Ser Gly Gln Gln Ala
   945 950 955 960
   Thr Ile Leu Phe Gly Phe Ser Ser Ser Lys Met His Ser Lys Pro Val
   965 970 975
   Trp Glu Ala Lys Pro Thr Ile Arg Glu Thr Asp Glu Asn Arg Asp Ile
   980 985 990
   Gly Pro Gly Asp Leu Lys Ser Leu His Leu Glu Ser Leu Gln Phe Ser
   995 1000 1005
   Tyr Pro Met Arg Pro His Ala Arg Val Leu Arg Gly Ile Asp Leu His
   1010 1015 1020
   Ile Glu Lys Glu Gln Phe Val Ala Phe Val Gly Ala Ser Gly Cys Gly
   025 1030 1035 1040
   Lys Ser Thr Met Ile Ser Met Leu Glu Arg Phe Tyr Asp Pro Val Ala
   1045 1050 1055
   Gly His Ile Lys Ile Asp Ala Leu Thr Leu Asp Ser Leu Asn Pro Trp
   1060 1065 1070
   Leu Tyr Arg Gly Gln Val Ala Leu Val Gln Gln Glu Pro Thr Leu Tyr
   1075 1080 1085
   Pro Gly Thr Ile Arg Glu Asn Val Ala Met Gly Thr Pro Thr Asp Asp
   1090 1095 1100
   Gly Ser Thr Val Pro Asp Ser Asp Ile Glu Ala Ala Cys Arg Ala Ala
   105 1110 1115 1120
   Asn Ala Trp Glu Phe Ile Ser Ser Leu Pro Asp Gly Leu Met Thr Leu
   1125 1130 1135
   Cys Gly Ala Asn Gly Thr Gln Leu Ser Gly Gly Gln Arg Gln Arg Ile
   1140 1145 1150
   Ala Ile Ala Arg Ala Leu Leu Arg Asn Pro Arg Leu Leu Leu Leu Asp
   1155 1160 1165
   Glu Ala Thr Ser Ala Leu Asp Thr Gln Ser Glu Arg Ile Val Gln Asp
   1170 1175 1180
   Ala Leu Asn Glu Ala Ala Ser His Gly Asp Arg Ile Thr Ile Ala Val
   185 1190 1195 1200
   Ala His Arg Leu Ser Thr Ile Arg His Ala Asp Met Ile Cys Val Phe
                   1205                1210                1215
   Asp Gly Gly Arg Ile Ala Glu Ser Gly Thr His Glu Glu Leu Leu Met
               1220                1225                1230
   Lys Gly Arg Leu Tyr Pro Lys Met Cys Glu Ala Gln Asn Leu Gly Thr
           1235                1240                1245
<210> 11
   <211> 387
   <212> DNA
   <213> Penicillium chrysogenum
<220>
   <223> Partial cDNA sequence of Penicillium chrysogenum ABC transporter aa3
<400> 11
   tcggggtcgg ggaaaagtac cctcagccaa ctactgatgc ggttctactt gccaacctca 60
   ggtgagatcc taattgacgg cactccaatg cagcaactcg atatcaactg gatccgcaac 120
   aacattaccc ttttagagca aaagagtgtt ctcttcaatg agtctgtcct cacaaacatt 180
   gcttttggtc gccaggacca tgccaccgtc accaagcaag atgtcaaaga acctattgag 240
   ttggccatgc ttgagaacac tatcgatggt ctccctaaag gaatttacac atgcgttggc 300
   ccgggtggaa gctttttgag tggtggccaa agacagcgag ttgcaattgc cagagcgaaa 360
   ttacgtgaca ccccaatcct gatccaa                                    387
<210> 12
   <211> 387
   <212> DNA
   <213> Penicillium chrysogenum
<220>
   <221> CDS
   <222> (1)..(387)
   <223> Translation of the cDNA sequence of Penicillium chrysogenum ABC transporter aa3 from SEQ ID NO. 11
<400> 12
   tcg ggg tcg ggg aaa agt acc ctc agc caa cta ctg atg cgg ttc tac    48
   Ser Gly Ser Gly Lys Ser Thr Leu Ser Gln Leu Leu Met Arg Phe Tyr
    1               5                  10                  15
   ttg cca acc tca ggt gag atc cta att gac ggc act cca atg cag caa    96
   Leu Pro Thr Ser Gly Glu Ile Leu Ile Asp Gly Thr Pro Met Gln Gln
                   20                  25                  30
   ctc gat atc aac tgg atc cgc aac aac att acc ctt tta gag caa aag    144
   Leu Asp Ile Asn Trp Ile Arg Asn Asn Ile Thr Leu Leu Glu Gln Lys
               35                  40                  45
   agt gtt ctc ttc aat gag tct gtc ctc aca aac att gct ttt ggt cgc    192
   Ser Val Leu Phe Asn Glu Ser Val Leu Thr Asn Ile Ala Phe Gly Arg
           50                  55                  60
   cag gac cat gcc acc gtc acc aag caa gat gtc aaa gaa cct att gag    240
   Gln Asp His Ala Thr Val Thr Lys Gln Asp Val Lys Glu Pro Ile Glu
   65                  70                  75                  80
   ttg gcc atg ctt gag aac act atc gat ggt ctc cct aaa gga att tac    288
   Leu Ala Met Leu Glu Asn Thr Ile Asp Gly Leu Pro Lys Gly Ile Tyr
                   85                  90                  95
   aca tgc gtt ggc ccg ggt gga agc ttt ttg agt ggt ggc caa aga cag    336
   Thr Cys Val Gly Pro Gly Gly Ser Phe Leu Ser Gly Gly Gln Arg Gln
               100                 105                 110
   cga gtt gca att gcc aga gcg aaa tta cgt gac acc cca atc ctg atc    384
   Arg Val Ala Ile Ala Arg Ala Lys Leu Arg Asp Thr Pro Ile Leu Ile
           115                 120                 125
   caa                                                                387
   Gln
<210> 13
   <211> 129
   <212> PRT
   <213> Penicillium chrysogenum
   <223> Protein sequence of the Penicillium chrysogenum ABC transporter aa3 (derived from the DNA sequence as shown in (SEQ ID NO. 11))
<400> 13
   Ser Gly Ser Gly Lys Ser Thr Leu Ser Gln Leu Leu Met Arg Phe Tyr
   1 5 10 15
   Leu Pro Thr Ser Gly Glu Ile Leu Ile Asp Gly Thr Pro Met Gln Gln
   20 25 30
   Leu Asp Ile Asn Trp Ile Arg Asn Asn Ile Thr Leu Leu Glu Gln Lys
   35 40 45
   Ser Val Leu Phe Asn Glu Ser Val Leu Thr Asn Ile Ala Phe Gly Arg
   50 55 60
   Gln Asp His Ala Thr Val Thr Lys Gln Asp Val Lys Glu Pro Ile Glu
   65 70 75 80
   Leu Ala Met Leu Glu Asn Thr Ile Asp Gly Leu Pro Lys Gly Ile Tyr
   85 90 95
   Thr Cys Val Gly Pro Gly Gly Ser Phe Leu Ser Gly Gly Gln Arg Gln
   100 105 110
   Arg Val Ala Ile Ala Arg Ala Lys Leu Arg Asp Thr Pro Ile Leu Ile
   115 120 125
   Gln
<210> 14
   <211> 408
   <212> DNA
   <213> Penicillium chrysogenum
<220>
   <223> Partial cDNA sequence of Penicillium chrysogenum ABC transporter aa4
<400> 14
   tcgggggcgg ggaaaacgac tttgcttgat gtattggcgc accgcacatc aatgggcgtt 60
   atcaccggtg atatgtttgt caatggccat ggactggatc agagtttcca gcgaaagaca 120
   ggttatgttc agcaacagga tcttcacctc gataccgcta ctgtgcgtga gtctctgcga 180
   ttcagtgcta tgttgcgtca gccagcctcc gtatcggtta aggagaagta cgactatgtt 240
   gaggacgtca tcaaaatgtt gaagatggag gagtttgccg aagccattgt tggtgtccca 300
   ggtgagggtt tgaatgtcga acagcgcaag ctgttgacta taggtgtcga attggccgcc 360
   aaacccaagc tgctactctt tttggatgag gccacctccg gcctcgaa 408
<210> 15
   <211> 408
   <212> DNA
   <213> Penicillium chrysogenum
<220>
   <221> CDS
   <222> (1)..(408)
   <223> Translation of the cDNA sequence of Penicillium chrysogenum ABC transporter aa4 from SEQ ID NO. 14
<400> 15
   tcg ggg gcg ggg aaa acg act ttg ctt gat gta ttg gcg cac cgc aca 48
   Ser Gly Ala Gly Lys Thr Thr Leu Leu Asp Val Leu Ala His Arg Thr
   1 5 10 15
   tca atg ggc gtt atc acc ggt gat atg ttt gtc aat ggc cat gga ctg 96
   Ser Met Gly Val Ile Thr Gly Asp Met Phe Val Asn Gly His Gly Leu
   20 25 30
   gat cag agt ttc cag cga aag aca ggt tat gtt cag caa cag gat ctt 144
   Asp Gln Ser Phe Gln Arg Lys Thr Gly Tyr Val Gln Gln Gln Asp Leu
   35 40 45
   cac ctc gat acc gct act gtg cgt gag tct ctg cga ttc agt gct atg 192
   His Leu Asp Thr Ala Thr Val Arg Glu Ser Leu Arg Phe Ser Ala Met
   50 55 60
   ttg cgt cag cca gcc tcc gta tcg gtt aag gag aag tac gac tat gtt 240
   Leu Arg Gln Pro Ala Ser Val Ser Val Lys Glu Lys Tyr Asp Tyr Val
   65 70 75 80
   gag gac gtc atc aaa atg ttg aag atg gag gag ttt gcc gaa gcc att 288
   Glu Asp Val Ile Lys Met Leu Lys Met Glu Glu Phe Ala Glu Ala Ile
   85 90 95
   gtt ggt gtc cca ggt gag ggt ttg aat gtc gaa cag cgc aag ctg ttg 336
   Val Gly Val Pro Gly Glu Gly Leu Asn Val Glu Gln Arg Lys Leu Leu
   100 105 110
   act ata ggt gtc gaa ttg gcc gcc aaa ccc aag ctg cta ctc ttt ttg 384
   Thr Ile Gly Val Glu Leu Ala Ala Lys Pro Lys Leu Leu Leu Phe Leu
   115 120 125
   gat gag gcc acc tcc ggc ctc gaa 408
   Asp Glu Ala Thr Ser Gly Leu Glu
   130 135
<210> 16
   <211> 136
   <212> PRT
   <213> Penicillium chrysogenum
   <223> Protein sequence of the Penicillium chrysogenum ABC transporter aa4 (derived from the DNA sequence as shown in SEQ ID NO. 14)
<400> 16
   Ser Gly Ala Gly Lys Thr Thr Leu Leu Asp Val Leu Ala His Arg Thr
    1               5                  10                  15
   Ser Met Gly Val Ile Thr Gly Asp Met Phe Val Asn Gly His Gly Leu
               20                  25                  30
   Asp Gln Ser Phe Gln Arg Lys Thr Gly Tyr Val Gln Gln Gln Asp Leu
           35                  40                  45
   His Leu Asp Thr Ala Thr Val Arg Glu Ser Leu Arg Phe Ser Ala Met
       50                  55                  60
   Leu Arg Gln Pro Ala Ser Val Ser Val Lys Glu Lys Tyr Asp Tyr Val
   65                  70                  75                  80
   Glu Asp Val Ile Lys Met Leu Lys Met Glu Glu Phe Ala Glu Ala Ile
                   85                  90                  95
   Val Gly Val Pro Gly Glu Gly Leu Asn Val Glu Gln Arg Lys Leu Leu
               100                 105                 110
   Thr Ile Gly Val Glu Leu Ala Ala Lys Pro Lys Leu Leu Leu Phe Leu
           115                 120                 125
   Asp Glu Ala Thr Ser Gly Leu Glu
       130                 135
<210> 17
   <211> 402
   <212> DNA
   <213> Penicillium chrysogenum
<220>
   <223> Partial cDNA sequence of Penicillium chrysogenum ABC transporter aa5
<400> 17
   tcgggggcgg ggaagacgac acttcttgat gtgcttgctc agcgtaagac agatggtact 60
   atcaacggct ctatcatggt tgatggccgt gagctcccag tgtctttcca gaggatggcg 120
   ggatactgcg aacaactaga tgttcacgag ccatacgcaa ccgtgagaga agcattagaa 180
   ttctctgccc ttcttcgcca gtctcgcaac acccctaaag cagataagct caagtatgtc 240
   gacaccatca ttgatcttct cgagctcgat gacctggcag acactcttat cggtactatt 300
   gggaatggac ttagcgttga gcaaagaaag cgtgtcacaa ttggcgtgga actggtatcc 360
   aagcctagca ttctgatctt cttggacgaa cccaaatcga at                    402
<210> 18
   <211> 7529
   <212> DNA
   <213> Penicillium chrysogenum
<220>
   <223> Complete genomic DNA sequence of Penicillium chrysogenum ABC transporter aa5
<400> 18
   cgaattcttc tcctggatct cgcaatttaa ccttcctcaa ttgattaagg attttcgccg 60
   aaagccacca ctcaagtcaa tcttgtcaga cagtcatgga tgtgtcaagc ccggagaaat 120
   gttgcttgtg ttgggccgcc ctggttcggg gtgcaccacg ttgctcaaga tgctgtcaaa 180
   tcgccgcgaa ggttatcaca ccgtgaatgg tgacgtacgt ttcggaagca tgtcccccaa 240
   ggaagctgag gactataatg gccagattgt gatgaacaca gaagaagagc tcttctatcc 300
   acgtttgaca gtaggccaga ccatggactt tgcggccaga ctcaaggttc ctttccatct 360
   accggaggga gtccaaagtg ttgacgaata cacagcggag accaaacagt ttcttctgga 420
   gtctatggga atcagccata cggcagatac caaggttgga aatgagtttg tccgtggtgt 480
   atcgggtggt gagcggaagc gagtctctat tatcgagtgt ctggcgacta ggggttctgt 540
   ttactcctgg gataacagta cccgtggttt ggatgccagt accgcattgg aatgggctaa 600
   ggcacttcgc gctatgactg atgttctcgg cctttccacc attgtgaccc tatatcaagc 660
   gggaaatgga atttataatc tcttcgacaa ggtccttgtc cttgacgagg gaaagcagat 720
   ctactatggt cctgcggctg cagctaagcc tttcatggaa gacctaggct tcgtgtacac 780
   tgatggagca aacatcggta cgtatttcct acatctgaca accaagccta gtatcaacta 840
   acaaaaccat aggcgacttc ctcaccggtg tgacagtccc gacagagcgg aaaatcagac 900
   ccggatacga aaacacattc cccagaaacg ccgatgccat tttggccgaa tacaaaaagt 960
   ccagcattta tgaccgtatg gtctcaacat atgactatcc cgactccaac ctcagccgcg 1020
   aacgcacaga tgccttcaaa gagtctgttg cgtgggaaaa gtccagccac ctgcctaagg 1080
   gtagtagtct gaccaccagc ttttgggttc agctcattgc atgcaccaag cgacagtatc 1140
   agattctgtg gggtgagaag tcaacattca tcatcaaaca ggttctgtca tgtgtgatgg 1200
   cgttgatcgc tggttcttgc ttctacgact cgccagacac ttccgcaggt ctcttcacca 1260
   agggtggcgc tgtattcttc tcgcttttgt acaactgcat cgttgccatg tctgaggtca 1320
   ctgaatcctt taagggccgt ccggtcttgg tcaaacacaa gggctttgct atgtaccacc 1380
   cggctgcgtt ttccttggct cagatcatgg cggatttccc ggtgctgttg ttccagtgca 1440
   ctatcttctc ggttgtcatc tattggatgt ctggcttgaa acatactgcg gctgctttct 1500
   ttaccttctg gattattttg ttcacaacga ctctggtaag tagtcgtttt cttattcgat 1560
   tctgacaggt tgtcgacttg gactaactct tacatctttc agtgcatcac cgctcttttc 1620
   agatttatcg gatccgcttt cagtacattc gaagccgcgt ctaaaatctc gggaactgca 1680
   gtcaagggca ttgtcatgta cgcaggttac atgattccca agccccagat gaagaactgg 1740
   ttcctcgaac tgtactacac gaaccccttt gcctatgcct tccaagctgc catgtcgaac 1800
   gagttccatg ggcgccatat cccttgtgtt ggtaacaacc tgatcccaag tggccctggc 1860
   tacgaggaag tcggagccga gaaccaagct tgtgcgggtg ttggtggtgc tttgccaggc 1920
   gcaaactatg tgaccggcga tcagtacctt ggatcacttc actacaagca ctctcaaatg 1980
   tggcggaact tcggtgtcgt ctggggctgg tggggattct tcgccatcct gacaattgtt 2040
   ttcacatcct actggaaagc gggtgctggc gctggctctt ctcttcttat tccccgcgag 2100
   aagctcaagc agcatcatgc cgccgtctcc gacgaagaag cacaaaataa cgaaaagtct 2160
   actacgcgcg agactcccga cgagcccatt caggttgacg atgaaaatct caaccgaaac 2220
   acatcgatat ttacctggaa gaacctcacg tatacagtcc aaacccccac gggcgaccga 2280
   gtgctgttgg ataacattca cggatgggtc aagcccggca tgctaggcgc ccttatgggc 2340
   tcctctggtg cgggaaagac aacacttctt gatgtgcttg ctcagcgtaa gacagatggt 2400
   actatcaacg gctctatcat ggttgatggc cgtgagctcc cagtgtcttt ccagaggatg 2460
   gcgggatact gcgaacaact agatgttcac gagccatacg caaccgtgag agaagcatta 2520
   gaattctctg cccttcttcg ccagtctcgc aacaccccta aagcagataa gctcaagtat 2580
   gtcgacacca tcattgatct tctcgagctc gatgacctgg cagacactct tatcggtact 2640
   attgggaatg gacttagcgt tgagcaaaga aagcgtgtca caattggcgt ggaactggta 2700
   tccaagccta gcattctgat cttcttggac gagcccacct ctggtttgga tggacaatcg 2760
   gcctataaca ccgttcgttt ccttcgcaaa ttggctgatg ttggtcaagc tgttttggta 2820
   ggtcttctat ttctccaatt caacaactac aagaccattt tgctgacaag tgacattcta 2880
   ggttacgatt caccagcctt cggcgcagct ctttgctcaa ttcgacactc tccttcttct 2940
   tgcaaagggc ggaaagactg tctactttgg cgacattggc gataatgcgg cctgcgttaa 3000
   gcaatacttt ggccaatatg gggcccagtg ccctaccgat gctaacgcag ctgaatttat 3060
   gattgacgtt gttaccggtg gtatcgaatc tgtcaaggac aaggactggc atcagatatg 3120
   gctcgactct cctgagcaga ctcgcatgat cgcggaactg gaccgaatga ttgcggatgc 3180
   tgctagcaag ccccccggaa ctgtcgatga tggattcgaa ttctccatgc ctctttggga 3240
   gcagaccaaa atcgtcactc atcggatgaa tgtcgcgctt ttccgaaaca ccaactatgt 3300
   caacaacaag ttctcccttc acattatctc agccatgctc aatgggttct ctttctggcg 3360
   tcctggccca agtgtgtcag ctttgaacct gaagatgttc acgatcttca actttgtctt 3420
   cgtcgctccc ggtgtcatca accagcttca accgctcttc atccagcgcc gcgatatcta 3480
   cgatactcgc gagaagaagt cgaagatgta ctcctgggtt gccttcgtta ccggattgat 3540
   tgtctccgag ttcccgtatc tctgcatctg tgcggttctc tactttgtct gctggtacta 3600
   ccccgtctgg agactgcctc atgagtcgag ccgatctggt gccacattct tcatgatgtt 3660
   gatctacgag ctcatttaca ctggtattgg tcaattcgtc gccgcctatt cacctaaccc 3720
   gacctttgct gcgcttgtca accccttgat catcagtact cttgtcctgt tctgcggtat 3780
   tttcgtccca tacatccagc tcaacgtgtt ctggcggtac tggatgtact acctcaatcc 3840
   cttcaactac gttgtctcgg gtatgttgac cttcggcctc tggggtgcca aggttacctg 3900
   caatgaagat gaatttgctt tgttcgagcc gctcaacggc accacctgcg cgcagtacct 3960
   gtcggattat atgtccggcg ctggctcaag catcaatttg gtcaaccccg atgctacctc 4020
   ggcctgcaag gtctgccagt atacagacgg aagtgatttc ctgagaaatc tcaatatcat 4080
   gaactacacc accggatgga gagatatcgg tatctcggtc atcttcgcca tcagtggata 4140
   tgccttggta tttggactca tgaagttgag aaccaaggct tccaagaagg ccgagtaggt 4200
   tgatcgtttc atcatttttc tccaagccct tcatttcttc ggaatgctca ttgattgagc 4260
   cttttccttc tccgatcttt cttttgccat tctctggtta tttcttgtta acctgttata 4320
   caacatacac tcacttcgcg gcctttcacg catgaagccc tacaccgcgc atttctgcgt 4380
   tttctgtcat cgagaacttt tccctcccct gcatacagtg acggtgcata agtttggaca 4440
   cccccctacc ccacccacta atattatgta atattctgcc ggtccaaaac ctccggattt 4500
   ccattgacat ctacctagta cggtgtatac cagccctgac tttcaatgat agcttccacc 4560
   cgatgcggca ttgtagcaga taggttctct atatgcttcc aatccagcag attccatgct 4620
   tcttgcgcag tagcgattag caggtccttt gtttcatcgg tattacgcat atgaatcagc 4680
   tcaggtcgga gctcatatat cttctgcttt aacaacgacc aaaggttctc gatcgggttt 4740
   aaatctggag atataggtgg ccaatctata acctcgattt gcatctcatc tagagcctcc 4800
   ctaacaatga ttgcagtgtg cgaaggcgca ttatcatact gaaaaatgct atcctcgtgc 4860
   acaatcaatg taggtaggat tcgacgatat aactctttaa taacaaagcg gttaataccc 4920
   cccctcttag attaagcgtc tccaaaaagg ggaattaaac cagttcgccg gaaatcacca 4980
   caaaaagcac cctaaaacat ctgcttcact tgcttaccct tatagggtaa gccttgacat 5040
   tgcccctacc gaggctggtc ccgcgggcgt ataaaagtct attcttactt aacaccaata 5100
   ccacgttcaa tagagcactc attagaccaa tagactcggc cccaatcctt aggtgtaaag 5160
   tgacgatact tcagagccca ttgtagacgt ttaaccgcgt gagcttcggt taagtaaggc 5220
   cgccaagagc aacgccactt ccgtatattc ttagcattta aaaggcgtcg aattgagtca 5280
   tatttaacct atataaaagg ttagaaagga gtatataata gcataaagta gtgttatact 5340
   aactttatac gataccttag caagcatatc ctcacgagtg attttcgggt tcttagcgat 5400
   agtatcaagc agtttctcac gatcagcttc gtccagcttc ctagggcgtc cagtacgggg 5460
   cttcgtgaca ccgccgattc tctcacgatc gcgtttaatg gtataacgaa ccccagagat 5520
   agaaatgaaa ggatatcgct tctgaattcg tttatacccc cacccagcag cgctatgaag 5580
   ctcacagatt cgctctcgta gtgctggggt aagctcacta cgcggagcca tagtgaaaat 5640
   gggaggtgta gggggtaaaa ccgcgtagag acgcgagatc atactagcta tactagcgtg 5700
   ttagggcgtg ttaggatagg cggtagggag ggcggtgagg gtgacccact atagggggtg 5760
   tccaaactta tgcaccgtca ctgtacatgt taatgttact aggtagcata tagattttag 5820
   aacatagagc atagaatata gattgtacat tctcggacct ttatacaaga gcctctacgt 5880
   gatcaagtta cagtgtttat gcacatgact ctatgtaatt gaaataacca cgagtctaaa 5940
   aggaagtgag taaagccata cgacgtatat atattcgctg agtagtcaca tcgacgtata 6000
   ctacaaccaa cccgtattcc atttctccga gttagccgcc catattggaa catgtactcc 6060
   gtactccgta ctccatactt cctgagccct acctgtctcc acaatgtgat gaaattgcat 6120
   cgacactagg gccaacgagc tggcgcgtgg ggatgtatcc atttgtgcac tggagacatt 6180
   atccggggct agaagtgtga cacatatttc accttcccga tcaggtagtg gaaaggtaaa 6240
   cccaaaatac aaaggaatct tacaccgatg gactatctcc acaagcgaaa gggatctctc 6300
   gagtggaaat gatggggtcg acccagcatc gtgggccagc cttggagaac gggaccattt 6360
   gctttcctag aatatcaccg atcattagct aacgcttgca agcttacacc gacaacatgg 6420
   aacctcctgt catagttgaa ttccatcgag tacggagtat ggagtagctt acccccaatc 6480
   ttcccgcgga aaccgcgaaa tcggaataaa tcccacattc tccgacgaga tgcacgtggg 6540
   tgggaactct ccacagtgac agaatccgat tcggcgctgt gaagaactcg ggatctgcgc 6600
   acgagctggt ctgcagaaac tctggtgaaa tttaatggtt tttgggtttt tgaaaaaaaa 6660
   aaatgaaaaa aaaggaaaat ttttgcttcc ccttttctat ttctcgtttc ttcgaagatt 6720
   tggttggcag aggctggggt ttcttatcac atctcacaca aaatactggt attaccgtat 6780
   ttgcccagga aggattccaa ggttgctgat cgctcggcaa atctcctttc atagaaacct 6840
   ggccctaagc ggggagagcc attgaaagat ggggagtaat ccgtaagtcc gagcttgtta 6900
   aacaagataa gagtccacgg gtatgttgtc atagtctcag ggaattgagg gcatgttctt 6960
   cgttcccgat ttcacagaga aacggtcctt gtttcccgcg tctattccgt cacgtaatcc 7020
   agttaagcaa agatcagcag tgatagtgga gtgaaaaagg cgtgtttctt ggttctcatt 7080
   agaaccaaaa gccaaagctc cgccgtgagc acttctgtgt ggtccgtacg gagtagatac 7140
   cgggctttaa actagaccac ttgattcccg atgcagtggt ccactggagt aaatagttga 7200
   ctccagagaa tattccattt ccggctaaat gggaccgttg ctagaacaac caacgacgaa 7260
   cactgttgag gattccttgt gtatctgagt ttaggagaaa gcgaattaaa aaagggttgc 7320
   cgtcgaaacc acaaagtaac cggttcggct tttcggcttg cctacaatgt tgtagggaag 7380
   cttcaagccc ttccaaggtt atccccacag aatgacgcag taaccataac tgtccatgag 7440
   ttgcgccagg taaggctatg tatagagaag acttcggagt acggagtaga actctgggta 7500
   tgtcggaaac agtggagttg atgcgatcc                                  7529
<210> 19
   <211> 7529
   <212> DNA
   <213> Penicillium chrysogenum
<220>
   <221> CDS
   <222> (119)..(796)
<220>
   <221> CDS
   <222> (852)..(1535)
<220>
   <221> CDS
   <222> (1603)..(2862)
<220>
   <221> CDS
   <222> (2882)..(4195)
<220>
   <223> Translation of the complete genomic DNA sequence of Penicillium chrysogenum ABC transporter aa5
<400> 19
   cgaattcttc tcctggatct cgcaatttaa ccttcctcaa ttgattaagg attttcgccg 60
   aaagccacca ctcaagtcaa tcttgtcaga cagtcatgga tgtgtcaagc ccggagaa   118
   atg ttg ctt gtg ttg ggc cgc cct ggt tcg ggg tgc acc acg ttg ctc   166
   Met Leu Leu Val Leu Gly Arg Pro Gly Ser Gly Cys Thr Thr Leu Leu
     1               5                  10                  15
   aag atg ctg tca aat cgc cgc gaa ggt tat cac acc gtg aat ggt gac   214
   Lys Met Leu Ser Asn Arg Arg Glu Gly Tyr His Thr Val Asn Gly Asp
                20                  25                  30
   gta cgt ttc gga agc atg tcc ccc aag gaa gct gag gac tat aat ggc   262
   Val Arg Phe Gly Ser Met Ser Pro Lys Glu Ala Glu Asp Tyr Asn Gly
            35                  40                  45
   cag att gtg atg aac aca gaa gaa gag ctc ttc tat cca cgt ttg aca   310
   Gln Ile Val Met Asn Thr Glu Glu Glu Leu Phe Tyr Pro Arg Leu Thr
        50                  55                  60
   gta ggc cag acc atg gac ttt gcg gcc aga ctc aag gtt cct ttc cat   358
   Val Gly Gln Thr Met Asp Phe Ala Ala Arg Leu Lys Val Pro Phe His
    65                 70                  75                  80
   cta ccg gag gga gtc caa agt gtt gac gaa tac aca gcg gag acc aaa   406
   Leu Pro Glu Gly Val Gln Ser Val Asp Glu Tyr Thr Ala Glu Thr Lys
                   85                  90                  95
   cag ttt ctt ctg gag tct atg gga atc agc cat acg gca gat acc aag   454
   Gln Phe Leu Leu Glu Ser Met Gly Ile Ser His Thr Ala Asp Thr Lys
               100                 105                 110
   gtt gga aat gag ttt gtc cgt ggt gta tcg ggt ggt gag cgg aag cga   502
   Val Gly Asn Glu Phe Val Arg Gly Val Ser Gly Gly Glu Arg Lys Arg
           115                 120                 125
   gtc tct att atc gag tgt ctg gcg act agg ggt tct gtt tac tcc tgg   550
   Val Ser Ile Ile Glu Cys Leu Ala Thr Arg Gly Ser Val Tyr Ser Trp
       130                 135                 140
   gat aac agt acc cgt ggt ttg gat gcc agt acc gca ttg gaa tgg gct   598
   Asp Asn Ser Thr Arg Gly Leu Asp Ala Ser Thr Ala Leu Glu Trp Ala
   145                 150                 155                 160
   aag gca ctt cgc gct atg act gat gtt ctc ggc ctt tcc acc att gtg   646
   Lys Ala Leu Arg Ala Met Thr Asp Val Leu Gly Leu Ser Thr Ile Val
                   165                 170                 175
   acc cta tat caa gcg gga aat gga att tat aat ctc ttc gac aag gtc   694
   Thr Leu Tyr Gln Ala Gly Asn Gly Ile Tyr Asn Leu Phe Asp Lys Val
               180                 185                 190
   ctt gtc ctt gac gag gga aag cag atc tac tat ggt cct gcg gct gca   742
   Leu Val Leu Asp Glu Gly Lys Gln Ile Tyr Tyr Gly Pro Ala Ala Ala
           195                 200                 205
   gct aag cct ttc atg gaa gac cta ggc ttc gtg tac act gat gga gca   790
   Ala Lys Pro Phe Met Glu Asp Leu Gly Phe Val Tyr Thr Asp Gly Ala
       210                 215                 220
   aac atc ggtacgtatt tcctacatct gacaaccaag cctagtatca actaacaaaa   846
   Asn Ile
   225
   ccata ggc gac ttc ctc acc ggt gtg aca gtc ccg aca gag cgg aaa atc 896
         Gly Asp Phe Leu Thr Gly Val Thr Val Pro Thr Glu Arg Lys Ile
                   230                 235                 240
   aga ccc gga tac gaa aac aca ttc ccc aga aac gcc gat gcc att ttg   944
   Arg Pro Gly Tyr Glu Asn Thr Phe Pro Arg Asn Ala Asp Ala Ile Leu
               245                 250                 255
   gcc gaa tac aaa aag tcc agc att tat gac cgt atg gtc tca aca tat   992
   Ala Glu Tyr Lys Lys Ser Ser Ile Tyr Asp Arg Met Val Ser Thr Tyr
           260                 265                 270
   gac tat ccc gac tcc aac ctc agc cgc gaa cgc aca gat gcc ttc aaa   1040
   Asp Tyr Pro Asp Ser Asn Leu Ser Arg Glu Arg Thr Asp Ala Phe Lys
       275                 280                 285
   gag tct gtt gcg tgg gaa aag tcc agc cac ctg cct aag ggt agt agt   1088
   Glu Ser Val Ala Trp Glu Lys Ser Ser His Leu Pro Lys Gly Ser Ser
   290                 295                 300                 305
   ctg acc acc agc ttt tgg gtt cag ctc att gca tgc acc aag cga cag    1136
   Leu Thr Thr Ser Phe Trp Val Gln Leu Ile Ala Cys Thr Lys Arg Gln
                   310                 315                 320
   tat cag att ctg tgg ggt gag aag tca aca ttc atc atc aaa cag gtt    1184
   Tyr Gln Ile Leu Trp Gly Glu Lys Ser Thr Phe Ile Ile Lys Gln Val
               325                 330                 335
   ctg tca tgt gtg atg gcg ttg atc gct ggt tct tgc ttc tac gac tcg    1232
   Leu Ser Cys Val Met Ala Leu Ile Ala Gly Ser Cys Phe Tyr Asp Ser
           340                 345                 350
   cca gac act tcc gca ggt ctc ttc acc aag ggt ggc gct gta ttc ttc    1280
   Pro Asp Thr Ser Ala Gly Leu Phe Thr Lys Gly Gly Ala Val Phe Phe
       355                 360                 365
   tcg ctt ttg tac aac tgc atc gtt gcc atg tct gag gtc act gaa tcc    1328
   Ser Leu Leu Tyr Asn Cys Ile Val Ala Met Ser Glu Val Thr Glu Ser
   370                 375                 380                 385
   ttt aag ggc cgt ccg gtc ttg gtc aaa cac aag ggc ttt gct atg tac    1376
   Phe Lys Gly Arg Pro Val Leu Val Lys His Lys Gly Phe Ala Met Tyr
                   390                 395                 400
   cac ccg gct gcg ttt tcc ttg gct cag atc atg gcg gat ttc ccg gtg    1424
   His Pro Ala Ala Phe Ser Leu Ala Gln Ile Met Ala Asp Phe Pro Val
               405                 410                 415
   ctg ttg ttc cag tgc act atc ttc tcg gtt gtc atc tat tgg atg tct    1472
   Leu Leu Phe Gln Cys Thr Ile Phe Ser Val Val Ile Tyr Trp Met Ser
           420                 425                 430
   ggc ttg aaa cat act gcg gct gct ttc ttt acc ttc tgg att att ttg    1520
   Gly Leu Lys His Thr Ala Ala Ala Phe Phe Thr Phe Trp Ile Ile Leu
       435                 440                 445
   ttc aca acg act ctg gtaagtagtc gttttcttat tcgattctga caggttgtcg   1575
   Phe Thr Thr Thr Leu
   450
   acttggacta actcttacat ctttcag tgc atc acc gct ctt ttc aga ttt atc 1629
                                 Cys Ile Thr Ala Leu Phe Arg Phe Ile
                                 455                 460
   gga tcc gct ttc agt aca ttc gaa gcc gcg tct aaa atc tcg gga act    1677
   Gly Ser Ala Phe Ser Thr Phe Glu Ala Ala Ser Lys Ile Ser Gly Thr
       465                 470                 475
   gca gtc aag ggc att gtc atg tac gca ggt tac atg att ccc aag ccc    1725
   Ala Val Lys Gly Ile Val Met Tyr Ala Gly Tyr Met Ile Pro Lys Pro
   480                 485                 490                 495
   cag atg aag aac tgg ttc ctc gaa ctg tac tac acg aac ccc ttt gcc    1773
   Gln Met Lys Asn Trp Phe Leu Glu Leu Tyr Tyr Thr Asn Pro Phe Ala
                   500                 505                 510
   tat gcc ttc caa gct gcc atg tcg aac gag ttc cat ggg cgc cat atc    1821
   Tyr Ala Phe Gln Ala Ala Met Ser Asn Glu Phe His Gly Arg His Ile
               515                 520                 525
   cct tgt gtt ggt aac aac ctg atc cca agt ggc cct ggc tac gag gaa    1869
   Pro Cys Val Gly Asn Asn Leu Ile Pro Ser Gly Pro Gly Tyr Glu Glu
           530                 535                 540
   gtc gga gcc gag aac caa gct tgt gcg ggt gtt ggt ggt gct ttg cca    1917
   Val Gly Ala Glu Asn Gln Ala Cys Ala Gly Val Gly Gly Ala Leu Pro
       545                 550                 555
   ggc gca aac tat gtg acc ggc gat cag tac ctt gga tca ctt cac tac    1965
   Gly Ala Asn Tyr Val Thr Gly Asp Gln Tyr Leu Gly Ser Leu His Tyr
   560                 565                 570                     575
   aag cac tct caa atg tgg cgg aac ttc ggt gtc gtc tgg ggc tgg tgg    2013
   Lys His Ser Gln Met Trp Arg Asn Phe Gly Val Val Trp Gly Trp Trp
                   580                 585                 590
   gga ttc ttc gcc atc ctg aca att gtt ttc aca tcc tac tgg aaa gcg    2061
   Gly Phe Phe Ala Ile Leu Thr Ile Val Phe Thr Ser Tyr Trp Lys Ala
               595                 600                 605
   ggt gct ggc gct ggc tct tct ctt ctt att ccc cgc gag aag ctc aag    2109
   Gly Ala Gly Ala Gly Ser Ser Leu Leu Ile Pro Arg Glu Lys Leu Lys
           610                 615                 620
   cag cat cat gcc gcc gtc tcc gac gaa gaa gca caa aat aac gaa aag    2157
   Gln His His Ala Ala Val Ser Asp Glu Glu Ala Gln Asn Asn Glu Lys
       625                 630                 635
   tct act acg cgc gag act ccc gac gag ccc att cag gtt gac gat gaa    2205
   Ser Thr Thr Arg Glu Thr Pro Asp Glu Pro Ile Gln Val Asp Asp Glu
   640                 645                 650                     655
   aat ctc aac cga aac aca tcg ata ttt acc tgg aag aac ctc acg tat    2253
   Asn Leu Asn Arg Asn Thr Ser Ile Phe Thr Trp Lys Asn Leu Thr Tyr
                   660                 665                 670
   aca gtc caa acc ccc acg ggc gac cga gtg ctg ttg gat aac att cac    2301
   Thr Val Gln Thr Pro Thr Gly Asp Arg Val Leu Leu Asp Asn Ile His
               675                 680                 685
   gga tgg gtc aag ccc ggc atg cta ggc gcc ctt atg ggc tcc tct ggt    2349
   Gly Trp Val Lys Pro Gly Met Leu Gly Ala Leu Met Gly Ser Ser Gly
           690                 695                 700
   gcg gga aag aca aca ctt ctt gat gtg ctt gct cag cgt aag aca gat    2397
   Ala Gly Lys Thr Thr Leu Leu Asp Val Leu Ala Gln Arg Lys Thr Asp
       705                 710                 715
   ggt act atc aac ggc tct atc atg gtt gat ggc cgt gag ctc cca gtg    2445
   Gly Thr Ile Asn Gly Ser Ile Met Val Asp Gly Arg Glu Leu Pro Val
   720                 725                 730                     735
   tct ttc cag agg atg gcg gga tac tgc gaa caa cta gat gtt cac gag    2493
   Ser Phe Gln Arg Met Ala Gly Tyr Cys Glu Gln Leu Asp Val His Glu
                   740                 745                 750
   cca tac gca acc gtg aga gaa gca tta gaa ttc tct gcc ctt ctt cgc    2541
   Pro Tyr Ala Thr Val Arg Glu Ala Leu Glu Phe Ser Ala Leu Leu Arg
               755                 760                 765
   cag tct cgc aac acc cct aaa gca gat aag ctc aag tat gtc gac acc   2589
   Gln Ser Arg Asn Thr Pro Lys Ala Asp Lys Leu Lys Tyr Val Asp Thr
           770                 775                 780
   atc att gat ctt ctc gag ctc gat gac ctg gca gac act ctt atc ggt   2637
   Ile Ile Asp Leu Leu Glu Leu Asp Asp Leu Ala Asp Thr Leu Ile Gly
       785                 790                 795
   act att ggg aat gga ctt agc gtt gag caa aga aag cgt gtc aca att   2685
   Thr Ile Gly Asn Gly Leu Ser Val Glu Gln Arg Lys Arg Val Thr Ile
   800                 805                 810                 815
   ggc gtg gaa ctg gta tcc aag cct agc att ctg atc ttc ttg gac gag   2733
   Gly Val Glu Leu Val Ser Lys Pro Ser Ile Leu Ile Phe Leu Asp Glu
                   820                 825                 830
   ccc acc tct ggt ttg gat gga caa tcg gcc tat aac acc gtt cgt ttc   2781
   Pro Thr Ser Gly Leu Asp Gly Gln Ser Ala Tyr Asn Thr Val Arg Phe
               835                 840                 845
   ctt cgc aaa ttg gct gat gtt ggt caa gct gtt ttg gta ggt ctt cta   2829
   Leu Arg Lys Leu Ala Asp Val Gly Gln Ala Val Leu Val Gly Leu Leu
           850                 855                 860
   ttt ctc caa ttc aac aac tac aag acc att ttg ctgacaagtg acattctag  2881
   Phe Leu Gln Phe Asn Asn Tyr Lys Thr Ile Leu
       865                 870
   gtt acg att cac cag cct tcg gcg cag ctc ttt gct caa ttc gac act   2929
   Val Thr Ile His Gln Pro Ser Ala Gln Leu Phe Ala Gln Phe Asp Thr
   875                 880                 885                 890
   ctc ctt ctt ctt gca aag ggc gga aag act gtc tac ttt ggc gac att   2977
   Leu Leu Leu Leu Ala Lys Gly Gly Lys Thr Val Tyr Phe Gly Asp Ile
                   895                 900                 905
   ggc gat aat gcg gcc tgc gtt aag caa tac ttt ggc caa tat ggg gcc   3025
   Gly Asp Asn Ala Ala Cys Val Lys Gln Tyr Phe Gly Gln Tyr Gly Ala
               910                 915                 920
   cag tgc cct acc gat gct aac gca gct gaa ttt atg att gac gtt gtt   3073
   Gln Cys Pro Thr Asp Ala Asn Ala Ala Glu Phe Met Ile Asp Val Val
           925                 930                 935
   acc ggt ggt atc gaa tct gtc aag gac aag gac tgg cat cag ata tgg   3121
   Thr Gly Gly Ile Glu Ser Val Lys Asp Lys Asp Trp His Gln Ile Trp
       940                 945                 950
   ctc gac tct cct gag cag act cgc atg atc gcg gaa ctg gac cga atg   3169
   Leu Asp Ser Pro Glu Gln Thr Arg Met Ile Ala Glu Leu Asp Arg Met
   955                 960                 965                 970
   att gcg gat gct gct agc aag ccc ccc gga act gtc gat gat gga ttc   3217
   Ile Ala Asp Ala Ala Ser Lys Pro Pro Gly Thr Val Asp Asp Gly Phe
                   975                 980                 985
   gaa ttc tcc atg cct ctt tgg gag cag acc aaa atc gtc act cat cgg   3265
   Glu Phe Ser Met Pro Leu Trp Glu Gln Thr Lys Ile Val Thr His Arg
               990                 995                1000
   atg aat gtc gcg ctt ttc cga aac acc aac tat gtc aac aac aag ttc   3313
   Met Asn Val Ala Leu Phe Arg Asn Thr Asn Tyr Val Asn Asn Lys Phe
           1005                1010                1015
   tcc ctt cac att atc tca gcc atg ctc aat ggg ttc tct ttc tgg cgt     3361
   Ser Leu His Ile Ile Ser Ala Met Leu Asn Gly Phe Ser Phe Trp Arg
       1020                1025                1030
   cct ggc cca agt gtg tca gct ttg aac ctg aag atg ttc acg atc ttc     3409
   Pro Gly Pro Ser Val Ser Ala Leu Asn Leu Lys Met Phe Thr Ile Phe
   1035                1040                1045                1050
   aac ttt gtc ttc gtc gct ccc ggt gtc atc aac cag ctt caa ccg ctc     3457
   Asn Phe Val Phe Val Ala Pro Gly Val Ile Asn Gln Leu Gln Pro Leu
                   1055                1060                1065
   ttc atc cag cgc cgc gat atc tac gat act cgc gag aag aag tcg aag     3505
   Phe Ile Gln Arg Arg Asp Ile Tyr Asp Thr Arg Glu Lys Lys Ser Lys
               1070                1075                1080
   atg tac tcc tgg gtt gcc ttc gtt acc gga ttg att gtc tcc gag ttc     3553
   Met Tyr Ser Trp Val Ala Phe Val Thr Gly Leu Ile Val Ser Glu Phe
           1085                1090                1095
   ccg tat ctc tgc atc tgt gcg gtt ctc tac ttt gtc tgc tgg tac tac     3601
   Pro Tyr Leu Cys Ile Cys Ala Val Leu Tyr Phe Val Cys Trp Tyr Tyr
       1100                1105                1110
   ccc gtc tgg aga ctg cct cat gag tcg agc cga tct ggt gcc aca ttc     3649
   Pro Val Trp Arg Leu Pro His Glu Ser Ser Arg Ser Gly Ala Thr Phe
   1115                1120                1125                1130
   ttc atg atg ttg atc tac gag ctc att tac act ggt att ggt caa ttc     3697
   Phe Met Met Leu Ile Tyr Glu Leu Ile Tyr Thr Gly Ile Gly Gln Phe
                   1135                1140                1145
   gtc gcc gcc tat tca cct aac ccg acc ttt gct gcg ctt gtc aac ccc     3745
   Val Ala Ala Tyr Ser Pro Asn Pro Thr Phe Ala Ala Leu Val Asn Pro
               1150                1155                1160
   ttg atc atc agt act ctt gtc ctg ttc tgc ggt att ttc gtc cca tac     3793
   Leu Ile Ile Ser Thr Leu Val Leu Phe Cys Gly Ile Phe Val Pro Tyr
           1165                1170                1175
   atc cag ctc aac gtg ttc tgg cgg tac tgg atg tac tac ctc aat ccc     3841
   Ile Gln Leu Asn Val Phe Trp Arg Tyr Trp Met Tyr Tyr Leu Asn Pro
       1180                1185                1190
   ttc aac tac gtt gtc tcg ggt atg ttg acc ttc ggc ctc tgg ggt gcc     3889
   Phe Asn Tyr Val Val Ser Gly Met Leu Thr Phe Gly Leu Trp Gly Ala
   1195                1200                1205                1210
   aag gtt acc tgc aat gaa gat gaa ttt gct ttg ttc gag ccg ctc aac     3937
   Lys Val Thr Cys Asn Glu Asp Glu Phe Ala Leu Phe Glu Pro Leu Asn
                   1215                1220                1225
   ggc acc acc tgc gcg cag tac ctg tcg gat tat atg tcc ggc gct ggc     3985
   Gly Thr Thr Cys Ala Gln Tyr Leu Ser Asp Tyr Met Ser Gly Ala Gly
               1230                1235                1240
   tca agc atc aat ttg gtc aac ccc gat gct acc tcg gcc tgc aag gtc     4033
   Ser Ser Ile Asn Leu Val Asn Pro Asp Ala Thr Ser Ala Cys Lys Val
                   1245                1250                1255
   tgc cag tat aca gac gga agt gat ttc ctg aga aat ctc aat atc atg    4081
   Cys Gln Tyr Thr Asp Gly Ser Asp Phe Leu Arg Asn Leu Asn Ile Met
      1260                1265                1270
   aac tac acc acc gga tgg aga gat atc ggt atc tcg gtc atc ttc gcc    4129
   Asn Tyr Thr Thr Gly Trp Arg Asp Ile Gly Ile Ser Val Ile Phe Ala
   1275                1280                1285                1290
   atc agt gga tat gcc ttg gta ttt gga ctc atg aag ttg aga acc aag    4177
   Ile Ser Gly Tyr Ala Leu Val Phe Gly Leu Met Lys Leu Arg Thr Lys
                   1295                1300                1305
   gct tcc aag aag gcc gag taggttgatc gtttcatcat ttttctccaa           4225
   Ala Ser Lys Lys Ala Glu
               1310
   gcccttcatt tcttcggaat gctcattgat tgagcctttt ccttctccga tctttctttt 4285
   gccattctct ggttatttct tgttaacctg ttatacaaca tacactcact tcgcggcctt 4345
   tcacgcatga agccctacac cgcgcatttc tgcgttttct gtcatcgaga acttttccct 4405
   cccctgcata cagtgacggt gcataagttt ggacaccccc ctaccccacc cactaatatt 4465
   atgtaatatt ctgccggtcc aaaacctccg gatttccatt gacatctacc tagtacggtg 4525
   tataccagcc ctgactttca atgatagctt ccacccgatg cggcattgta gcagataggt 4585
   tctctatatg cttccaatcc agcagattcc atgcttcttg cgcagtagcg attagcaggt 4645
   cctttgtttc atcggtatta cgcatatgaa tcagctcagg tcggagctca tatatcttct 4705
   gctttaacaa cgaccaaagg ttctcgatcg ggtttaaatc tggagatata ggtggccaat 4765
   ctataacctc gatttgcatc tcatctagag cctccctaac aatgattgca gtgtgcgaag 4825
   gcgcattatc atactgaaaa atgctatcct cgtgcacaat caatgtaggt aggattcgac 4885
   gatataactc tttaataaca aagcggttaa taccccccct cttagattaa gcgtctccaa 4945
   aaaggggaat taaaccagtt cgccggaaat caccacaaaa agcaccctaa aacatctgct 5005
   tcacttgctt acccttatag ggtaagcctt gacattgccc ctaccgaggc tggtcccgcg 5065
   ggcgtataaa agtctattct tacttaacac caataccacg ttcaatagag cactcattag 5125
   accaatagac tcggccccaa tccttaggtg taaagtgacg atacttcaga gcccattgta 5185
   gacgtttaac cgcgtgagct tcggttaagt aaggccgcca agagcaacgc cacttccgta 5245
   tattcttagc atttaaaagg cgtcgaattg agtcatattt aacctatata aaaggttaga 5305
   aaggagtata taatagcata aagtagtgtt atactaactt tatacgatac cttagcaagc 5365
   atatcctcac gagtgatttt cgggttctta gcgatagtat caagcagttt ctcacgatca 5425
   gcttcgtcca gcttcctagg gcgtccagta cggggcttcg tgacaccgcc gattctctca 5485
   cgatcgcgtt taatggtata acgaacccca gagatagaaa tgaaaggata tcgcttctga 5545
   attcgtttat acccccaccc agcagcgcta tgaagctcac agattcgctc tcgtagtgct 5605
   ggggtaagct cactacgcgg agccatagtg aaaatgggag gtgtaggggg taaaaccgcg 5665
   tagagacgcg agatcatact agctatacta gcgtgttagg gcgtgttagg ataggcggta 5725
   gggagggcgg tgagggtgac ccactatagg gggtgtccaa acttatgcac cgtcactgta 5785
   catgttaatg ttactaggta gcatatagat tttagaacat agagcataga atatagattg 5845
   tacattctcg gacctttata caagagcctc tacgtgatca agttacagtg tttatgcaca 5905
   tgactctatg taattgaaat aaccacgagt ctaaaaggaa gtgagtaaag ccatacgacg 5965
   tatatatatt cgctgagtag tcacatcgac gtatactaca accaacccgt attccatttc 6025
   tccgagttag ccgcccatat tggaacatgt actccgtact ccgtactcca tacttcctga 6085
   gccctacctg tctccacaat gtgatgaaat tgcatcgaca ctagggccaa cgagctggcg 6145
   cgtggggatg tatccatttg tgcactggag acattatccg gggctagaag tgtgacacat 6205
   atttcacctt cccgatcagg tagtggaaag gtaaacccaa aatacaaagg aatcttacac 6265
   cgatggacta tctccacaag cgaaagggat ctctcgagtg gaaatgatgg ggtcgaccca 6325
   gcatcgtggg ccagccttgg agaacgggac catttgcttt cctagaatat caccgatcat 6385
   tagctaacgc ttgcaagctt acaccgacaa catggaacct cctgtcatag ttgaattcca 6445
   tcgagtacgg agtatggagt agcttacccc caatcttccc gcggaaaccg cgaaatcgga 6505
   ataaatccca cattctccga cgagatgcac gtgggtggga actctccaca gtgacagaat 6565
   ccgattcggc gctgtgaaga actcgggatc tgcgcacgag ctggtctgca gaaactctgg 6625
   tgaaatttaa tggtttttgg gtttttgaaa aaaaaaaatg aaaaaaaagg aaaatttttg 6685
   cttccccttt tctatttctc gtttcttcga agatttggtt ggcagaggct ggggtttctt 6745
   atcacatctc acacaaaata ctggtattac cgtatttgcc caggaaggat tccaaggttg 6805
   ctgatcgctc ggcaaatctc ctttcataga aacctggccc taagcgggga gagccattga 6865
   aagatgggga gtaatccgta agtccgagct tgttaaacaa gataagagtc cacgggtatg 6925
   ttgtcatagt ctcagggaat tgagggcatg ttcttcgttc ccgatttcac agagaaacgg 6985
   tccttgtttc ccgcgtctat tccgtcacgt aatccagtta agcaaagatc agcagtgata 7045
   gtggagtgaa aaaggcgtgt ttcttggttc tcattagaac caaaagccaa agctccgccg 7105
   tgagcacttc tgtgtggtcc gtacggagta gataccgggc tttaaactag accacttgat 7165
   tcccgatgca gtggtccact ggagtaaata gttgactcca gagaatattc catttccggc 7225
   taaatgggac cgttgctaga acaaccaacg acgaacactg ttgaggattc cttgtgtatc 7285
   tgagtttagg agaaagcgaa ttaaaaaagg gttgccgtcg aaaccacaaa gtaaccggtt 7345
   cggcttttcg gcttgcctac aatgttgtag ggaagcttca agcccttcca aggttatccc 7405
   cacagaatga cgcagtaacc ataactgtcc atgagttgcg ccaggtaagg ctatgtatag 7465
   agaagacttc ggagtacgga gtagaactct gggtatgtcg gaaacagtgg agttgatgcg 7525
   atcc 7529
<210> 20
   <211> 1312
   <212> PRT
   <213> Penicillium chrysogenum
   <223> Protein sequence of the Penicillium chrysogenum ABC transporter aa5 derived from the DNA sequence as shown in SEQ ID NO. 18
<400> 20
   Met Leu Leu Val Leu Gly Arg Pro Gly Ser Gly Cys Thr Thr Leu Leu
   1               5                   10                  15
   Lys Met Leu Ser Asn Arg Arg Glu Gly Tyr His Thr Val Asn Gly Asp
               20                  25                  30
   Val Arg Phe Gly Ser Met Ser Pro Lys Glu Ala Glu Asp Tyr Asn Gly
           35                  40                  45
   Gln Ile Val Met Asn Thr Glu Glu Glu Leu Phe Tyr Pro Arg Leu Thr
       50                  55                  60
   Val Gly Gln Thr Met Asp Phe Ala Ala Arg Leu Lys Val Pro Phe His
   65                  70                  75                  80
   Leu Pro Glu Gly Val Gln Ser Val Asp Glu Tyr Thr Ala Glu Thr Lys
                   85                  90                  95
   Gln Phe Leu Leu Glu Ser Met Gly Ile Ser His Thr Ala Asp Thr Lys
               100                 105                 110
   Val Gly Asn Glu Phe Val Arg Gly Val Ser Gly Gly Glu Arg Lys Arg
           115                 120                 125
   Val Ser Ile Ile Glu Cys Leu Ala Thr Arg Gly Ser Val Tyr Ser Trp
       130                 135                 140
   Asp Asn Ser Thr Arg Gly Leu Asp Ala Ser Thr Ala Leu Glu Trp Ala
   145                 150                 155                 160
   Lys Ala Leu Arg Ala Met Thr Asp Val Leu Gly Leu Ser Thr Ile Val
                   165                 170                 175
   Thr Leu Tyr Gln Ala Gly Asn Gly Ile Tyr Asn Leu Phe Asp Lys Val
               180                 185                 190
   Leu Val Leu Asp Glu Gly Lys Gln Ile Tyr Tyr Gly Pro Ala Ala Ala
           195                 200                 205
   Ala Lys Pro Phe Met Glu Asp Leu Gly Phe Val Tyr Thr Asp Gly Ala
       210                 215                 220
   Asn Ile Gly Asp Phe Leu Thr Gly Val Thr Val Pro Thr Glu Arg Lys
   225 230 235 240
   Ile Arg Pro Gly Tyr Glu Asn Thr Phe Pro Arg Asn Ala Asp Ala Ile
   245 250 255
   Leu Ala Glu Tyr Lys Lys Ser Ser Ile Tyr Asp Arg Met Val Ser Thr
   260 265 270
   Tyr Asp Tyr Pro Asp Ser Asn Leu Ser Arg Glu Arg Thr Asp Ala Phe
   275 280 285
   Lys Glu Ser Val Ala Trp Glu Lys Ser Ser His Leu Pro Lys Gly Ser
   290 295 300
   Ser Leu Thr Thr Ser Phe Trp Val Gln Leu Ile Ala Cys Thr Lys Arg
   305 310 315 320
   Gln Tyr Gln Ile Leu Trp Gly Glu Lys Ser Thr Phe Ile Ile Lys Gln
   325 330 335
   Val Leu Ser Cys Val Met Ala Leu Ile Ala Gly Ser Cys Phe Tyr Asp
   340 345 350
   Ser Pro Asp Thr Ser Ala Gly Leu Phe Thr Lys Gly Gly Ala Val Phe
   355 360 365
   Phe Ser Leu Leu Tyr Asn Cys Ile Val Ala Met Ser Glu Val Thr Glu
   370 375 380
   Ser Phe Lys Gly Arg Pro Val Leu Val Lys His Lys Gly Phe Ala Met
   385 390 395 400
   Tyr His Pro Ala Ala Phe Ser Leu Ala Gln Ile Met Ala Asp Phe Pro
   405 410 415
   Val Leu Leu Phe Gln Cys Thr Ile Phe Ser Val Val Ile Tyr Trp Met
   420 425 430
   Ser Gly Leu Lys His Thr Ala Ala Ala Phe Phe Thr Phe Trp Ile Ile
   435 440 445
   Leu Phe Thr Thr Thr Leu Cys Ile Thr Ala Leu Phe Arg Phe Ile Gly
   450 455 460
   Ser Ala Phe Ser Thr Phe Glu Ala Ala Ser Lys Ile Ser Gly Thr Ala
   465 470 475 480
   Val Lys Gly Ile Val Met Tyr Ala Gly Tyr Met Ile Pro Lys Pro Gln
   485 490 495
   Met Lys Asn Trp Phe Leu Glu Leu Tyr Tyr Thr Asn Pro Phe Ala Tyr
   500 505 510
   Ala Phe Gln Ala Ala Met Ser Asn Glu Phe His Gly Arg His Ile Pro
   515 520 525
   Cys Val Gly Asn Asn Leu Ile Pro Ser Gly Pro Gly Tyr Glu Glu Val
   530 535 540
   Gly Ala Glu Asn Gln Ala Cys Ala Gly Val Gly Gly Ala Leu Pro Gly
   545 550 555 560
   Ala Asn Tyr Val Thr Gly Asp Gln Tyr Leu Gly Ser Leu His Tyr Lys
   565 570 575
   His Ser Gln Met Trp Arg Asn Phe Gly Val Val Trp Gly Trp Trp Gly
   580 585 590
   Phe Phe Ala Ile Leu Thr Ile Val Phe Thr Ser Tyr Trp Lys Ala Gly
   595 600 605
   Ala Gly Ala Gly Ser Ser Leu Leu Ile Pro Arg Glu Lys Leu Lys Gln
   610 615 620
   His His Ala Ala Val Ser Asp Glu Glu Ala Gln Asn Asn Glu Lys Ser
   625 630 635 640
   Thr Thr Arg Glu Thr Pro Asp Glu Pro Ile Gln Val Asp Asp Glu Asn
   645 650 655
   Leu Asn Arg Asn Thr Ser Ile Phe Thr Trp Lys Asn Leu Thr Tyr Thr
   660 665 670
   Val Gln Thr Pro Thr Gly Asp Arg Val Leu Leu Asp Asn Ile His Gly
   675 680 685
   Trp Val Lys Pro Gly Met Leu Gly Ala Leu Met Gly Ser Ser Gly Ala
   690 695 700
   Gly Lys Thr Thr Leu Leu Asp Val Leu Ala Gln Arg Lys Thr Asp Gly
   705 710 715 720
   Thr Ile Asn Gly Ser Ile Met Val Asp Gly Arg Glu Leu Pro Val Ser
   725 730 735
   Phe Gln Arg Met Ala Gly Tyr Cys Glu Gln Leu Asp Val His Glu Pro
   740 745 750
   Tyr Ala Thr Val Arg Glu Ala Leu Glu Phe Ser Ala Leu Leu Arg Gln
   755 760 765
   Ser Arg Asn Thr Pro Lys Ala Asp Lys Leu Lys Tyr Val Asp Thr Ile
   770 775 780
   Ile Asp Leu Leu Glu Leu Asp Asp Leu Ala Asp Thr Leu Ile Gly Thr
   785 790 795 800
   Ile Gly Asn Gly Leu Ser Val Glu Gln Arg Lys Arg Val Thr Ile Gly
   805 810 815
   Val Glu Leu Val Ser Lys Pro Ser Ile Leu Ile Phe Leu Asp Glu Pro
   820 825 830
   Thr Ser Gly Leu Asp Gly Gln Ser Ala Tyr Asn Thr Val Arg Phe Leu
   835 840 845
   Arg Lys Leu Ala Asp Val Gly Gln Ala Val Leu Val Gly Leu Leu Phe
   850 855 860
   Leu Gln Phe Asn Asn Tyr Lys Thr Ile Leu Val Thr Ile His Gln Pro
   865 870 875 880
   Ser Ala Gln Leu Phe Ala Gln Phe Asp Thr Leu Leu Leu Leu Ala Lys
                   885                 890                 895
   Gly Gly Lys Thr Val Tyr Phe Gly Asp Ile Gly Asp Asn Ala Ala Cys
               900                 905                 910
   Val Lys Gln Tyr Phe Gly Gln Tyr Gly Ala Gln Cys Pro Thr Asp Ala
           915                 920                 925
   Asn Ala Ala Glu Phe Met Ile Asp Val Val Thr Gly Gly Ile Glu Ser
       930                 935                 940
   Val Lys Asp Lys Asp Trp His Gln Ile Trp Leu Asp Ser Pro Glu Gln
   945                 950                 955                 960
   Thr Arg Met Ile Ala Glu Leu Asp Arg Met Ile Ala Asp Ala Ala Ser
                   965                 970                 975
   Lys Pro Pro Gly Thr Val Asp Asp Gly Phe Glu Phe Ser Met Pro Leu
               980                 985                 990
   Trp Glu Gln Thr Lys Ile Val Thr His Arg Met Asn Val Ala Leu Phe
           995                 1000                1005
   Arg Asn Thr Asn Tyr Val Asn Asn Lys Phe Ser Leu His Ile Ile Ser
      1010                 1015                1020
   Ala Met Leu Asn Gly Phe Ser Phe Trp Arg Pro Gly Pro Ser Val Ser
   025                 1030                1035                1040
   Ala Leu Asn Leu Lys Met Phe Thr Ile Phe Asn Phe Val Phe Val Ala
                  1045                1050                1055
   Pro Gly Val Ile Asn Gln Leu Gln Pro Leu Phe Ile Gln Arg Arg Asp
              1060                1065                1070
   Ile Tyr Asp Thr Arg Glu Lys Lys Ser Lys Met Tyr Ser Trp Val Ala
          1075                1080                1085
   Phe Val Thr Gly Leu Ile Val Ser Glu Phe Pro Tyr Leu Cys Ile Cys
      1090                1095                1100
   Ala Val Leu Tyr Phe Val Cys Trp Tyr Tyr Pro Val Trp Arg Leu Pro
   105                 1110                1115                1120
   His Glu Ser Ser Arg Ser Gly Ala Thr Phe Phe Met Met Leu Ile Tyr
                  1125                1130                1135
   Glu Leu Ile Tyr Thr Gly Ile Gly Gln Phe Val Ala Ala Tyr Ser Pro
              1140                1145                1150
   Asn Pro Thr Phe Ala Ala Leu Val Asn Pro Leu Ile Ile Ser Thr Leu
          1155                1160                1165
   Val Leu Phe Cys Gly Ile Phe Val Pro Tyr Ile Gln Leu Asn Val Phe
      1170                1175                1180
   Trp Arg Tyr Trp Met Tyr Tyr Leu Asn Pro Phe Asn Tyr Val Val Ser
   185                 1190                1195                1200
   Gly Met Leu Thr Phe Gly Leu Trp Gly Ala Lys Val Thr Cys Asn Glu
                   1205                1210                1215
   Asp Glu Phe Ala Leu Phe Glu Pro Leu Asn Gly Thr Thr Cys Ala Gln
               1220                1225                1230
   Tyr Leu Ser Asp Tyr Met Ser Gly Ala Gly Ser Ser Ile Asn Leu Val
           1235                1240                1245
   Asn Pro Asp Ala Thr Ser Ala Cys Lys Val Cys Gln Tyr Thr Asp Gly
       1250                1255                1260
   Ser Asp Phe Leu Arg Asn Leu Asn Ile Met Asn Tyr Thr Thr Gly Trp
   265                 1270                1275                1280
   Arg Asp Ile Gly Ile Ser Val Ile Phe Ala Ile Ser Gly Tyr Ala Leu
                   1285                1290                1295
   Val Phe Gly Leu Met Lys Leu Arg Thr Lys Ala Ser Lys Lys Ala Glu
               1300                1305                1310
<210> 21
   <211> 408
   <212> DNA
   <213> Penicillium chrysogenum
<220>
   <223> Partial cDNA sequence of Penicillium chrysogenum ABC transporter aa6
<400> 21
   tcgggggcgg ggaagacgac tctactcgat gtcctcgctc agcgcaaaac tgaaggaact 60
   attaatggtt ctatcatggt tgatggccgt ccattacccg tctcgttcca acggtcagcc 120
   ggctatgttg agcagctcga cattcatgaa cgtatggcaa cggttcggga atcattggag 180
   ttctctgctc tattgcgcca acctgctacc actccacgcg aagagaaact tgcttacgtg 240
   gacgttatta ttgacttgct cgagctccat gatctggctg ataccatgat cggtagtgtt 300
   ggagctggat tgagtgtcga acaacgcaag cgcgttacta tcggagtcga acttgtttct 360
   aagcctagca tcttgatctt cctcgacgaa cccacctccg ccctcgaa              408
<210> 22
   <211> 408
   <212> DNA
   <213> Penicillium chrysogenum
<220>
   <221> CDS
   <222> (1)..(408)
<220>
   <223> Translation of the cDNA sequence of Penicillium chrysogenum ABC transporter aa6 from SEQ ID NO. 21
<400> 22
   tcg ggg gcg ggg aag acg act cta ctc gat gtc ctc gct cag cgc aaa    48
   Ser Gly Ala Gly Lys Thr Thr Leu Leu Asp Val Leu Ala Gln Arg Lys
    1               5                  10                  15
   act gaa gga act att aat ggt tct atc atg gtt gat ggc cgt cca tta    96
   Thr Glu Gly Thr Ile Asn Gly Ser Ile Met Val Asp Gly Arg Pro Leu
               20                  25                  30
   ccc gtc tcg ttc caa cgg tca gcc ggc tat gtt gag cag ctc gac att   144
   Pro Val Ser Phe Gln Arg Ser Ala Gly Tyr Val Glu Gln Leu Asp Ile
           35                  40                  45
   cat gaa cgt atg gca acg gtt cgg gaa tca ttg gag ttc tct gct cta   192
   His Glu Arg Met Ala Thr Val Arg Glu Ser Leu Glu Phe Ser Ala Leu
       50                  55                  60
   ttg cgc caa cct gct acc act cca cgc gaa gag aaa ctt gct tac gtg   240
   Leu Arg Gln Pro Ala Thr Thr Pro Arg Glu Glu Lys Leu Ala Tyr Val
   65                  70                  75                  80
   gac gtt att att gac ttg ctc gag ctc cat gat ctg gct gat acc atg   288
   Asp Val Ile Ile Asp Leu Leu Glu Leu His Asp Leu Ala Asp Thr Met
                   85                  90                  95
   atc ggt agt gtt gga gct gga ttg agt gtc gaa caa cgc aag cgc gtt   336
   Ile Gly Ser Val Gly Ala Gly Leu Ser Val Glu Gln Arg Lys Arg Val
               100                 105                 110
   act atc gga gtc gaa ctt gtt tct aag cct agc atc ttg atc ttc ctc   384
   Thr Ile Gly Val Glu Leu Val Ser Lys Pro Ser Ile Leu Ile Phe Leu
           115                 120                 125
   gac gaa ccc acc tcc gcc ctc gaa                                   408
   Asp Glu Pro Thr Ser Ala Leu Glu
       130                 135
<210> 23
   <211> 136
   <212> PRT
   <213> Penicillium chrysogenum
   <223> Protein sequence of the Penicillium chrysogenum ABC transporter aa6 (derived from the DNA sequence as shown in SEQ ID NO. 21)
<400> 23
   Ser Gly Ala Gly Lys Thr Thr Leu Leu Asp Val Leu Ala Gln Arg Lys
    1               5                  10                  15
   Thr Glu Gly Thr Ile Asn Gly Ser Ile Met Val Asp Gly Arg Pro Leu
               20                  25                  30
   Pro Val Ser Phe Gln Arg Ser Ala Gly Tyr Val Glu Gln Leu Asp Ile
           35                  40                  45
   His Glu Arg Met Ala Thr Val Arg Glu Ser Leu Glu Phe Ser Ala Leu
       50                  55                  60
   Leu Arg Gln Pro Ala Thr Thr Pro Arg Glu Glu Lys Leu Ala Tyr Val
   65                  70                  75                  80
   Asp Val Ile Ile Asp Leu Leu Glu Leu His Asp Leu Ala Asp Thr Met
                   85                  90                  95
   Ile Gly Ser Val Gly Ala Gly Leu Ser Val Glu Gln Arg Lys Arg Val
               100                 105                 110
   Thr Ile Gly Val Glu Leu Val Ser Lys Pro Ser Ile Leu Ile Phe Leu
           115                 120                 125
   Asp Glu Pro Thr Ser Ala Leu Glu
       130                 135
<210> 24
   <211> 429
   <212> DNA
   <213> Penicillium chrysogenum
<220>
   <223> Partial cDNA sequence of Penicillium chrysogenum ABC transporter aa7
<400> 24
   tcggggtcgg ggaaaagcac tataatcgaa cttctcgagc gtttctatga ccccgtttcc 60
   ggcgatattc tcttggacgg tcataaactc tcagagttga gcccaaattg gctgagacag 120
   caaatctcgc tggtccaaca gtcacccaca ttgtttgcta cgactatctt cgagaatatc 180
   cggtatggcc tggttggaac gccggatgaa aatgcatcca gggaaaatat cgagaatatt 240
   gtttacggtg ccactcgcct cgcgaatgcc cacgacttta tcaccaagtt acctgacggc 300
   tacgataccc ttgtcggcga ggccggcgtg ttgctttccg gcggccagaa acagcgcatt 360
   gctattgctc gcgccctcat ttccgatcct cgaatacttc tccttgacga acccacctcc 420
   gccctcgaa                                                         429
<210> 25
   <211> 7476
   <212> DNA
   <213> Penicillium chrysogenum
<220>
   <223> Complete genomic DNA sequence of Penicillium chrysogenum ABC transporter aa7
<400> 25
   gatcccttcg tattatcttc ttctcttccg catctcaaag gatccgagcc aaccgattat 60
   atggactctc acgtaccgag gtctcccaga tgcctcctcg cagagcgcat acaaaatccc 120
   gccaaggctg cgaccagtgc aagagacgtc gcgtcaaggt tagttttgga aactcgggat 180
   ggccccatag attgatccgg gctccatcaa ataacgagac taacgaccgc agtgtgacga 240
   aaaggggcca ccgtgctcca attgtatttc gcgagaactc tcatgcacat attttagagc 300
   gccgcctcgg cccgattcac aaaccagcca gaccccggcc ccgttggcta taatagaccc 360
   aagtcccctg cggactagga tccctagcct agcatcttcc gtttctccaa ccacttcagg 420
   ggtacgagat ctggagctta tgcacaagtt ttctaccgaa acttttagga gtctttgctc 480
   ttccaactcg gagcatcacg tctggcaaat tgccattcct cgtctggctc ttcagtatga 540
   ctttttaatg aatggcattt tggccctggg ggctcttcat attgccgcca ccattgagcc 600
   accggcatcg ctggtgtaca ttgacgttgc acttcaatat cacaatctca catttgcacc 660
   atatcgcgca gctgttgaca gcatcaatcc actgaactgc gaggctgcgc tggcgcagtc 720
   cattatcacc actgttatag gcattgctct gccaagagtc actgctgccc gcggggaaag 780
   ttcaagcata acagagaata tcgtcgtcgt atttgaactt ttgcaaggcg tgagaaaaat 840
   ccaccggatt ggtcaatctt ggatgaaact agagctcttt cgtcgaagaa actattggga 900
   tgctggctca gcgggtctag atagcgacac gcgggttgcc ctggaccgac tcggcacttt 960
   aaatgatgag accctcgccg gtatcgaccc cgatcagcat caggtcaaca aagatgctat 1020
   tgtctacttg cggcacttct caaccagagt ggccgattct gttgaccctg caaacgttct 1080
   ggcttggctc gctatgatcg acaaggaatt cgtggacaat gtgcgacgcc ggcagccttt 1140
   agcattgctg gttctcatgc actggggtgt gttgcttggg gagcttgatg gtcagcattg 1200
   gtgggctcga ggttcggggc gggcattagt ctctgaatta ttggatacat tgcatcccga 1260
   agatattgaa tgggccaacg ctgtagcttg gccacagaaa aagatgggac tctgatgtac 1320
   tatacctgct tagaatttat ataaggaaac aaacgatcgc cgtttaggta gatccctttg 1380
   ccgccttcca aggttgttac aatatcatag agatatggaa tgcaggaaaa tatataattg 1440
   atataagtaa aaaccgaaag aatcagacaa gatgccgcta atccatcatt ccacagaggc 1500
   ataatcgtcg ttttgtagtt cttggagggt ctattcaatg ttctgcttgt tctcgggcgg 1560
   actttgtcta gatatatata tgtacccaac ttatattaga catggcaatc tagtctgact 1620
   aatacaagaa tcgtcgtgta tgcggccgta ggctgatctt ctcaggtagt cttctctgat 1680
   agactttttg aatgtctggt tacatagaca ttctccttag tttgttgggt agacagcaga 1740
   aaaaggccaa aaagggttga tttgtgaaat ggtctttttc tatgtatagc ctttggtata 1800
   tacctaccta ggtaggtagc tttatacagt ttttatatag gtgaggctat aatggaaggc 1860
   cctgacagct ataagagcat aagagcataa gagcatatgt atgtgactgt aacttttgta 1920
   acaaaaagtg agagcctagt gggatccctg tcacttggca gatgggggcg acagatcact 1980
   gatttatcaa atattcgctc atctccggat tctttctcgt tatgttcacc aagttcacca 2040
   ccttcaccct ttcacttctg actctttcag tatggcaagc atatcggagc aggaaatact 2100
   cgcccagcaa atcacaaccc ccataatcaa gattaattat ctccaactct tccgatatgc 2160
   gaccaagaaa gattggttca ttatcggtgt atcctttctt gctgcgacca tcgccggggc 2220
   aatcacaaca atgccggcgc tgttaacagg cttattgatt ggcagcatac aagccagctg 2280
   gtccggtggt ccatcccaag acaggtccaa tagcgaattg acgcgcttca caatttactt 2340
   cgtatatctt tttctgggcg aaatagtgtc atgctacatt gcgaatatcg gattcatccg 2400
   cactggcatc atattatcga gtcggatcag agaacggtac ttggccgcgc ttctgagtca 2460
   aaatattgcc ttcttcgaca acattggagc cggagaaata tccactcgta tcactgccga 2520
   cgcaaatctg attcgagacg gtatttcaga gaaagtcagc gtcgccgtac agtgctccgc 2580
   atcggttgtc gcagcttttg tcataagttt catgcgagat tggaggttga ccctcatcct 2640
   ggcctccagc ctcatatgca tcgccctagt gtttgcagct gccggtataa tgctcacaaa 2700
   gtatcgccag caatggttgg gagagaccgc agaatctggt accattgtcg aagaggtctt 2760
   ttcctccatc agaacggtcg tggggctcaa tgcgcaatct gagcttgtgg ctcgatacga 2820
   tggctctcta gctaaagctg aacgctttgc aaataatgcc agactcatat ctggcgccct 2880
   gctcggtgct gtttttgccg ttatttacct ggctatcggc ttagggttct ggatgggttc 2940
   acgcttccta gtagcgggca catcatcgta cgtcgatgtt ttgacaatta tcctcgcgac 3000
   agtgaccggt atcgcctgcc tcggcggcat cgtccctcca cttcaagtat ttactattgc 3060
   cacggctgct ggatcaagac tatacagcac cattgatcga aggccttcag ggaccgccaa 3120
   tcggttttcg gagggaagtc tagacagcgt gctcggtcat attgagttgc aaaatgtgag 3180
   acacatttac ccttcaagac cagatatcgt tgttttggac aacctaagct tggatattga 3240
   gccaggaaaa acgacagcca ttgttggacc atctgggtcc ggaaaaagta ctataatcga 3300
   acttctcgag cgtttctatg accccgtttc cggcgatatt ctcttggacg gtcataaact 3360
   ctcagagttg agcccaaatt ggctgagaca gcaaatctcg ctggtccaac agtcacccac 3420
   attgtttgct acgactatct tcgagaatat ccggtatggc ctggttggaa cgcccgatga 3480
   aaatgcatcc agggaaaata tcgagaatat tgtttacggt gccactcgcc tcgcgaatgc 3540
   ccacgacttt atcaccaagt tacctgacgg ctacgatacc cttgtcggcg aggccggcgt 3600
   gttgctttcc ggcggccaga aacagcgcat tgctattgct cgcgccctca tttccgatcc 3660
   tcgaatactt ctccttgatg aagctacctc agccttggac tcgatgagtg aatccattgt 3720
   tcaagctgca atcgagaaag catctcaagg gagaacgacc attgttgtag ctcatcgttt 3780
   gtcgactgtc aaatccgcgg ataagattat tgtcttgtct ggaggtcagc ttatcgaaca 3840
   aggcaaccat gactttcttc aacgtctcaa tggagtttat agtcgactgg ccaaatcaca 3900
   ggctgtcaac ctgagcgaga agaaacctcc cgacgatgat gattctttgg gggttcccat 3960
   ccctcatgct aagtctgttt ctgtagagtc agaaaaggag gctccgccgc ttgtcggaag 4020
   tggaggtcct ttgtttgaca cagtgcgtaa gggcgcagat gctctcgcga gtctggatcg 4080
   gccaccccat cagtattcaa tgcagaccct tctgaggttt gtgaatagtt ttcacaagga 4140
   ctctataaaa ctggtgtcaa tgggctttct cgcttcaatc caaacaggag ctggagcacc 4200
   cgtccaggca gtctttctgg ccaaatgtct tgtggcactc gccagacctc ccactgaatc 4260
   cccgcagctt cggtctgaga caaatctatg ggctggaatg catgttgtcc tggctttcgt 4320
   gcagttcttc gcctactcgg ctcaagcttc tgctctcgga aaatgcacag agaggcttat 4380
   ccgtcgtctc ggtgaccttt ccttccgggc ccttctcgag acagacatgt ctttctttga 4440
   catggaggag cacggtgtgg gcgcattggt gtcatttctc ggcactgagc cagccagtat 4500
   ggcaggcatg ggttgctacg cgattgggaa ttacatcatg gcactgacaa ctctcatcgg 4560
   tgccatcgca accagcatgg ctgtcggatg gaaacttggc cttgtcggtg cagctacagt 4620
   acctgttctt ttgatgtgtg gatttcttcg attccgcgtc atggctcagc ttgaggccca 4680
   tcttcgccaa gtgtatcaag aaacagcatc attggccagt gaagcggtct cggccatccg 4740
   caccgtctta tctctgaacc gtgagtccga ggttgcaaga gtatttcacc ataagctagc 4800
   cgaacaagac tcgaagagca ttcgctcaag cctgacatct tcgggccttt ttgcgttttc 4860
   gcaatcggca cccttgttgt gtactgccct tggcttatgg tatggtggca ccctcgttat 4920
   cagtggggag tatggattgt tccagttcat cctgagtttt gctgccgtta acatttgtgg 4980
   tgatgctgct ggctcgatat ttagcagctc gccagattta gcaaaggcaa aactttcggc 5040
   cacccgactt aaagggctat tggatcgaca accacagcgc ccattggact ttgtcgaacc 5100
   tgcaactctt ttgcagggga ccattgaatt ccggaatgtc cacttttcgt atcctacgcg 5160
   ccccgaccgg caaatactaa aaggtctcga tctcactgta cacaagggta aatacatcgc 5220
   cctagttgga cccagtggat gcgggaaaag caccattgtt gcacttttag agcgtttcta 5280
   ccacccactg gccggtattg tcacaatgga cggcctggat ctctcctcaa tggatatgag 5340
   cgcctaccgc aatcaagtgg cactcgtgga tcaagagccc acactcttcc agggcaccat 5400
   ccgagataat ctgctcctcg gactcgatgc atcaaagtat tctcaggaag aattggagat 5460
   catctgcaag gatgcaaata tacttgattt tatccgttcg ctgccgtagg tcatccacca 5520
   aataaccatc actagcagca actgacttga tcatacagtc tcgggttcaa tacgatgtgt 5580
   ggagggaaag gaaacaactt ttccggtggc cagaaacagc gactagcaat tgcgcgggcc 5640
   cttctgagac ggcccagtgt cttgttacta gatgaagtca cttcagcgct ggattcggaa 5700
   tcccaaagtg tcgtccaggc tgcgctagac caagctgcaa agcagcgcac gacagttgcg 5760
   attgcccatc gcttgagtgc tgtccggaat gcggacctca tctgcttcct agaggacggt 5820
   gtgatcaccg agtccgggac acacgctgag ctcatacgaa gacgagggcg ttattttgca 5880
   atgttgagct tacagaacct agaaaaatag gtggtgatcc ttttgggctc gaaccttccc 5940
   caagttcctt catatttgat tctgattaag aataagatca tactctctgc tgatatagtc 6000
   tatatatttg caagcgccag tggcaacgag gttatggaat caggttttat tgagcggaat 6060
   gacgttcttc gagtaccgcg gctagtcgag gacgtcggtg ctagggagaa tctgcagtct 6120
   ggaagtagga cgcgagggtc caaggcgaca ccatcactac agcggctgga caaagtgggc 6180
   tgctctggtc ttgtggatac gcctggactg ctggacagtc ttcacttcgc gccagacgag 6240
   cgtactgctc gtcatctgga gaatgggaag gtcgaggtcg aggtcaaggc ggcgggtatc 6300
   aatttcaagg acgtcatgat ggccatgggt cagatccagg caaatgacct gggttgtgag 6360
   tgcagtggaa tcgtttcggc tgtggctggt gacgtcgtga ttgaccctca cggtcttcgg 6420
   gttggggatc gggccatgcg cttgagttca gggtccttct gtacgaggtt acggctggac 6480
   gctaggctgg cggcacgcat tcccgatttt atgtccttcg agacagcggc cgcgctacca 6540
   ctcatgaaac aatatcgcgc ccctacgcag aggcgagacc atcttgatcc acgccgccac 6600
   aggtgggtcg ggtcaggcct tcgtcgaaat gagccagctg cctgtcgcgg acatctttgt 6660
   caccgtgggc aacgcgaaga aaaaaagttt catcatggat cgatttggga tggcggaaga 6720
   acgcatcttg taccccgcga tatgagcttc gcttgagttc agaaattggc tttcaaagga 6780
   gatggagagc gtcgttccta tctttgagct gttggctgct gagtctcttg ttgttttagc 6840
   ctccaaaatc gctggtcgaa gcaggctagt agtcagtccg agcggataat tttcgattta 6900
   gctcaggttt gttactgtta ttgcgggttt gtctgagact tctgttcact tctttacttg 6960
   ccgagcatta cttcattgcc ttgggtcgac tcgggccagt ccttgtatca aaaaccaccc 7020
   catgttcccc ccacgggtgg acctacagtt aatgcaatgt agactcagct tatttgaact 7080
   gactactacc atcaagaggg tttcccctct ctaggatgaa cccactaaag ataaatttag 7140
   gaaggtatat tatgataagg cgtagcaccg gcggactata cgaagctcat atttctcatt 7200
   tatttcgaaa atcacgaagg ttacgagtat tctggccagt ctgcactctc cagtctcact 7260
   gtgactcatt tttcatatcc gagctttctc ctttctaggc ccgaggacat cgtgagtccg 7320
   tatcaaattt agagcatctg cctcatgctt gcacacctgc atgctcaatg gcccgctcac 7380
   ctgcttgagc cagtaggatc gtccgcaatc gaacaaccag ttacgagttc ctacttgaat 7440
   caactcagca aggaagcact gtggtgtgct gcgatc                           7476
<210> 26
   <211> 7476
   <212> DNA
   <213> Penicillium chrysogenum
<220>
   <221> CDS
   <222> (2072)..(5506)
<220>
   <221> CDS
   <222> (5560)..(5907)
<220>
   <223> Translation of the complete genomic DNA sequence of Penicillium chrysogenum ABC transporter aa7
<400> 26
   gatcccttcg tattatcttc ttctcttccg catctcaaag gatccgagcc aaccgattat 60
   atggactctc acgtaccgag gtctcccaga tgcctcctcg cagagcgcat acaaaatccc 120
   gccaaggctg cgaccagtgc aagagacgtc gcgtcaaggt tagttttgga aactcgggat 180
   ggccccatag attgatccgg gctccatcaa ataacgagac taacgaccgc agtgtgacga 240
   aaaggggcca ccgtgctcca attgtatttc gcgagaactc tcatgcacat attttagagc 300
   gccgcctcgg cccgattcac aaaccagcca gaccccggcc ccgttggcta taatagaccc 360
   aagtcccctg cggactagga tccctagcct agcatcttcc gtttctccaa ccacttcagg 420
   ggtacgagat ctggagctta tgcacaagtt ttctaccgaa acttttagga gtctttgctc 480
   ttccaactcg gagcatcacg tctggcaaat tgccattcct cgtctggctc ttcagtatga 540
   ctttttaatg aatggcattt tggccctggg ggctcttcat attgccgcca ccattgagcc 600
   accggcatcg ctggtgtaca ttgacgttgc acttcaatat cacaatctca catttgcacc 660
   atatcgcgca gctgttgaca gcatcaatcc actgaactgc gaggctgcgc tggcgcagtc 720
   cattatcacc actgttatag gcattgctct gccaagagtc actgctgccc gcggggaaag 780
   ttcaagcata acagagaata tcgtcgtcgt atttgaactt ttgcaaggcg tgagaaaaat 840
   ccaccggatt ggtcaatctt ggatgaaact agagctcttt cgtcgaagaa actattggga 900
   tgctggctca gcgggtctag atagcgacac gcgggttgcc ctggaccgac tcggcacttt 960
   aaatgatgag accctcgccg gtatcgaccc cgatcagcat caggtcaaca aagatgctat 1020
   tgtctacttg cggcacttct caaccagagt ggccgattct gttgaccctg caaacgttct 1080
   ggcttggctc gctatgatcg acaaggaatt cgtggacaat gtgcgacgcc ggcagccttt 1140
   agcattgctg gttctcatgc actggggtgt gttgcttggg gagcttgatg gtcagcattg 1200
   gtgggctcga ggttcggggc gggcattagt ctctgaatta ttggatacat tgcatcccga 1260
   agatattgaa tgggccaacg ctgtagcttg gccacagaaa aagatgggac tctgatgtac 1320
   tatacctgct tagaatttat ataaggaaac aaacgatcgc cgtttaggta gatccctttg 1380
   ccgccttcca aggttgttac aatatcatag agatatggaa tgcaggaaaa tatataattg 1440
   atataagtaa aaaccgaaag aatcagacaa gatgccgcta atccatcatt ccacagaggc 1500
   ataatcgtcg ttttgtagtt cttggagggt ctattcaatg ttctgcttgt tctcgggcgg 1560
   actttgtcta gatatatata tgtacccaac ttatattaga catggcaatc tagtctgact 1620
   aatacaagaa tcgtcgtgta tgcggccgta ggctgatctt ctcaggtagt cttctctgat 1680
   agactttttg aatgtctggt tacatagaca ttctccttag tttgttgggt agacagcaga 1740
   aaaaggccaa aaagggttga tttgtgaaat ggtctttttc tatgtatagc ctttggtata 1800
   tacctaccta ggtaggtagc tttatacagt ttttatatag gtgaggctat aatggaaggc 1860
   cctgacagct ataagagcat aagagcataa gagcatatgt atgtgactgt aacttttgta 1920
   acaaaaagtg agagcctagt gggatccctg tcacttggca gatgggggcg acagatcact 1980
   gatttatcaa atattcgctc atctccggat tctttctcgt tatgttcacc aagttcacca 2040
   ccttcaccct ttcacttctg actctttcag t atg gca agc ata tcg gag cag    2092
                                      Met Ala Ser Ile Ser Glu Gln
                                      1               5
   gaa ata ctc gcc cag caa atc aca acc ccc ata atc aag att aat tat   2140
   Glu Ile Leu Ala Gln Gln Ile Thr Thr Pro Ile Ile Lys Ile Asn Tyr
           10                  15                  20
   ctc caa ctc ttc cga tat gcg acc aag aaa gat tgg ttc att atc ggt   2188
   Leu Gln Leu Phe Arg Tyr Ala Thr Lys Lys Asp Trp Phe Ile Ile Gly
       25                  30                  35
   gta tcc ttt ctt gct gcg acc atc gcc ggg gca atc aca aca atg ccg   2236
   Val Ser Phe Leu Ala Ala Thr Ile Ala Gly Ala Ile Thr Thr Met Pro
   40                  45                  50                  55
   gcg ctg tta aca ggc tta ttg att ggc agc ata caa gcc agc tgg tcc   2284
   Ala Leu Leu Thr Gly Leu Leu Ile Gly Ser Ile Gln Ala Ser Trp Ser
                   60                  65                  70
   ggt ggt cca tcc caa gac agg tcc aat agc gaa ttg acg cgc ttc aca   2332
   Gly Gly Pro Ser Gln Asp Arg Ser Asn Ser Glu Leu Thr Arg Phe Thr
               75                  80                  85
   att tac ttc gta tat ctt ttt ctg ggc gaa ata gtg tca tgc tac att   2380
   Ile Tyr Phe Val Tyr Leu Phe Leu Gly Glu Ile Val Ser Cys Tyr Ile
           90                  95                  100
   gcg aat atc gga ttc atc cgc act ggc atc ata tta tcg agt cgg atc   2428
   Ala Asn Ile Gly Phe Ile Arg Thr Gly Ile Ile Leu Ser Ser Arg Ile
       105                 110                 115
   aga gaa cgg tac ttg gcc gcg ctt ctg agt caa aat att gcc ttc ttc   2476
   Arg Glu Arg Tyr Leu Ala Ala Leu Leu Ser Gln Asn Ile Ala Phe Phe
   120                 125                 130                 135
   gac aac att gga gcc gga gaa ata tcc act cgt atc act gcc gac gca   2524
   Asp Asn Ile Gly Ala Gly Glu Ile Ser Thr Arg Ile Thr Ala Asp Ala
                   140                 145                 150
   aat ctg att cga gac ggt att tca gag aaa gtc agc gtc gcc gta cag   2572
   Asn Leu Ile Arg Asp Gly Ile Ser Glu Lys Val Ser Val Ala Val Gln
               155                 160                 165
   tgc tcc gca tcg gtt gtc gca gct ttt gtc ata agt ttc atg cga gat   2620
   Cys Ser Ala Ser Val Val Ala Ala Phe Val Ile Ser Phe Met Arg Asp
           170                 175                 180
   tgg agg ttg acc ctc atc ctg gcc tcc agc ctc ata tgc atc gcc cta   2668
   Trp Arg Leu Thr Leu Ile Leu Ala Ser Ser Leu Ile Cys Ile Ala Leu
       185                 190                 195
   gtg ttt gca gct gcc ggt ata atg ctc aca aag tat cgc cag caa tgg    2716
   Val Phe Ala Ala Ala Gly Ile Met Leu Thr Lys Tyr Arg Gln Gln Trp
   200                 205                 210                 215
   ttg gga gag acc gca gaa tct ggt acc att gtc gaa gag gtc ttt tcc    2764
   Leu Gly Glu Thr Ala Glu Ser Gly Thr Ile Val Glu Glu Val Phe Ser
                   220                 225                 230
   tcc atc aga acg gtc gtg ggg ctc aat gcg caa tct gag ctt gtg gct    2812
   Ser Ile Arg Thr Val Val Gly Leu Asn Ala Gln Ser Glu Leu Val Ala
               235                 240                 245
   cga tac gat ggc tct cta gct aaa gct gaa cgc ttt gca aat aat gcc    2860
   Arg Tyr Asp Gly Ser Leu Ala Lys Ala Glu Arg Phe Ala Asn Asn Ala
           250                 255                 260
   aga ctc ata tct ggc gcc ctg ctc ggt gct gtt ttt gcc gtt att tac    2908
   Arg Leu Ile Ser Gly Ala Leu Leu Gly Ala Val Phe Ala Val Ile Tyr
       265                 270                 275
   ctg gct atc ggc tta ggg ttc tgg atg ggt tca cgc ttc cta gta gcg    2956
   Leu Ala Ile Gly Leu Gly Phe Trp Met Gly Ser Arg Phe Leu Val Ala
   280                 285                 290                 295
   ggc aca tca tcg tac gtc gat gtt ttg aca att atc ctc gcg aca gtg    3004
   Gly Thr Ser Ser Tyr Val Asp Val Leu Thr Ile Ile Leu Ala Thr Val
                   300                 305                 310
   acc ggt atc gcc tgc ctc ggc ggc atc gtc cct cca ctt caa gta ttt    3052
   Thr Gly Ile Ala Cys Leu Gly Gly Ile Val Pro Pro Leu Gln Val Phe
               315                 320                 325
   act att gcc acg gct gct gga tca aga cta tac agc acc att gat cga    3100
   Thr Ile Ala Thr Ala Ala Gly Ser Arg Leu Tyr Ser Thr Ile Asp Arg
           330                 335                 340
   agg cct tca ggg acc gcc aat cgg ttt tcg gag gga agt cta gac agc    3148
   Arg Pro Ser Gly Thr Ala Asn Arg Phe Ser Glu Gly Ser Leu Asp Ser
       345                 350                 355
   gtg ctc ggt cat att gag ttg caa aat gtg aga cac att tac cct tca    3196
   Val Leu Gly His Ile Glu Leu Gln Asn Val Arg His Ile Tyr Pro Ser
   360                 365                 370                 375
   aga cca gat atc gtt gtt ttg gac aac cta agc ttg gat att gag cca    3244
   Arg Pro Asp Ile Val Val Leu Asp Asn Leu Ser Leu Asp Ile Glu Pro
                   380                 385                 390
   gga aaa acg aca gcc att gtt gga cca tct ggg tcc gga aaa agt act    3292
   Gly Lys Thr Thr Ala Ile Val Gly Pro Ser Gly Ser Gly Lys Ser Thr
               395                 400                 405
   ata atc gaa ctt ctc gag cgt ttc tat gac ccc gtt tcc ggc gat att    3340
   Ile Ile Glu Leu Leu Glu Arg Phe Tyr Asp Pro Val Ser Gly Asp Ile
           410                 415                 420
   ctc ttg gac ggt cat aaa ctc tca gag ttg agc cca aat tgg ctg aga    3388
   Leu Leu Asp Gly His Lys Leu Ser Glu Leu Ser Pro Asn Trp Leu Arg
       425                 430                 435
   cag caa atc tcg ctg gtc caa cag tca ccc aca ttg ttt gct acg act    3436
   Gln Gln Ile Ser Leu Val Gln Gln Ser Pro Thr Leu Phe Ala Thr Thr
   440                 445                 450                 455
   atc ttc gag aat atc cgg tat ggc ctg gtt gga acg ccc gat gaa aat    3484
   Ile Phe Glu Asn Ile Arg Tyr Gly Leu Val Gly Thr Pro Asp Glu Asn
                   460                 465                 470
   gca tcc agg gaa aat atc gag aat att gtt tac ggt gcc act cgc ctc    3532
   Ala Ser Arg Glu Asn Ile Glu Asn Ile Val Tyr Gly Ala Thr Arg Leu
               475                 480                 485
   gcg aat gcc cac gac ttt atc acc aag tta cct gac ggc tac gat acc    3580
   Ala Asn Ala His Asp Phe Ile Thr Lys Leu Pro Asp Gly Tyr Asp Thr
           490                 495                 500
   ctt gtc ggc gag gcc ggc gtg ttg ctt tcc ggc ggc cag aaa cag cgc    3628
   Leu Val Gly Glu Ala Gly Val Leu Leu Ser Gly Gly Gln Lys Gln Arg
       505                 510                 515
   att gct att gct cgc gcc ctc att tcc gat cct cga ata ctt ctc ctt    3676
   Ile Ala Ile Ala Arg Ala Leu Ile Ser Asp Pro Arg Ile Leu Leu Leu
   520                 525                 530                 535
   gat gaa gct acc tca gcc ttg gac tcg atg agt gaa tcc att gtt caa    3724
   Asp Glu Ala Thr Ser Ala Leu Asp Ser Met Ser Glu Ser Ile Val Gln
                   540                 545                 550
   gct gca atc gag aaa gca tct caa ggg aga acg acc att gtt gta gct    3772
   Ala Ala Ile Glu Lys Ala Ser Gln Gly Arg Thr Thr Ile Val Val Ala
               555                 560                 565
   cat cgt ttg tcg act gtc aaa tcc gcg gat aag att att gtc ttg tct    3820
   His Arg Leu Ser Thr Val Lys Ser Ala Asp Lys Ile Ile Val Leu Ser
           570                 575                 580
   gga ggt cag ctt atc gaa caa ggc aac cat gac ttt ctt caa cgt ctc    3868
   Gly Gly Gln Leu Ile Glu Gln Gly Asn His Asp Phe Leu Gln Arg Leu
       585                 590                 595
   aat gga gtt tat agt cga ctg gcc aaa tca cag gct gtc aac ctg agc    3916
   Asn Gly Val Tyr Ser Arg Leu Ala Lys Ser Gln Ala Val Asn Leu Ser
   600                 605                 610                 615
   gag aag aaa cct ccc gac gat gat gat tct ttg ggg gtt ccc atc cct    3964
   Glu Lys Lys Pro Pro Asp Asp Asp Asp Ser Leu Gly Val Pro Ile Pro
                   620                 625                 630
   cat gct aag tct gtt tct gta gag tca gaa aag gag gct ccg ccg ctt    4012
   His Ala Lys Ser Val Ser Val Glu Ser Glu Lys Glu Ala Pro Pro Leu
               635                 640                 645
   gtc gga agt gga ggt cct ttg ttt gac aca gtg cgt aag ggc gca gat    4060
   Val Gly Ser Gly Gly Pro Leu Phe Asp Thr Val Arg Lys Gly Ala Asp
           650                 655                 660
   gct ctc gcg agt ctg gat cgg cca ccc cat cag tat tca atg cag acc    4108
   Ala Leu Ala Ser Leu Asp Arg Pro Pro His Gln Tyr Ser Met Gln Thr
       665                 670                 675
   ctt ctg agg ttt gtg aat agt ttt cac aag gac tct ata aaa ctg gtg    4156
   Leu Leu Arg Phe Val Asn Ser Phe His Lys Asp Ser Ile Lys Leu Val
   680                 685                 690                 695
   tca atg ggc ttt ctc gct tca atc caa aca gga gct gga gca ccc gtc     4204
   Ser Met Gly Phe Leu Ala Ser Ile Gln Thr Gly Ala Gly Ala Pro Val
                   700                 705                 710
   cag gca gtc ttt ctg gcc aaa tgt ctt gtg gca ctc gcc aga cct ccc     4252
   Gln Ala Val Phe Leu Ala Lys Cys Leu Val Ala Leu Ala Arg Pro Pro
               715                 720                 725
   act gaa tcc ccg cag ctt cgg tct gag aca aat cta tgg gct gga atg     4300
   Thr Glu Ser Pro Gln Leu Arg Ser Glu Thr Asn Leu Trp Ala Gly Met
           730                 735                 740
   cat gtt gtc ctg gct ttc gtg cag ttc ttc gcc tac tcg gct caa gct     4348
   His Val Val Leu Ala Phe Val Gln Phe Phe Ala Tyr Ser Ala Gln Ala
       745                 750                 755
   tct gct ctc gga aaa tgc aca gag agg ctt atc cgt cgt ctc ggt gac     4396
   Ser Ala Leu Gly Lys Cys Thr Glu Arg Leu Ile Arg Arg Leu Gly Asp
   760                 765                 770                 775
   ctt tcc ttc cgg gcc ctt ctc gag aca gac atg tct ttc ttt gac atg     4444
   Leu Ser Phe Arg Ala Leu Leu Glu Thr Asp Met Ser Phe Phe Asp Met
                   780                 785                 790
   gag gag cac ggt gtg ggc gca ttg gtg tca ttt ctc ggc act gag cca     4492
   Glu Glu His Gly Val Gly Ala Leu Val Ser Phe Leu Gly Thr Glu Pro
               795                 800                 805
   gcc agt atg gca ggc atg ggt tgc tac gcg att ggg aat tac atc atg     4540
   Ala Ser Met Ala Gly Met Gly Cys Tyr Ala Ile Gly Asn Tyr Ile Met
           810                 815                 820
   gca ctg aca act ctc atc ggt gcc atc gca acc agc atg gct gtc gga     4588
   Ala Leu Thr Thr Leu Ile Gly Ala Ile Ala Thr Ser Met Ala Val Gly
       825                 830                 835
   tgg aaa ctt ggc ctt gtc ggt gca gct aca gta cct gtt ctt ttg atg     4636
   Trp Lys Leu Gly Leu Val Gly Ala Ala Thr Val Pro Val Leu Leu Met
   840                 845                 850                 855
   tgt gga ttt ctt cga ttc cgc gtc atg gct cag ctt gag gcc cat ctt     4684
   Cys Gly Phe Leu Arg Phe Arg Val Met Ala Gln Leu Glu Ala His Leu
                   860                 865                 870
   cgc caa gtg tat caa gaa aca gca tca ttg gcc agt gaa gcg gtc tcg     4732
   Arg Gln Val Tyr Gln Glu Thr Ala Ser Leu Ala Ser Glu Ala Val Ser
               875                 880                 885
   gcc atc cgc acc gtc tta tct ctg aac cgt gag tcc gag gtt gca aga     4780
   Ala Ile Arg Thr Val Leu Ser Leu Asn Arg Glu Ser Glu Val Ala Arg
           890                 895                 900
   gta ttt cac cat aag cta gcc gaa caa gac tcg aag agc att cgc tca     4828
   Val Phe His His Lys Leu Ala Glu Gln Asp Ser Lys Ser Ile Arg Ser
       905                 910                 915
   agc ctg aca tct tcg ggc ctt ttt gcg ttt tcg caa tcg gca ccc ttg     4876
   Ser Leu Thr Ser Ser Gly Leu Phe Ala Phe Ser Gln Ser Ala Pro Leu
   920                 925                 930                 935
   ttg tgt act gcc ctt ggc tta tgg tat ggt ggc acc ctc gtt atc agt   4924
   Leu Cys Thr Ala Leu Gly Leu Trp Tyr Gly Gly Thr Leu Val Ile Ser
                   940                 945                 950
   ggg gag tat gga ttg ttc cag ttc atc ctg agt ttt gct gcc gtt aac   4972
   Gly Glu Tyr Gly Leu Phe Gln Phe Ile Leu Ser Phe Ala Ala Val Asn
               955                 960                 965
   att tgt ggt gat gct gct ggc tcg ata ttt agc agc tcg cca gat tta   5020
   Ile Cys Gly Asp Ala Ala Gly Ser Ile Phe Ser Ser Ser Pro Asp Leu
           970                 975                 980
   gca aag gca aaa ctt tcg gcc acc cga ctt aaa ggg cta ttg gat cga   5068
   Ala Lys Ala Lys Leu Ser Ala Thr Arg Leu Lys Gly Leu Leu Asp Arg
       985                 990                 995
   caa cca cag cgc cca ttg gac ttt gtc gaa cct gca act ctt ttg cag   5116
   Gln Pro Gln Arg Pro Leu Asp Phe Val Glu Pro Ala Thr Leu Leu Gln
   1000                1005                1010                1015
   ggg acc att gaa ttc cgg aat gtc cac ttt tcg tat cct acg cgc ccc   5164
   Gly Thr Ile Glu Phe Arg Asn Val His Phe Ser Tyr Pro Thr Arg Pro
                   1020                1025                1030
   gac cgg caa ata cta aaa ggt ctc gat ctc act gta cac aag ggt aaa   5212
   Asp Arg Gln Ile Leu Lys Gly Leu Asp Leu Thr Val His Lys Gly Lys
               1035                1040                1045
   tac atc gcc cta gtt gga ccc agt gga tgc ggg aaa agc acc att gtt   5260
   Tyr Ile Ala Leu Val Gly Pro Ser Gly Cys Gly Lys Ser Thr Ile Val
           1050                1055                1060
   gca ctt tta gag cgt ttc tac cac cca ctg gcc ggt att gtc aca atg   5308
   Ala Leu Leu Glu Arg Phe Tyr His Pro Leu Ala Gly Ile Val Thr Met
       1065                1070                1075
   gac ggc ctg gat ctc tcc tca atg gat atg agc gcc tac cgc aat caa   5356
   Asp Gly Leu Asp Leu Ser Ser Met Asp Met Ser Ala Tyr Arg Asn Gln
   1080                1085                1090                1095
   gtg gca ctc gtg gat caa gag ccc aca ctc ttc cag ggc acc atc cga   5404
   Val Ala Leu Val Asp Gln Glu Pro Thr Leu Phe Gln Gly Thr Ile Arg
                   1100                1105                1110
   gat aat ctg ctc ctc gga ctc gat gca tca aag tat tct cag gaa gaa   5452
   Asp Asn Leu Leu Leu Gly Leu Asp Ala Ser Lys Tyr Ser Gln Glu Glu
               1115                1120                1125
   ttg gag atc atc tgc aag gat gca aat ata ctt gat ttt atc cgt tcg   5500
   Leu Glu Ile Ile Cys Lys Asp Ala Asn Ile Leu Asp Phe Ile Arg Ser
           1130                1135                1140
   ctg ccg taggtcatcc accaaataac catcactagc agcaactgac ttgatcatac    5556
   Leu Pro
       1145
   agt ctc ggg ttc aat acg atg tgt gga ggg aaa gga aac aac ttt tcc   5604
       Leu Gly Phe Asn Thr Met Cys Gly Gly Lys Gly Asn Asn Phe Ser
                   1150                1155                1160
   ggt ggc cag aaa cag cga cta gca att gcg cgg gcc ctt ctg aga cgg   5652
   Gly Gly Gln Lys Gln Arg Leu Ala Ile Ala Arg Ala Leu Leu Arg Arg
                   1165                1170                1175
   ccc agt gtc ttg tta cta gat gaa gtc act tca gcg ctg gat tcg gaa   5700
   Pro Ser Val Leu Leu Leu Asp Glu Val Thr Ser Ala Leu Asp Ser Glu
               1180                1185                1190
   tcc caa agt gtc gtc cag gct gcg cta gac caa gct gca aag cag cgc   5748
   Ser Gln Ser Val Val Gln Ala Ala Leu Asp Gln Ala Ala Lys Gln Arg
           1195                1200                1205
   acg aca gtt gcg att gcc cat cgc ttg agt gct gtc cgg aat gcg gac   5796
   Thr Thr Val Ala Ile Ala His Arg Leu Ser Ala Val Arg Asn Ala Asp
       1210                1215                1220
   ctc atc tgc ttc cta gag gac ggt gtg atc acc gag tcc ggg aca cac   5844
   Leu Ile Cys Phe Leu Glu Asp Gly Val Ile Thr Glu Ser Gly Thr His
   1225                1230                1235                1240
   gct gag ctc ata cga aga cga ggg cgt tat ttt gca atg ttg agc tta   5892
   Ala Glu Leu Ile Arg Arg Arg Gly Arg Tyr Phe Ala Met Leu Ser Leu
                   1245                1250                1255
   cag aac cta gaa aaa taggtggtga tccttttggg ctcgaacctt ccccaagttc   5947
   Gln Asn Leu Glu Lys
               1260
   cttcatattt gattctgatt aagaataaga tcatactctc tgctgatata gtctatatat 6007
   ttgcaagcgc cagtggcaac gaggttatgg aatcaggttt tattgagcgg aatgacgttc 6067
   ttcgagtacc gcggctagtc gaggacgtcg gtgctaggga gaatctgcag tctggaagta 6127
   ggacgcgagg gtccaaggcg acaccatcac tacagcggct ggacaaagtg ggctgctctg 6187
   gtcttgtgga tacgcctgga ctgctggaca gtcttcactt cgcgccagac gagcgtactg 6247
   ctcgtcatct ggagaatggg aaggtcgagg tcgaggtcaa ggcggcgggt atcaatttca 6307
   aggacgtcat gatggccatg ggtcagatcc aggcaaatga cctgggttgt gagtgcagtg 6367
   gaatcgtttc ggctgtggct ggtgacgtcg tgattgaccc tcacggtctt cgggttgggg 6427
   atcgggccat gcgcttgagt tcagggtcct tctgtacgag gttacggctg gacgctaggc 6487
   tggcggcacg cattcccgat tttatgtcct tcgagacagc ggccgcgcta ccactcatga 6547
   aacaatatcg cgcccctacg cagaggcgag accatcttga tccacgccgc cacaggtggg 6607
   tcgggtcagg ccttcgtcga aatgagccag ctgcctgtcg cggacatctt tgtcaccgtg 6667
   ggcaacgcga agaaaaaaag tttcatcatg gatcgatttg ggatggcgga agaacgcatc 6727
   ttgtaccccg cgatatgagc ttcgcttgag ttcagaaatt ggctttcaaa ggagatggag 6787
   agcgtcgttc ctatctttga gctgttggct gctgagtctc ttgttgtttt agcctccaaa 6847
   atcgctggtc gaagcaggct agtagtcagt ccgagcggat aattttcgat ttagctcagg 6907
   tttgttactg ttattgcggg tttgtctgag acttctgttc acttctttac ttgccgagca 6967
   ttacttcatt gccttgggtc gactcgggcc agtccttgta tcaaaaacca ccccatgttc 7027
   cccccacggg tggacctaca gttaatgcaa tgtagactca gcttatttga actgactact 7087
   accatcaaga gggtttcccc tctctaggat gaacccacta aagataaatt taggaaggta 7147
   tattatgata aggcgtagca ccggcggact atacgaagct catatttctc atttatttcg 7207
   aaaatcacga aggttacgag tattctggcc agtctgcact ctccagtctc actgtgactc 7267
   atttttcata tccgagcttt ctcctttcta ggcccgagga catcgtgagt ccgtatcaaa 7327
   tttagagcat ctgcctcatg cttgcacacc tgcatgctca atggcccgct cacctgcttg 7387
   agccagtagg atcgtccgca atcgaacaac cagttacgag ttcctacttg aatcaactca 7447
   gcaaggaagc actgtggtgt gctgcgatc                                  7476
<210> 27
   <211> 3416
   <212> DNA
   <213> Penicillium chrysogenum
<220>
   <223> Large, partial cDNA sequence of Penicillium chrysogenum ABC transporter aa7
<400> 27
   gcgtccgcgg acgcgtgggc ggacgcgtgg gttgatcgaa ggccttcagg gaccgccaat 60
   cggttttcgg agggaagtct agacagcgtg ctcggtcata ttgagttgca aaatgtgaga 120
   cacatttacc cttcaagacc agatatcgtt gttttggaca acctaagctt ggatattgag 180
   ccaggaaaaa cgacggccat tgttggacca tctgggtccg gaaaaagtac tataatcgaa 240
   cttctcgagc gtttctatga ccccgtttcc ggcgatattc tcttggacgg tcataaactc 300
   tcagagttga gcccaaattg gctgagacag caaatctcgc tggtccaaca gtcacccaca 360
   ttgtttgcta cgactatctt cgagaatatc cggtatggcc tggttggaac gcccgatgaa 420
   aatgcatcca gggaaaatat cgagaatatt gtttacggtg ccactcgcct cgcgaatgcc 480
   cacgacttta tcaccaagtt acctgacggc tacgataccc ttgtcggcga ggccggcgtg 540
   ttgctttccg gcggccagaa acagcgcatt gctattgctc gcgccctcat ttccgatcct 600
   cgaatacttc tccttgatga agctacctca gccttggact cgatgagtga atccattgtt 660
   caagctgcaa tcgagaaagc atctcaaggg agaacgacca ttgttgtagc tcatcgtttg 720
   tcgactgtca aatccgcgga taagattatt gtcttgtctg gaggtcagct tatcgaacaa 780
   ggcaaccatg actttcttca acgtctcaat ggagtttata gtcgactggc caaatcacag 840
   gctgtcaacc tgagcgagaa gaaacctccc gacgatgatg attccttggg ggttcccatc 900
   cctcatgcta agtctgtttc tgtagagtca gaaaaggagg ctccgccgct tgtcggaagt 960
   ggaggtcctt tgtttgacac agtgcgtaag ggcgcagatg ctctcgcgag tctggatcgg 1020
   ccaccccatc agtattcaat gcagaccctt ctgaggtttg tgaatagttt tcacaaggac 1080
   tctataaaac tggtgtcaat gggctttctc gcttcaatcc aaacaggagc tggagcaccc 1140
   gtccaggcag tcctttctgg caaatgtctt gtggcactcg ccagacctcc cactgaatcc 1200
   ccgcagcttc ggtctgagac aaatctatgg gctggaatgc atgttgtcct ggctttcgcg 1260
   cagttcttcg cctactcggc tcaagcttct gctctcggaa aatgcacaga gaggcttatc 1320
   cgtcgtctcg gcgacctttc cttccgggcc cttctcgaga cagacatgtc tttctttgac 1380
   atggaggagc acggtgtggg cgcattggtg tcatttctcg gcactgagcc agccagtatg 1440
   gcaggcatgg gttgctacgc gattgggaat tacatcatgg cactgacaac tctcatcggt 1500
   gccatcgcaa ccagcatggc tgtcggatgg aaacttggcc ttgtcggtgc agctacagta 1560
   cctgttcttt tgatgtgtgg atttcttcga ttccgcgtca tggctcagct tgaggcccat 1620
   cttcgccaag tgtatcaaga aacagcatca ttggccagtg aagcggtctc ggccatccgc 1680
   accgtcttat ctctgaaccg tgagtccgag gttgcaagag tatttcacca taagctagcc 1740
   ggacaagact cgaagagcat tcgctcaagc ctgacatctt cgggcctttt tgcgttttcg 1800
   caatcggcac ccttgttgtg tactgccctt ggcttatggt atggtggcac cctcgttatc 1860
   agtggggagt atggattgtt ccagttcatc ctgagttttg ctgccgttaa catttgtggt 1920
   gatgctgctg gctcgatatt tagcagctcg ccagatttag caaaggcaaa actttcggcc 1980
   acccgactta aagggctatt ggatcgacaa ccacagcgcc cattggactt tgtcgaacct 2040
   gcaactcttt tgcaggggac cattgaattc cggaatgtcc acttttcgta tcctacgcgc 2100
   cccgaccggc aaatactaaa aggtctcgat ctcactgtac acaagggtaa atacatcgcc 2160
   ctagttggac ccagtggatg cgggaaaagc accattgttg cacttttaga gcgtttctac 2220
   cacccactgg ccggtattgt cacaatggac ggcctggatc tctcctcaat ggatatgagc 2280
   gcctaccgca atcaagtggc actcgtggat caagagccca cactcttcca gggcaccatc 2340
   cgagataatc tgctcctcgg actcgatgca tcaaagtatt ctcaggaaga attggagatc 2400
   atctgcaagg atgcaaatat acttgatttt atccgttcgc tgcctctcgg gttcaatacg 2460
   atgtgtggag ggaaaggaaa caacttttcc ggtggccaga aacagcgact agcaattgcg 2520
   cgggcccttc tgagacggcc cagtgtcttg ttactagatg aagtcacttc agcgctggat 2580
   tcggaatccc aaagtgtcgt ccaggctgcg ctagaccaag ctgcaaagca gcgcacgaca 2640
   gttgcgattg cccatcgctt gagtgctgtc cggaatgcgg acctcatctg cttcctagag 2700
   gacggtgtga tcacggagtc cgggacacac gctgagctca tacgaagacg agggcgttat 2760
   tttgcaatgt tgagcttaca gacctagaaa aataggtggt gacccttttg ggctcgacct 2820
   tccccaagtt ccttcatatt tgattctgat taagaataag acatactctc tgctgatata 2880
   gtctatatat ttgcaagcgc cagtggcaac gaggttatgg aatcaggttt tattgagcgg 2940
   aatgacgttc ttcgagtacc gcggctagtc gaggacgtcg gtgctaggga gaatctgcag 3000
   tctggaagta ggacgcgagg gtccaaggcg acaccatcac tacagcggct ggacaaagtg 3060
   ggctgctctg gtcttgtgga tacgcctgga ctgctggaca gtcttcactt cgcgccagac 3120
   gagcgtactg ctcgtcatct ggagaatggg aaggtcgagg tcgaggtcaa ggcggcgggt 3180
   atcaatttca aggacgtcat gatggccatg ggtcagatcc aggcaaatga cctgggttgt 3240
   gagtgcagtg gaatcgtttc ggctgtggct ggtgacgtcg tgattgaccc tcacggtctt 3300
   cgggttgggg atcgggccat gcgcttgagt tcagggtcct tctgtacgag gttacggctg 3360
   gacgctaggc tggcggcacg cattcccgat tttatgtcct tcgagacagc ggccgc 3416
<210> 28
   <211> 1261
   <212> PRT
   <213> Penicillium chrysogenum
   <223> Protein sequence of the Penicillium chrysogenum ABC transporter aa7 (derived from the DNA sequence as Shown in SEQ ID NO. 26)
<400> 28
   Met Ala Ser Ile Ser Glu Gln Glu Ile Leu Ala Gln Gln Ile Thr Thr
    1               5                  10                  15
   Pro Ile Ile Lys Ile Asn Tyr Leu Gln Leu Phe Arg Tyr Ala Thr Lys
               20                  25                  30
   Lys Asp Trp Phe Ile Ile Gly Val Ser Phe Leu Ala Ala Thr Ile Ala
           35                  40                  45
   Gly Ala Ile Thr Thr Met Pro Ala Leu Leu Thr Gly Leu Leu Ile Gly
       50                  55                  60
   Ser Ile Gln Ala Ser Trp Ser Gly Gly Pro Ser Gln Asp Arg Ser Asn
   65                  70                  75                  80
   Ser Glu Leu Thr Arg Phe Thr Ile Tyr Phe Val Tyr Leu Phe Leu Gly
                   85                  90                  95
   Glu Ile Val Ser Cys Tyr Ile Ala Asn Ile Gly Phe Ile Arg Thr Gly
               100                 105                 110
   Ile Ile Leu Ser Ser Arg Ile Arg Glu Arg Tyr Leu Ala Ala Leu Leu
   115 120 125
   Ser Gln Asn Ile Ala Phe Phe Asp Asn Ile Gly Ala Gly Glu Ile Ser
   130 135 140
   Thr Arg Ile Thr Ala Asp Ala Asn Leu Ile Arg Asp Gly Ile Ser Glu
   145 150 155 160
   Lys Val Ser Val Ala Val Gln Cys Ser Ala Ser Val Val Ala Ala Phe
   165 170 175
   Val Ile Ser Phe Met Arg Asp Trp Arg Leu Thr Leu Ile Leu Ala Ser
   180 185 190
   Ser Leu Ile Cys Ile Ala Leu Val Phe Ala Ala Ala Gly Ile Met Leu
   195 200 205
   Thr Lys Tyr Arg Gln Gln Trp Leu Gly Glu Thr Ala Glu Ser Gly Thr
   210 215 220
   Ile Val Glu Glu Val Phe Ser Ser Ile Arg Thr Val Val Gly Leu Asn
   225 230 235 240
   Ala Gln Ser Glu Leu Val Ala Arg Tyr Asp Gly Ser Leu Ala Lys Ala
   245 250 255
   Glu Arg Phe Ala Asn Asn Ala Arg Leu Ile Ser Gly Ala Leu Leu Gly
   260 265 270
   Ala Val Phe Ala Val Ile Tyr Leu Ala Ile Gly Leu Gly Phe Trp Met
   275 280 285
   Gly Ser Arg Phe Leu Val Ala Gly Thr Ser Ser Tyr Val Asp Val Leu
   290 295 300
   Thr Ile Ile Leu Ala Thr Val Thr Gly Ile Ala Cys Leu Gly Gly Ile
   305 310 315 320
   Val Pro Pro Leu Gln Val Phe Thr Ile Ala Thr Ala Ala Gly Ser Arg
   325 330 335
   Leu Tyr Ser Thr Ile Asp Arg Arg Pro Ser Gly Thr Ala Asn Arg Phe
   340 345 350
   Ser Glu Gly Ser Leu Asp Ser Val Leu Gly His Ile Glu Leu Gln Asn
   355 360 365
   Val Arg His Ile Tyr Pro Ser Arg Pro Asp Ile Val Val Leu Asp Asn
   370 375 380
   Leu Ser Leu Asp Ile Glu Pro Gly Lys Thr Thr Ala Ile Val Gly Pro
   385 390 395 400
   Ser Gly Ser Gly Lys Ser Thr Ile Ile Glu Leu Leu Glu Arg Phe Tyr
   405 410 415
   Asp Pro Val Ser Gly Asp Ile Leu Leu Asp Gly His Lys Leu Ser Glu
   420 425 430
   Leu Ser Pro Asn Trp Leu Arg Gln Gln Ile Ser Leu Val Gln Gln Ser
   435 440 445
   Pro Thr Leu Phe Ala Thr Thr Ile Phe Glu Asn Ile Arg Tyr Gly Leu
   450 455 460
   Val Gly Thr Pro Asp Glu Asn Ala Ser Arg Glu Asn Ile Glu Asn Ile
   465 470 475 480
   Val Tyr Gly Ala Thr Arg Leu Ala Asn Ala His Asp Phe Ile Thr Lys
   485 490 495
   Leu Pro Asp Gly Tyr Asp Thr Leu Val Gly Glu Ala Gly Val Leu Leu
   500 505 510
   Ser Gly Gly Gln Lys Gln Arg Ile Ala Ile Ala Arg Ala Leu Ile Ser
   515 520 525
   Asp Pro Arg Ile Leu Leu Leu Asp Glu Ala Thr Ser Ala Leu Asp Ser
   530 535 540
   Met Ser Glu Ser Ile Val Gln Ala Ala Ile Glu Lys Ala Ser Gln Gly
   545 550 555 560
   Arg Thr Thr Ile Val Val Ala His Arg Leu Ser Thr Val Lys Ser Ala
   565 570 575
   Asp Lys Ile Ile Val Leu Ser Gly Gly Gln Leu Ile Glu Gln Gly Asn
   580 585 590
   His Asp Phe Leu Gln Arg Leu Asn Gly Val Tyr Ser Arg Leu Ala Lys
   595 600 605
   Ser Gln Ala Val Asn Leu Ser Glu Lys Lys Pro Pro Asp Asp Asp Asp
   610 615 620
   Ser Leu Gly Val Pro Ile Pro His Ala Lys Ser Val Ser Val Glu Ser
   625 630 635 640
   Glu Lys Glu Ala Pro Pro Leu Val Gly Ser Gly Gly Pro Leu Phe Asp
   645 650 655
   Thr Val Arg Lys Gly Ala Asp Ala Leu Ala Ser Leu Asp Arg Pro Pro
   660 665 670
   His Gln Tyr Ser Met Gln Thr Leu Leu Arg Phe Val Asn Ser Phe His
   675 680 685
   Lys Asp Ser Ile Lys Leu Val Ser Met Gly Phe Leu Ala Ser Ile Gln
   690 695 700
   Thr Gly Ala Gly Ala Pro Val Gln Ala Val Phe Leu Ala Lys Cys Leu
   705 710 715 720
   Val Ala Leu Ala Arg Pro Pro Thr Glu Ser Pro Gln Leu Arg Ser Glu
   725 730 735
   Thr Asn Leu Trp Ala Gly Met His Val Val Leu Ala Phe Val Gln Phe
   740 745 750
   Phe Ala Tyr Ser Ala Gln Ala Ser Ala Leu Gly Lys Cys Thr Glu Arg
   755 760 765
   Leu Ile Arg Arg Leu Gly Asp Leu Ser Phe Arg Ala Leu Leu Glu Thr
   770 775 780
   Asp Met Ser Phe Phe Asp Met Glu Glu His Gly Val Gly Ala Leu Val
   785 790 795 800
   Ser Phe Leu Gly Thr Glu Pro Ala Ser Met Ala Gly Met Gly Cys Tyr
   805 810 815
   Ala Ile Gly Asn Tyr Ile Met Ala Leu Thr Thr Leu Ile Gly Ala Ile
   820 825 830
   Ala Thr Ser Met Ala Val Gly Trp Lys Leu Gly Leu Val Gly Ala Ala
   835 840 845
   Thr Val Pro Val Leu Leu Met Cys Gly Phe Leu Arg Phe Arg Val Met
   850 855 860
   Ala Gln Leu Glu Ala His Leu Arg Gln Val Tyr Gln Glu Thr Ala Ser
   865 870 875 880
   Leu Ala Ser Glu Ala Val Ser Ala Ile Arg Thr Val Leu Ser Leu Asn
   885 890 895
   Arg Glu Ser Glu Val Ala Arg Val Phe His His Lys Leu Ala Glu Gln
   900 905 910
   Asp Ser Lys Ser Ile Arg Ser Ser Leu Thr Ser Ser Gly Leu Phe Ala
   915 920 925
   Phe Ser Gln Ser Ala Pro Leu Leu Cys Thr Ala Leu Gly Leu Trp Tyr
   930 935 940
   Gly Gly Thr Leu Val Ile Ser Gly Glu Tyr Gly Leu Phe Gln Phe Ile
   945 950 955 960
   Leu Ser Phe Ala Ala Val Asn Ile Cys Gly Asp Ala Ala Gly Ser Ile
   965 970 975
   Phe Ser Ser Ser Pro Asp Leu Ala Lys Ala Lys Leu Ser Ala Thr Arg
   980 985 990
   Leu Lys Gly Leu Leu Asp Arg Gln Pro Gln Arg Pro Leu Asp Phe Val
   995 1000 1005
   Glu Pro Ala Thr Leu Leu Gln Gly Thr Ile Glu Phe Arg Asn Val His
   1010 1015 1020
   Phe Ser Tyr Pro Thr Arg Pro Asp Arg Gln Ile Leu Lys Gly Leu Asp
   025 1030 1035 1040
   Leu Thr Val His Lys Gly Lys Tyr Ile Ala Leu Val Gly Pro Ser Gly
   1045 1050 1055
   Cys Gly Lys Ser Thr Ile Val Ala Leu Leu Glu Arg Phe Tyr His Pro
   1060 1065 1070
   Leu Ala Gly Ile Val Thr Met Asp Gly Leu Asp Leu Ser Ser Met Asp
   1075 1080 1085
   Met Ser Ala Tyr Arg Asn Gln Val Ala Leu Val Asp Gln Glu Pro Thr
   1090 1095 1100
   Leu Phe Gln Gly Thr Ile Arg Asp Asn Leu Leu Leu Gly Leu Asp Ala
   105 1110 1115 1120
   Ser Lys Tyr Ser Gln Glu Glu Leu Glu Ile Ile Cys Lys Asp Ala Asn
   1125 1130 1135
   Ile Leu Asp Phe Ile Arg Ser Leu Pro Leu Gly Phe Asn Thr Met Cys
   1140 1145 1150
   Gly Gly Lys Gly Asn Asn Phe Ser Gly Gly Gln Lys Gln Arg Leu Ala
   1155 1160 1165
   Ile Ala Arg Ala Leu Leu Arg Arg Pro Ser Val Leu Leu Leu Asp Glu
   1170 1175 1180
   Val Thr Ser Ala Leu Asp Ser Glu Ser Gln Ser Val Val Gln Ala Ala
   185 1190 1195 1200
   Leu Asp Gln Ala Ala Lys Gln Arg Thr Thr Val Ala Ile Ala His Arg
   1205 1210 1215
   Leu Ser Ala Val Arg Asn Ala Asp Leu Ile Cys Phe Leu Glu Asp Gly
   1220 1225 1230
   Val Ile Thr Glu Ser Gly Thr His Ala Glu Leu Ile Arg Arg Arg Gly
   1235 1240 1245
   Arg Tyr Phe Ala Met Leu Ser Leu Gln Asn Leu Glu Lys
   1250 1255 1260
<210> 29
   <211> 405
   <212> DNA
   <213> Penicillium chrysogenum
<220>
   <223> Partial cDNA sequence of Penicillium chrysogenum ABC transporter aa8
<400> 29
   tcgggggggg ggaaaacgac actgcttgac gtgctagcaa cgcgagtaca atggggggtt 60
   gtcagtggcg aaatgctcgt tgacggaaac cccacagatc agtccttcca gcgaaagacc 120
   ggatatgtcc aacagcagga cttgcacttg tctacgtcga cggttcggga agcactagag 180
   tttagtgctt tgctacgtca acccgcctct gttagccgga aggaaaagat agactatgct 240
   gacgaggtta tcaagctcct tggtatggag gcgtacgctg acgctgttgt cggtgttccg 300
   ggcgagggat tgaacgttga gcagcggaag agattgacga ttggtgtgga gcttgccgcg 360
   aaaccccagc tcctactctt cctggatgaa ccaacctcgg ccctg 405
<210> 30
   <211> 405
   <212> DNA
   <213> Penicillium chrysogenum
<220>
   <221> CDS
   <222> (1)..(405)
<220>
   <223> Translation of the cDNA sequence of Penicillium chrysogenum ABC transporter aa8 from SEQ ID NO. 29
<400> 30
   tcg ggg ggg ggg aaa acg aca ctg ctt gac gtg cta gca acg cga gta    48
   Ser Gly Gly Gly Lys Thr Thr Leu Leu Asp Val Leu Ala Thr Arg Val
   1               5                   10                  15
   caa tgg ggg gtt gtc agt ggc gaa atg ctc gtt gac gga aac ccc aca    96
   Gln Trp Gly Val Val Ser Gly Glu Met Leu Val Asp Gly Asn Pro Thr
               20                  25                  30
   gat cag tcc ttc cag cga aag acc gga tat gtc caa cag cag gac ttg    144
   Asp Gln Ser Phe Gln Arg Lys Thr Gly Tyr Val Gln Gln Gln Asp Leu
           35                  40                  45
   cac ttg tct acg tcg acg gtt cgg gaa gca cta gag ttt agt gct ttg    192
   His Leu Ser Thr Ser Thr Val Arg Glu Ala Leu Glu Phe Ser Ala Leu
       50                  55                  60
   cta cgt caa ccc gcc tct gtt agc cgg aag gaa aag ata gac tat gct    240
   Leu Arg Gln Pro Ala Ser Val Ser Arg Lys Glu Lys Ile Asp Tyr Ala
   65                  70                  75                  80
   gac gag gtt atc aag ctc ctt ggt atg gag gcg tac gct gac gct gtt    288
   Asp Glu Val Ile Lys Leu Leu Gly Met Glu Ala Tyr Ala Asp Ala Val
                   85                  90                  95
   gtc ggt gtt ccg ggc gag gga ttg aac gtt gag cag cgg aag aga ttg    336
   Val Gly Val Pro Gly Glu Gly Leu Asn Val Glu Gln Arg Lys Arg Leu
               100                 105                 110
   acg att ggt gtg gag ctt gcc gcg aaa ccc cag ctc cta ctc ttc ctg    384
   Thr Ile Gly Val Glu Leu Ala Ala Lys Pro Gln Leu Leu Leu Phe Leu
           115                 120                 125
   gat gaa cca acc tcg gcc ctg                                        405
   Asp Glu Pro Thr Ser Ala Leu
       130                 135
<210> 31
   <211> 135
   <212> PRT
   <213> Penicillium chrysogenum
   <223> Protein sequence of the Penicillium chrysogenum ABC transporter aa8 (derived from the DNA as shown in SEQ ID NO. 29)
<400> 31
   Ser Gly Gly Gly Lys Thr Thr Leu Leu Asp Val Leu Ala Thr Arg Val
   1               5                   10                  15
   Gln Trp Gly Val Val Ser Gly Glu Met Leu Val Asp Gly Asn Pro Thr
               20                  25                  30
   Asp Gln Ser Phe Gln Arg Lys Thr Gly Tyr Val Gln Gln Gln Asp Leu
           35                  40                  45
   His Leu Ser Thr Ser Thr Val Arg Glu Ala Leu Glu Phe Ser Ala Leu
       50                  55                  60
   Leu Arg Gln Pro Ala Ser Val Ser Arg Lys Glu Lys Ile Asp Tyr Ala
   65                  70                  75                  80
   Asp Glu Val Ile Lys Leu Leu Gly Met Glu Ala Tyr Ala Asp Ala Val
                   85                  90                  95
   Val Gly Val Pro Gly Glu Gly Leu Asn Val Glu Gln Arg Lys Arg Leu
               100                 105                 110
   Thr Ile Gly Val Glu Leu Ala Ala Lys Pro Gln Leu Leu Leu Phe Leu
           115                 120                 125
   Asp Glu Pro Thr Ser Ala Leu
       130                 135
<210> 32
   <211> 411
   <212> DNA
   <213> Penicillium chrysogenum
<220>
   <223> Partial cDNA sequence of Penicillium chrysogenum ABC transporter aa10
<400> 32
   tcgggggcgg ggaagagtac cgtcgtcggt cttgtcgagc ggttctatct cccagtcgga 60
   ggtcaagtat tcttggacgg ccacgacatc cagactctca acttgcgatg gctacggcag 120
   cagatctccc tggtcagcca ggaacctgtt ctgtttggca caaccatcta tcaaaatatt 180
   cgccacggtc tgattggaac acgcttcgaa catgagtccg aggaaaagat caaggagctt 240
   attgagattg ccgccaagat ggccaacgct cacgaattta ttactgctct tccggaaggc 300
   tatgagacaa acgtcggcca gcgtggattc ttgctgtctg gcggccagaa gcaacgtatt 360
   gccatcgctc gtgctatggt cagcgacccc aagattttac ttctggacga a          411
<210> 33
   <211> 3133
   <212> DNA
   <213> Penicillium chrysogenum
<220>
   <223> Large, partial cDNA sequence of Penicillium chrysogenum ABC transporter aa10
<400> 33
   gcggccgcta ccaaaatctt tgcgactatc gaccgggact ccccgctgga ccctacttcg 60
   gacgaaggca tcatccttga ccacgtcgag ggtcatatcg agttccgcaa tgtgaaacac 120
   atttacccct cccgtccgga agttactgtc atgaatgacg tttccctcgg cattcctgct 180
   gggaacacca ccgcattggt cggaccctca ggttctggca agagcaccgt cgtcggtctt 240
   gtcgagcggt tctatctccc agtcggtggt caagtattct tggacggcca cgacatccag 300
   actctcaact tgcgatggct acggcagcag atctccctgg tcagccagga acctgttctg 360
   tttggcacaa ccatctatca aaatattcgc cacggtctga ttggaacacg cttcgaacat 420
   gagtccgagg aaaagatcaa ggagcttatt gagaatgccg ccaagatggc caacgctcac 480
   gaatttatta ctgctcttcc ggaaggctat gagacaaacg tcggccagcg tggattcttg 540
   ctgtctggcg gccagaagca acgtattgcc atcgctcgtg ctatggtcag cgaccccaag 600
   attttacttc tggacgaagc cacttcagcc ctggatacga agtccgaggg cgttgttcag 660
   gcagcacttg atcgcgctgc tgaaggccgc accaccatcg tcatcgccca tcgtctgtct 720
   actatcaagt ctgctcataa cattgtcgtc ttcgttcaag gatccatcgt tgagcagggc 780
   actcactccc agctgactga acacgacggt ccttacttca agcttgtcga ggcccagcgc 840
   atcaatgagg agaaggatgc agatgctttg gacgcggatg aggatgagga tggcctcgag 900
   gagatgacca agtctcacat cgcacgtgtc aagtcaattg ccagtggctc cacctgtgtg 960
   taggacgagg ccgaaacatt ccaggacgcc acgcatcgcc aggagtctcg caagtccgtt 1020
   tccagcgtta ttctatcaca gaagactgct gaaggcggca gaaagcactc gctcttgact 1080
   ctgattaagt ttatcggctc gttcaacaag gaagagcggt ggttcatggc catcggtctt 1140
   tgcttctcga ttcttgccgg ttgtggtcaa ccaactcagg ctttctcgta tgccaaagct 1200
   atcagcgctc tttccttgcc caaaacccaa tatgacaagc tgagatcgga tgccaacttc 1260
   tggtcactga tgttctttat cgtcggtatt gttcagatca tcaccttctc agtccacggt 1320
   atcgccttcg cgttcagctc tgagagattg attcgcaagg ctcgcggcaa tgccttccgt 1380
   gtcatgctcc gacaggatat caacttcttc gatcgtgagg agaatagcac tggcgctttg 1440
   acttctttcc tttcaactga aaccaagcac ctggccggta tcagtggtca aactcttgga 1500
   acaattctca tgacttcaac aacactcatt gcttcaattg tgatcgcgct tgctttcgga 1560
   tggaagcttg ctctcgtttg catgtccgtc atcccgattc ttctcggctg cggcttctac 1620
   cgtttctaca tgcttgccgc gttccaggca cgttccaagg ccgcttacga gggatcagcc 1680
   agctacgcct gcgaggctac atctgccatt cgcactgtgg cttcgcttac tcgtgagacc 1740
   gacgtttggt cattctacca tggtcaactc gagcgtcagg gccgcacaag cctgatctcg 1800
   gtattcaaat catcgctctt gtatgctgcc tcccaggcct tggtcttctt ttgtgtcgcc 1860
   cttggctttt ggtatggtgg aaccctgctc ggtcaccatg aatacgacgt gttccgcttc 1920
   ttcgtctgct tcagtgaaat cttgttcggt gctcagtccg ctggcacagt gttctcyttc 1980
   tcccccgaca tgggcaaggc caagaacgcc gccgccgagt tccttaggtt gttcgagcgt 2040
   cggcctacaa ttgacacatg gtccgaagag ggcgagaatt tggaccactg cgaaggaacc 2100
   attgagttca aggacgtgca cttccgctac cccacccgtc ccgagcagcc tgtgctccgc 2160
   ggtctgaacc tcactgtcaa gcctggccag tacattgccc tggtcggacc tagtggatgc 2220
   ggcaagagta cgaccattgc cctcctcgaa cgtttctacg atgctctttc cggaggtgtc 2280
   tacatagatg acaagaatat cgccgacctc aatgttaatt cctaccgcag tcacctggcg 2340
   ctggtcagcc aggagcccac cctctaccag ggtaccatta aggagaacat cctgctcggt 2400
   agccctaacc aagatccaac cgaggaggag cttgtccagg tctgcaagga cgccaacatc 2460
   tacgacttca tcatgtcgct ccccgaaggc tttaacaccg tcgtcggaag caagggaggc 2520
   atgttgtccg gtggccaaaa gcagcgtgtc gccatcgccc gcgccctcct gcgcaatcct 2580
   aaggtcctac ttctggatga agctacctcg gctctcgact ccgaatccga gaaggtcgtc 2640
   caagctgctc tggatgctgc tgctcgcggt cgtactacca tcgctgtggc tcaccgtctc 2700
   agtaccattc aaaaggccga tatcatctat gtcttcgacc aaggcaagat tgtcgagagc 2760
   ggtactcaca ctgagctctt gcgcaacaag ggtcgctact tcgagctggt caacctccag 2820
   tcccttggca gggccggcta aatcttttga tttattttca tatccatgta tcataccccc 2880
   attttctttc ttgattctat ctcacattcc ctgccatttc ccccatctgt tgcgtctctt 2940
   tcaatttctc ccaccagggt attcttgtga tagattattt tttcagcagc aaagacgcgg 3000
   aaagtcaggg tacattgtct ccccctcgat aaagataaaa aattaaaaaa catccactag 3060
   ggatgtagga ttagatgaat gaatacaatg aaatctcacg gacgcgtggg cggacgcgtg 3120
   gcgacgcgtg gtc                                                    3133
<210> 34
   <211> 8777
   <212> DNA
   <213> Penicillium chrysogenum
<220>
   <223> Complete genomic DNA sequence of Penicillium chrysogenum ABC transporter aa10
<400> 34
   gatccataaa gtaggtaccc tatcagtagt agaggctatt ggaaacaatg ccatctacag 60
   agtccaccgc tagagttccg ataccacttc ggagagtgaa accatcaaat tcgttcaaga 120
   aacgcttcct aagtgcctgt gcctaaagta atctattcct aggtcgacgg aaatcgtcca 180
   ttcttggtgt tgaagagagt tccgggtata acactgagag atgcgtaggg aactatgtcc 240
   gctaccgaac aagatcctat ccttgatgag attgtttcta tatgcgatat actcgcatct 300
   attacttcta accgactcca gaatgtatat ggaggcccgt aattggaacc gaaccttgct 360
   cactcggaac gagatttgtt agagccgtta actatgtttg agagtagggg atatttcttg 420
   cgggaagacc ttcatccaaa cccagagatt gaagagaggt tccatctcta ccattcaatt 480
   tgcagagtgg ttcatggaat ggtgggggac aaaagctaga tagtgaatca agccggtgac 540
   attttgtgta gattcggcga tagatctgtc tctttctctc cttggacaaa atccacgact 600
   atatctggtc accttcttct ttccaaggct gggtttcaag gccagttgct atccaattca 660
   tcgcgtacat accaactctc agcagtaata ttgctacata gccgcggaca aaattaccgg 720
   gcagctacta gctacctagc gataaggtag tgatggtcta tgtaccaaaa gagatgtagc 780
   cctagagtgt gagatagtcg atttcgaggc gtcccaagtg ggtgtttcta ttttcctttt 840
   cttggctctt ggctctttcc tttcttggta gtcatagggc tgaaggaatt cggggagaag 900
   aatggtcttg ttatgcccta ctagttgact tttatcgcag cagcgatcca cagtttgagg 960
   atcttcgact tggctgggaa gcttgatttt gggcgaagag agatatggtc attcgtagta 1020
   tctataggcg acgttatttt agtttgagct catatttccc tctatggtat atggttgcat 1080
   cataaacgtg gcgaactagg aggtctagta cgacgtggag ggaaaaggta cggatttgac 1140
   aattccaaaa taatccctga attcgaacgt atgttggttc tgatcacaaa tttacatatg 1200
   gtatagctct acccgacgtc agacagaaga ggtttaggta cgttgcacct ccaaccttag 1260
   gaaggaccaa ggaaggtgct gtgttatctc aggcctcctg aggtcaacaa ccttggaagt 1320
   aatcaacttg tacgaaatag gcaaaaggag gcaaaaggga gtcaaagcgt ccaatgtcgg 1380
   acatcggaca tgaaactacc cgacgcataa aaaaagtgga atcggtccag atcttctgtc 1440
   ttagcggatc aaaattcgga cgaaatcctc cgtaatataa ctaatatacc tacatacctt 1500
   ccaaggttag gtaggattat gactcaggat atccgtattc gttctcgtgt ttcgcgtcat 1560
   gatgatgtgt ttcgtgttgt ccgtgttgta ctctctgcgt taaggtcaca gtatgtacta 1620
   agtgccacat gcatgcatgt ttcaatcatg ctggacctgc gtcaagcatt aagtaaagaa 1680
   gcaaatcctc cgtgctaact agtgttccct agaaaggggg cgcgttcgag ggatgccctt 1740
   gcacgaggaa tgtggaggta catcataagg aatgtccagc ctctgtataa acagattagc 1800
   aatctgtacg tacagggaac atacacccgt tggaatagag agataagacc aagttaggat 1860
   ctaggcagca ctcagtgata tagtaaatat cgtaaacggc gtaaacggcg taaatacgtc 1920
   gattagaaat tgcaaattgc ataaagacat gtcggagttc ggactacccg acccgcccaa 1980
   aaaaaataag cccttccgaa gtacctaaag taagtataat ccaagacatt tagatctcac 2040
   tccttccttc acccctttcg ttaatcttta ctcttcttaa tctaatactc tcatatccct 2100
   taatctctct aatatctctt ctttctgcta gcgccttgat tcatcccttc acgtctcgcc 2160
   tcttcagatc aaatcctcat cttcaagttc ttcatggagt agagattcaa acccaaaagc 2220
   ttcaacatgc aacagcccca ggagggcgtg acggccgttg agaaggtggc cgacggtcct 2280
   cagcttaacg agaagcccgt cgacaaacgt gattccgatt ccgcttccac atcaacagca 2340
   aaggaacacg aaagcaagac tccagtcgcg tcagacgaag atgcgctcta tgctcactta 2400
   cctgagcatg aaaaggacat cttgaaacaa cagctggatg cgcctcctgt caacatctct 2460
   tacttcggtc tctatcgata tgcctcccgc atagacatac tcatcattgt catcagcaca 2520
   ctctgcgcta tcgccgccgg tgctgccttg cctctcttca ctattctctt cggctccttg 2580
   gccacggctt tccaaaagat tatgctccgc actatcccct acgatgaatt ctatgatcaa 2640
   ttgaccacca acgtgttgta cttcatctat ctcggcatcg gagaattcgt caccgtctac 2700
   gtcagcaccg tcggtttcat ctacaccgga gagcatgtca cccagaagat tcgagagcac 2760
   tacctcgaag ccatcctacg tcagaacatc gcctacttcg acaagcttgg tgcaggtgaa 2820
   gtgactaccc gcatcacggc tgacaccaac cttattcagg atggtgtctc cgaaaaggtc 2880
   ggtttaaccc ttactgccgt cgctaccttc gtgactgctt tcatcgttgc atacatcaag 2940
   tatgccccct tggctggcat atgtacttcg actatggtcg cgctggtcct catcatgggc 3000
   ggaggctccc aactgatcat caagtttggc aagctttccc tggaaagtgc cggtgccggt 3060
   ggaactgtgg cggaggaagt catcagctcg atccgcaatg cgaccgcatt cggtactcaa 3120
   gacaagctcg caaagcagta cgagtcccac ttgcttcgcg cagagcgctg gggcatgcgc 3180
   ctccaaatga gtcttgccgt gatggtcggt atcatgtttg gactcatgtt catgaactac 3240
   ggcctcggat tctggatggg atctcagttc ctggtggatg gtaaggttga tgtcgggcaa 3300
   gttttgacca tcttgatggc tattcttatc ggttctttct ccctgggcaa tgtcagcccg 3360
   aacgcgtcag catttaccaa tgctgtggcg gccgctacca aaatctttgc gactatcgac 3420
   cgggactccc cgctggaccc tacttcggac gaaggcatca tccttgacca cgtcgagggt 3480
   catatcgagt tccgcaatgt gaaacacatt tacccctccc gtccggaagt tactgtcatg 3540
   aatgacgttt ccctcggcat tcctgctggg aacaccaccg cattggtcgg accctcaggt 3600
   tctggcaaga gcaccgtcgt cggtcttgtc gagcggttct atctcccagt cggtggtcaa 3660
   gtattcttgg acggccacga catccagact ctcaacttgc gatggctacg gcagcagatc 3720
   tccctggtca gccaggaacc tgttctgttt ggcacaacca tctatcaaaa tattcgccac 3780
   ggtctgattg gaacacgctt cgaacatgag tccgaggaaa agatcaagga gcttattgag 3840
   aatgccgcca agatggccaa cgctcacgaa tttattactg ctcttccgga aggctatgag 3900
   acaaacgtcg gccagcgtgg attcttgctg tctggcggcc agaagcaacg tattgccatc 3960
   gctcgtgcta tggtcagcga ccccaagatt ttacttctgg acgaagccac ttcagccctg 4020
   gatacgaagt ccgagggcgt tgttcaggca gcacttgatc gcgctgctga aggccgcacc 4080
   accatcgtca tcgcccatcg tctgtctact atcaagtctg ctcataacat tgtcgtcttc 4140
   gttcaaggat ccatcgttga gcagggcact cactcccagc tgactgaaca cgacggtcct 4200
   tacttcaagc ttgtcgaggc ccagcgcatc aatgaggaga aggatgcaga tgctttggac 4260
   gcggatgagg atgaggatgg cctcgaggag atgaccaagt ctcacatcgc acgtgtcaag 4320
   tcaattgcca gtggctccac ctgtgtgaag gacgaggccg aaacattcca ggacgccatg 4380
   catcgccagg agtctcgcaa gtccgtttcc agcgttattc tatcacagaa gactgctgaa 4440
   ggcggcagaa agcactcgct cttgactctg attaagttta tcggctcgtt caacaaggaa 4500
   gagcggtggt tcatggccat cggtctttgc ttctcgattc ttgccggttg tggtcaacca 4560
   actcaggctt tcttgtatgc caaagctatc agcgctcttt ccttgcccaa aacccaatat 4620
   gacaagctga gatcggatgc caacttctgg tcactgatgt tctttatcgt cggtattgtt 4680
   cagatcatca ccttctcagt ccacggtatc gccttcgcgt tcagctctga gagattgatt 4740
   cgcaaggctc gcggcaatgc cttccgtgtc atgctccgac aggatatcaa cttcttcgat 4800
   cgtgaggaga atagcactgg cgctttgact tctttccttt caactgaaac caagcacctg 4860
   gccggtatca gtggtcaaac tcttggaaca attctcatga cttcaacaac actcattgct 4920
   tcaattgtga tcgcgcttgc tttcggatgg aagcttgctc tcgtttgcat gtccgtcatc 4980
   ccgattcttc tcggctgcgg cttctaccgt ttctacatgc ttgccgcgtt ccaggcacgt 5040
   tccaaggccg cttacgaggg atcagccagc tacgcctgcg aggctacatc tgccattcgc 5100
   actgtggctt cgcttactcg tgagaccgac gtttggtcat tctaccatgg tcaactcgag 5160
   cgtcagggcc gcacaagcct gatctcggta ttcaaatcat cgctcttgta tgctgcctcc 5220
   caggccttgg tcttcttttg tgtcgccctt ggcttttggt atggtggaac cctgctcggt 5280
   caccatgaat acgacgtgtt ccgcttcttc gtctgcttca gtgaaatctt gttcggtgct 5340
   cagtccgctg gcacagtgtt ctccttctcc cccgacatgg gcaaggccaa gaacgccgcc 5400
   gccgagttcc ttaggttgtt cgagcgtcgg cctacaattg acacatggtc cgaagagggc 5460
   gagaatttgg accactgcga aggaaccatt gagttcaagg acgtgcactt ccgctacccc 5520
   acccgtcccg agcagcctgt gctccgcggt ctgaacctca ctgtcaagcc tggccagtac 5580
   attgccctgg tcggacctag tggatgcggc aagagtacga ccattgccct cctcgaacgt 5640
   ttctacgatg ctctttccgg aggtgtctac atagatgaca agaatatcgc cgacctcaat 5700
   gttaattcct accgcagtca cctggcgctg gtcagccagg agcccaccct ctaccagggt 5760
   accattaagg agaacatcct gctcggtagc cctaaccaag atccaaccga ggaggagctt 5820
   gtccaggtct gcaaggacgc caacatctac gacttcatca tgtcgctccc cgaaggcttt 5880
   aacaccgtcg tcggaagcaa gggaggcatg ttgtccggtg gccaaaagca gcgtgtcgcc 5940
   atcgcccgcg ccctcctgcg caatcctaag gtcctacttc tggatgaagc tacctcggct 6000
   ctcgactccg aatccgagaa ggtcgtccaa gctgctctgg atgctgctgc tcgcggtcgt 6060
   actaccatcg ctgtggctca ccgtctcagt accattcaaa aggccgatat catctatgtc 6120
   ttcgaccaag gcaagattgt cgagagcggt actcacactg agctcttgcg caacaagggt 6180
   cgctacttcg agctggtcaa cctccagtcc cttggcaggg ccggctaaat cttttgattt 6240
   attttcatat ccatgtatca tacccccatt ttctttcttg attctatctc acattccctg 6300
   ccatttcccc catctgttgc gtctctttca atttctccca ccagggtatt cttgtgatag 6360
   attatttttt cagcagcaaa gacgcggaaa gtcagggtac attgtctccc ctcgataaag 6420
   ataaaaaatt aaaaaacatc cactagggat gtaggattag atgaatgaat acaatgaaat 6480
   ctcaattcaa caccacccgg tttcgctcca aactccatcc gttcagttgg atcccttcaa 6540
   accatttcaa gtcctttcaa ttagtagacc tcttgccgtg tagcccttca atcctcctcc 6600
   gaaaacccaa actccctcac cacctcgacc tgcctcaccc ccctggcatc agcaataagc 6660
   tccatcaatc cctgcccaac atccctgatg tcattgtact catgcaagag ctgggtatag 6720
   cgtacgcatg tggtattgta atcgtccctg ggataaccaa tggttagaga tgtaagatgc 6780
   aatgtttcga tgtttatgta gactgcatgc aattggggga gctgaagggg ttttacttga 6840
   gttgggcggt cactgaagct aagtcggctt cgagcctcgc atatttagct tctagacttg 6900
   caatcgaagc gcggaggttg tcctacgtgg gtgttagtgc ttaacatgat gaacacgggt 6960
   agtggcaggg tgggggatac tacagacgta ccactcgttc ctggtctcgg aaggacctgg 7020
   tttctttggc ttgatccatg taggtgtgca attagctgca tatgcatatg tattgtggta 7080
   cgtgggatgc aagagaggaa gaagttatcc atgtacttac ccaagtaggt aggtacttcg 7140
   taccccagaa ttatatactt ctgaccaatc acagccacgc tgaataatcc actccaagcc 7200
   aaggtttctg cttgcgcgtc cttgattttg ctgtgatgtt attttttaaa agttgtttaa 7260
   taagtaaatg ataaaatagc tacctatatc acttggctat tcgagaatac agccctggag 7320
   atcaaaaaga cggttctttg actccacgcg tcgtcccgcc taatcggatg ccccggattg 7380
   ggtttacctg ttgtccgcat ttgttctctt ctcccttgca actccctacc tcaaccacac 7440
   catggcatat cttcgccagt ccttatggac tttgtctgta ttagtggcct cggctctcgc 7500
   ccatggcggc cacgaggctg ttcctgaggg cgaatctatc tcccaggatc caggagaaga 7560
   attcctgcag cccgggggat ccactagttc tagagcggcc gccaccgcgg tggagctcca 7620
   gcttttgttc cctttagtga gggttaattt cgagcttggc gtaatcatgg tcatagctgt 7680
   ttcctgtgtg aaattgttat ccgctcacaa ttccacacaa catacgagcc ggaagcataa 7740
   agtgtaaagc ctggggtgcc taatgagtga gctaactcac attaattgcg ttgcgctcac 7800
   tgcccgcttt ccagtcggga aacctgtcgt gccagctgca ttaatgaatc ggccaacgcg 7860
   cggggagagg cggtttgcgt attgggcgct cttccgcttt ctcgctcact gactcgctgc 7920
   gctcggtcgt tcggctgcgg cgagcggtat cagctcactc aaaggcggta atacggttat 7980
   ccacagaatc aggggataac gcaggaaaga acatgtgagc aaaaggccag caaaaggcca 8040
   ggaaccgtaa aaaggccgcg ttgctggcgt ttttccatag gctccgcccc cctgacgagc 8100
   atcacaaaaa tcgacgctca agtcagaggt ggcgaaaccc gacaggacta taaagatacc 8160
   aggcgtttcc ccctggaagc tccctcgtgc gctctcctgt tccgaccctg ccgcttaccg 8220
   gatacctgtc cgcctttctc ccttcgggaa gcgtggcgct ttctcatagc tcacgctgta 8280
   ggtatctcag ttcggtgtag gtcgttcgct ccaagctggg ctgtgtgcac gaaccccccg 8340
   ttcagcccga ccgctgcgcc ttatccggta actatcgtct tgagtccaac ccggtaagac 8400
   acgacttatc gccactggca gcagccactg gtaacaggat tagcagagcg aggtatgtag 8460
   gcggtgctac agagttcttg aagtggtggc ctaactacgg ctacactaga agaacagtat 8520
   ttggtatctg cgctctgctg aagccagtta ccttcggaaa aagagttggt agctcttgat 8580
   ccggcaaaca aaccaccgct ggtagcggtg gtttttttgt ttgcaagcag cagattacgc 8640
   gcagaaaaaa aggatctcaa gaagatcctt tgatcttttc tacggggtct gacgctcagt 8700
   ggaacgaaaa ctcacgttaa gggattttgg tcatgagatt atcaaaaagg atcttcacct 8760
   agtgtgggcg gacaaaa                                                8777
<210> 35
   <211> 8777
   <212> DNA
   <213> Penicillium chrysogenum
<220>
   <223> Translation of the comlete genomic DNA sequence of Penicillium chrysogenum ABC transporter aa10
<220>
   <221> CDS
   <222> (2227)..(6225)
<400> 35
   gatccataaa gtaggtaccc tatcagtagt agaggctatt ggaaacaatg ccatctacag 60
   agtccaccgc tagagttccg ataccacttc ggagagtgaa accatcaaat tcgttcaaga 120
   aacgcttcct aagtgcctgt gcctaaagta atctattcct aggtcgacgg aaatcgtcca 180
   ttcttggtgt tgaagagagt tccgggtata acactgagag atgcgtaggg aactatgtcc 240
   gctaccgaac aagatcctat ccttgatgag attgtttcta tatgcgatat actcgcatct 300
   attacttcta accgactcca gaatgtatat ggaggcccgt aattggaacc gaaccttgct 360
   cactcggaac gagatttgtt agagccgtta actatgtttg agagtagggg atatttcttg 420
   cgggaagacc ttcatccaaa cccagagatt gaagagaggt tccatctcta ccattcaatt 480
   tgcagagtgg ttcatggaat ggtgggggac aaaagctaga tagtgaatca agccggtgac 540
   attttgtgta gattcggcga tagatctgtc tctttctctc cttggacaaa atccacgact 600
   atatctggtc accttcttct ttccaaggct gggtttcaag gccagttgct atccaattca 660
   tcgcgtacat accaactctc agcagtaata ttgctacata gccgcggaca aaattaccgg 720
   gcagctacta gctacctagc gataaggtag tgatggtcta tgtaccaaaa gagatgtagc 780
   cctagagtgt gagatagtcg atttcgaggc gtcccaagtg ggtgtttcta ttttcctttt 840
   cttggctctt ggctctttcc tttcttggta gtcatagggc tgaaggaatt cggggagaag 900
   aatggtcttg ttatgcccta ctagttgact tttatcgcag cagcgatcca cagtttgagg 960
   atcttcgact tggctgggaa gcttgatttt gggcgaagag agatatggtc attcgtagta 1020
   tctataggcg acgttatttt agtttgagct catatttccc tctatggtat atggttgcat 1080
   cataaacgtg gcgaactagg aggtctagta cgacgtggag ggaaaaggta cggatttgac 1140
   aattccaaaa taatccctga attcgaacgt atgttggttc tgatcacaaa tttacatatg 1200
   gtatagctct acccgacgtc agacagaaga ggtttaggta cgttgcacct ccaaccttag 1260
   gaaggaccaa ggaaggtgct gtgttatctc aggcctcctg aggtcaacaa ccttggaagt 1320
   aatcaacttg tacgaaatag gcaaaaggag gcaaaaggga gtcaaagcgt ccaatgtcgg 1380
   acatcggaca tgaaactacc cgacgcataa aaaaagtgga atcggtccag atcttctgtc 1440
   ttagcggatc aaaattcgga cgaaatcctc cgtaatataa ctaatatacc tacatacctt 1500
   ccaaggttag gtaggattat gactcaggat atccgtattc gttctcgtgt ttcgcgtcat 1560
   gatgatgtgt ttcgtgttgt ccgtgttgta ctctctgcgt taaggtcaca gtatgtacta 1620
   agtgccacat gcatgcatgt ttcaatcatg ctggacctgc gtcaagcatt aagtaaagaa 1680
   gcaaatcctc cgtgctaact agtgttccct agaaaggggg cgcgttcgag ggatgccctt 1740
   gcacgaggaa tgtggaggta catcataagg aatgtccagc ctctgtataa acagattagc 1800
   aatctgtacg tacagggaac atacacccgt tggaatagag agataagacc aagttaggat 1860
   ctaggcagca ctcagtgata tagtaaatat cgtaaacggc gtaaacggcg taaatacgtc 1920
   gattagaaat tgcaaattgc ataaagacat gtcggagttc ggactacccg acccgcccaa 1980
   aaaaaataag cccttccgaa gtacctaaag taagtataat ccaagacatt tagatctcac 2040
   tccttccttc acccctttcg ttaatcttta ctcttcttaa tctaatactc tcatatccct 2100
   taatctctct aatatctctt ctttctgcta gcgccttgat tcatcccttc acgtctcgcc 2160
   tcttcagatc aaatcctcat cttcaagttc ttcatggagt agagattcaa acccaaaagc 2220
   ttcaac atg caa cag ccc cag gag ggc gtg acg gcc gtt gag aag gtg    2268
          Met Gln Gln Pro Gln Glu Gly Val Thr Ala Val Glu Lys Val
           1               5                   10
   gcc gac ggt cct cag ctt aac gag aag ccc gtc gac aaa cgt gat tcc   2316
   Ala Asp Gly Pro Gln Leu Asn Glu Lys Pro Val Asp Lys Arg Asp Ser
    15                 20                  25                  30
   gat tcc gct tcc aca tca aca gca aag gaa cac gaa agc aag act cca   2364
   Asp Ser Ala Ser Thr Ser Thr Ala Lys Glu His Glu Ser Lys Thr Pro
                   35                  40                  45
   gtc gcg tca gac gaa gat gcg ctc tat gct cac tta cct gag cat gaa   2412
   Val Ala Ser Asp Glu Asp Ala Leu Tyr Ala His Leu Pro Glu His Glu
                   50                  55                  60
   aag gac atc ttg aaa caa cag ctg gat gcg cct cct gtc aac atc tct     2460
   Lys Asp Ile Leu Lys Gln Gln Leu Asp Ala Pro Pro Val Asn Ile Ser
               65                  70                  75
   tac ttc ggt ctc tat cga tat gcc tcc cgc ata gac ata ctc atc att     2508
   Tyr Phe Gly Leu Tyr Arg Tyr Ala Ser Arg Ile Asp Ile Leu Ile Ile
           80                  85                  90
   gtc atc agc aca ctc tgc gct atc gcc gcc ggt gct gcc ttg cct ctc     2556
   Val Ile Ser Thr Leu Cys Ala Ile Ala Ala Gly Ala Ala Leu Pro Leu
    95                 100                 105                 110
   ttc act att ctc ttc ggc tcc ttg gcc acg gct ttc caa aag att atg     2604
   Phe Thr Ile Leu Phe Gly Ser Leu Ala Thr Ala Phe Gln Lys Ile Met
                   115                 120                 125
   ctc cgc act atc ccc tac gat gaa ttc tat gat caa ttg acc acc aac     2652
   Leu Arg Thr Ile Pro Tyr Asp Glu Phe Tyr Asp Gln Leu Thr Thr Asn
               130                 135                 140
   gtg ttg tac ttc atc tat ctc ggc atc gga gaa ttc gtc acc gtc tac     2700
   Val Leu Tyr Phe Ile Tyr Leu Gly Ile Gly Glu Phe Val Thr Val Tyr
           145                 150                 155
   gtc agc acc gtc ggt ttc atc tac acc gga gag cat gtc acc cag aag     2748
   Val Ser Thr Val Gly Phe Ile Tyr Thr Gly Glu His Val Thr Gln Lys
       160                 165                 170
   att cga gag cac tac ctc gaa gcc atc cta cgt cag aac atc gcc tac     2796
   Ile Arg Glu His Tyr Leu Glu Ala Ile Leu Arg Gln Asn Ile Ala Tyr
   175                 180                 185                 190
   ttc gac aag ctt ggt gca ggt gaa gtg act acc cgc atc acg gct gac     2844
   Phe Asp Lys Leu Gly Ala Gly Glu Val Thr Thr Arg Ile Thr Ala Asp
                   195                 200                 205
   acc aac ctt att cag gat ggt gtc tcc gaa aag gtc ggt tta acc ctt     2892
   Thr Asn Leu Ile Gln Asp Gly Val Ser Glu Lys Val Gly Leu Thr Leu
               210                 215                 220
   act gcc gtc gct acc ttc gtg act gct ttc atc gtt gca tac atc aag     2940
   Thr Ala Val Ala Thr Phe Val Thr Ala Phe Ile Val Ala Tyr Ile Lys
           225                 230                 235
   tat gcc ccc ttg gct ggc ata tgt act tcg act atg gtc gcg ctg gtc     2988
   Tyr Ala Pro Leu Ala Gly Ile Cys Thr Ser Thr Met Val Ala Leu Val
       240                 245                 250
   ctc atc atg ggc gga ggc tcc caa ctg atc atc aag ttt ggc aag ctt     3036
   Leu Ile Met Gly Gly Gly Ser Gln Leu Ile Ile Lys Phe Gly Lys Leu
   255                 260                 265                 270
   tcc ctg gaa agt gcc ggt gcc ggt gga act gtg gcg gag gaa gtc atc     3084
   Ser Leu Glu Ser Ala Gly Ala Gly Gly Thr Val Ala Glu Glu Val Ile
                   275                 280                 285
   agc tcg atc cgc aat gcg acc gca ttc ggt act caa gac aag ctc gca     3132
   Ser Ser Ile Arg Asn Ala Thr Ala Phe Gly Thr Gln Asp Lys Leu Ala
               290                 295                 300
   aag cag tac gag tcc cac ttg ctt cgc gca gag cgc tgg ggc atg cgc    3180
   Lys Gln Tyr Glu Ser His Leu Leu Arg Ala Glu Arg Trp Gly Met Arg
           305                 310                 315
   ctc caa atg agt ctt gcc gtg atg gtc ggt atc atg ttt gga ctc atg    3228
   Leu Gln Met Ser Leu Ala Val Met Val Gly Ile Met Phe Gly Leu Met
       320                 325                 330
   ttc atg aac tac ggc ctc gga ttc tgg atg gga tct cag ttc ctg gtg    3276
   Phe Met Asn Tyr Gly Leu Gly Phe Trp Met Gly Ser Gln Phe Leu Val
   335                 340                 345                 350
   gat ggt aag gtt gat gtc ggg caa gtt ttg acc atc ttg atg gct att    3324
   Asp Gly Lys Val Asp Val Gly Gln Val Leu Thr Ile Leu Met Ala Ile
                   355                 360                 365
   ctt atc ggt tct ttc tcc ctg ggc aat gtc agc ccg aac gcg tca gca    3372
   Leu Ile Gly Ser Phe Ser Leu Gly Asn Val Ser Pro Asn Ala Ser Ala
               370                 375                 380
   ttt acc aat gct gtg gcg gcc gct acc aaa atc ttt gcg act atc gac    3420
   Phe Thr Asn Ala Val Ala Ala Ala Thr Lys Ile Phe Ala Thr Ile Asp
           385                 390                 395
   cgg gac tcc ccg ctg gac cct act tcg gac gaa ggc atc atc ctt gac    3468
   Arg Asp Ser Pro Leu Asp Pro Thr Ser Asp Glu Gly Ile Ile Leu Asp
       400                 405                 410
   cac gtc gag ggt cat atc gag ttc cgc aat gtg aaa cac att tac ccc    3516
   His Val Glu Gly His Ile Glu Phe Arg Asn Val Lys His Ile Tyr Pro
   415                 420                 425                 430
   tcc cgt ccg gaa gtt act gtc atg aat gac gtt tcc ctc ggc att cct    3564
   Ser Arg Pro Glu Val Thr Val Met Asn Asp Val Ser Leu Gly Ile Pro
                   435                 440                 445
   gct ggg aac acc acc gca ttg gtc gga ccc tca ggt tct ggc aag agc    3612
   Ala Gly Asn Thr Thr Ala Leu Val Gly Pro Ser Gly Ser Gly Lys Ser
               450                 455                 460
   acc gtc gtc ggt ctt gtc gag cgg ttc tat ctc cca gtc ggt ggt caa    3660
   Thr Val Val Gly Leu Val Glu Arg Phe Tyr Leu Pro Val Gly Gly Gln
           465                 470                 475
   gta ttc ttg gac ggc cac gac atc cag act ctc aac ttg cga tgg cta    3708
   Val Phe Leu Asp Gly His Asp Ile Gln Thr Leu Asn Leu Arg Trp Leu
       480                 485                 490
   cgg cag cag atc tcc ctg gtc agc cag gaa cct gtt ctg ttt ggc aca    3756
   Arg Gln Gln Ile Ser Leu Val Ser Gln Glu Pro Val Leu Phe Gly Thr
   495                 500                 505                 510
   acc atc tat caa aat att cgc cac ggt ctg att gga aca cgc ttc gaa    3804
   Thr Ile Tyr Gln Asn Ile Arg His Gly Leu Ile Gly Thr Arg Phe Glu
                   515                 520                 525
   cat gag tcc gag gaa aag atc aag gag ctt att gag aat gcc gcc aag    3852
   His Glu Ser Glu Glu Lys Ile Lys Glu Leu Ile Glu Asn Ala Ala Lys
               530                 535                 540
   atg gcc aac gct cac gaa ttt att act gct ctt ccg gaa ggc tat gag    3900
   Met Ala Asn Ala His Glu Phe Ile Thr Ala Leu Pro Glu Gly Tyr Glu
           545                 550                 555
   aca aac gtc ggc cag cgt gga ttc ttg ctg tct ggc ggc cag aag caa    3948
   Thr Asn Val Gly Gln Arg Gly Phe Leu Leu Ser Gly Gly Gln Lys Gln
       560                 565                 570
   cgt att gcc atc gct cgt gct atg gtc agc gac ccc aag att tta ctt    3996
   Arg Ile Ala Ile Ala Arg Ala Met Val Ser Asp Pro Lys Ile Leu Leu
   575                 580                 585                 590
   ctg gac gaa gcc act tca gcc ctg gat acg aag tcc gag ggc gtt gtt    4044
   Leu Asp Glu Ala Thr Ser Ala Leu Asp Thr Lys Ser Glu Gly Val Val
                   595                 600                 605
   cag gca gca ctt gat cgc gct gct gaa ggc cgc acc acc atc gtc atc    4092
   Gln Ala Ala Leu Asp Arg Ala Ala Glu Gly Arg Thr Thr Ile Val Ile
               610                 615                 620
   gcc cat cgt ctg tct act atc aag tct gct cat aac att gtc gtc ttc    4140
   Ala His Arg Leu Ser Thr Ile Lys Ser Ala His Asn Ile Val Val Phe
           625                 630                 635
   gtt caa gga tcc atc gtt gag cag ggc act cac tcc cag ctg act gaa    4188
   Val Gln Gly Ser Ile Val Glu Gln Gly Thr His Ser Gln Leu Thr Glu
       640                 645                 650
   cac gac ggt cct tac ttc aag ctt gtc gag gcc cag cgc atc aat gag    4236
   His Asp Gly Pro Tyr Phe Lys Leu Val Glu Ala Gln Arg Ile Asn Glu
   655                 660                 665                 670
   gag aag gat gca gat gct ttg gac gcg gat gag gat gag gat ggc ctc    4284
   Glu Lys Asp Ala Asp Ala Leu Asp Ala Asp Glu Asp Glu Asp Gly Leu
                   675                 680                 685
   gag gag atg acc aag tct cac atc gca cgt gtc aag tca att gcc agt    4332
   Glu Glu Met Thr Lys Ser His Ile Ala Arg Val Lys Ser Ile Ala Ser
               690                 695                 700
   ggc tcc acc tgt gtg aag gac gag gcc gaa aca ttc cag gac gcc atg    4380
   Gly Ser Thr Cys Val Lys Asp Glu Ala Glu Thr Phe Gln Asp Ala Met
           705                 710                 715
   cat cgc cag gag tct cgc aag tcc gtt tcc agc gtt att cta tca cag    4428
   His Arg Gln Glu Ser Arg Lys Ser Val Ser Ser Val Ile Leu Ser Gln
       720                 725                 730
   aag act gct gaa ggc ggc aga aag cac tcg ctc ttg act ctg att aag    4476
   Lys Thr Ala Glu Gly Gly Arg Lys His Ser Leu Leu Thr Leu Ile Lys
   735                 740                 745                 750
   ttt atc ggc tcg ttc aac aag gaa gag cgg tgg ttc atg gcc atc ggt    4524
   Phe Ile Gly Ser Phe Asn Lys Glu Glu Arg Trp Phe Met Ala Ile Gly
                   755                 760                 765
   ctt tgc ttc tcg att ctt gcc ggt tgt ggt caa cca act cag gct ttc    4572
   Leu Cys Phe Ser Ile Leu Ala Gly Cys Gly Gln Pro Thr Gln Ala Phe
               770                 775                 780
   ttg tat gcc aaa gct atc agc gct ctt tcc ttg ccc aaa acc caa tat    4620
   Leu Tyr Ala Lys Ala Ile Ser Ala Leu Ser Leu Pro Lys Thr Gln Tyr
           785                 790                 795
   gac aag ctg aga tcg gat gcc aac ttc tgg tca ctg atg ttc ttt atc      4668
   Asp Lys Leu Arg Ser Asp Ala Asn Phe Trp Ser Leu Met Phe Phe Ile
       800                 805                 810
   gtc ggt att gtt cag atc atc acc ttc tca gtc cac ggt atc gcc ttc      4716
   Val Gly Ile Val Gln Ile Ile Thr Phe Ser Val His Gly Ile Ala Phe
   815                 820                 825                 830
   gcg ttc agc tct gag aga ttg att cgc aag gct cgc ggc aat gcc ttc      4764
   Ala Phe Ser Ser Glu Arg Leu Ile Arg Lys Ala Arg Gly Asn Ala Phe
                   835                 840                 845
   cgt gtc atg ctc cga cag gat atc aac ttc ttc gat cgt gag gag aat      4812
   Arg Val Met Leu Arg Gln Asp Ile Asn Phe Phe Asp Arg Glu Glu Asn
               850                 855                 860
   agc act ggc gct ttg act tct ttc ctt tca act gaa acc aag cac ctg      4860
   Ser Thr Gly Ala Leu Thr Ser Phe Leu Ser Thr Glu Thr Lys His Leu
           865                 870                 875
   gcc ggt atc agt ggt caa act ctt gga aca att ctc atg act tca aca      4908
   Ala Gly Ile Ser Gly Gln Thr Leu Gly Thr Ile Leu Met Thr Ser Thr
       880                 885                 890
   aca ctc att gct tca att gtg atc gcg ctt gct ttc gga tgg aag ctt      4956
   Thr Leu Ile Ala Ser Ile Val Ile Ala Leu Ala Phe Gly Trp Lys Leu
   895                 900                 905                 910
   gct ctc gtt tgc atg tcc gtc atc ccg att ctt ctc ggc tgc ggc ttc      5004
   Ala Leu Val Cys Met Ser Val Ile Pro Ile Leu Leu Gly Cys Gly Phe
                   915                 920                 925
   tac cgt ttc tac atg ctt gcc gcg ttc cag gca cgt tcc aag gcc gct      5052
   Tyr Arg Phe Tyr Met Leu Ala Ala Phe Gln Ala Arg Ser Lys Ala Ala
               930                 935                 940
   tac gag gga tca gcc agc tac gcc tgc gag gct aca tct gcc att cgc      5100
   Tyr Glu Gly Ser Ala Ser Tyr Ala Cys Glu Ala Thr Ser Ala Ile Arg
           945                 950                 955
   act gtg gct tcg ctt act cgt gag acc gac gtt tgg tca ttc tac cat      5148
   Thr Val Ala Ser Leu Thr Arg Glu Thr Asp Val Trp Ser Phe Tyr His
       960                 965                 970
   ggt caa ctc gag cgt cag ggc cgc aca agc ctg atc tcg gta ttc aaa      5196
   Gly Gln Leu Glu Arg Gln Gly Arg Thr Ser Leu Ile Ser Val Phe Lys
   975                 980                 985                 990
   tca tcg ctc ttg tat gct gcc tcc cag gcc ttg gtc ttc ttt tgt gtc      5244
   Ser Ser Leu Leu Tyr Ala Ala Ser Gln Ala Leu Val Phe Phe Cys Val
                   995                 1000                1005
   gcc ctt ggc ttt tgg tat ggt gga acc ctg ctc ggt cac cat gaa tac      5292
   Ala Leu Gly Phe Trp Tyr Gly Gly Thr Leu Leu Gly His His Glu Tyr
               1010                1015                1020
   gac gtg ttc cgc ttc ttc gtc tgc ttc agt gaa atc ttg ttc ggt gct      5340
   Asp Val Phe Arg Phe Phe Val Cys Phe Ser Glu Ile Leu Phe Gly Ala
           1025                1030                1035
   cag tcc gct ggc aca gtg ttc tcc ttc tcc ccc gac atg ggc aag gcc    5388
   Gln Ser Ala Gly Thr Val Phe Ser Phe Ser Pro Asp Met Gly Lys Ala
       1040                1045                1050
   aag aac gcc gcc gcc gag ttc ctt agg ttg ttc gag cgt cgg cct aca    5436
   Lys Asn Ala Ala Ala Glu Phe Leu Arg Leu Phe Glu Arg Arg Pro Thr
   1055                1060                1065                1070
   att gac aca tgg tcc gaa gag ggc gag aat ttg gac cac tgc gaa gga    5484
   Ile Asp Thr Trp Ser Glu Glu Gly Glu Asn Leu Asp His Cys Glu Gly
                   1075                1080                1085
   acc att gag ttc aag gac gtg cac ttc cgc tac ccc acc cgt ccc gag    5532
   Thr Ile Glu Phe Lys Asp Val His Phe Arg Tyr Pro Thr Arg Pro Glu
               1090                1095                1100
   cag cct gtg ctc cgc ggt ctg aac ctc act gtc aag cct ggc cag tac    5580
   Gln Pro Val Leu Arg Gly Leu Asn Leu Thr Val Lys Pro Gly Gln Tyr
           1105                1110                1115
   att gcc ctg gtc gga cct agt gga tgc ggc aag agt acg acc att gcc    5628
   Ile Ala Leu Val Gly Pro Ser Gly Cys Gly Lys Ser Thr Thr Ile Ala
       1120                1125                1130
   ctc ctc gaa cgt ttc tac gat gct ctt tcc gga ggt gtc tac ata gat    5676
   Leu Leu Glu Arg Phe Tyr Asp Ala Leu Ser Gly Gly Val Tyr Ile Asp
   1135                1140                1145                1150
   gac aag aat atc gcc gac ctc aat gtt aat tcc tac cgc agt cac ctg    5724
   Asp Lys Asn Ile Ala Asp Leu Asn Val Asn Ser Tyr Arg Ser His Leu
                   1155                1160                1165
   gcg ctg gtc agc cag gag ccc acc ctc tac cag ggt acc att aag gag    5772
   Ala Leu Val Ser Gln Glu Pro Thr Leu Tyr Gln Gly Thr Ile Lys Glu
               1170                1175                1180
   aac atc ctg ctc ggt agc cct aac caa gat cca acc gag gag gag ctt    5820
   Asn Ile Leu Leu Gly Ser Pro Asn Gln Asp Pro Thr Glu Glu Glu Leu
           1185                1190                1195
   gtc cag gtc tgc aag gac gcc aac atc tac gac ttc atc atg tcg ctc    5868
   Val Gln Val Cys Lys Asp Ala Asn Ile Tyr Asp Phe Ile Met Ser Leu
       1200                1205                1210
   ccc gaa ggc ttt aac acc gtc gtc gga agc aag gga ggc atg ttg tcc    5916
   Pro Glu Gly Phe Asn Thr Val Val Gly Ser Lys Gly Gly Met Leu Ser
   1215                1220                1225                1230
   ggt ggc caa aag cag cgt gtc gcc atc gcc cgc gcc ctc ctg cgc aat    5964
   Gly Gly Gln Lys Gln Arg Val Ala Ile Ala Arg Ala Leu Leu Arg Asn
                   1235                1240                1245
   cct aag gtc cta ctt ctg gat gaa gct acc tcg gct ctc gac tcc gaa    6012
   Pro Lys Val Leu Leu Leu Asp Glu Ala Thr Ser Ala Leu Asp Ser Glu
               1250                1255                1260
   tcc gag aag gtc gtc caa gct gct ctg gat gct gct gct cgc ggt cgt    6060
   Ser Glu Lys Val Val Gln Ala Ala Leu Asp Ala Ala Ala Arg Gly Arg
           1265                1270                1275
   act acc atc gct gtg gct cac cgt ctc agt acc att caa aag gcc gat   6108
   Thr Thr Ile Ala Val Ala His Arg Leu Ser Thr Ile Gln Lys Ala Asp
      1280                1285                1290
   atc atc tat gtc ttc gac caa ggc aag att gtc gag agc ggt act cac   6156
   Ile Ile Tyr Val Phe Asp Gln Gly Lys Ile Val Glu Ser Gly Thr His
   1295                1300                1305                1310
   act gag ctc ttg cgc aac aag ggt cgc tac ttc gag ctg gtc aac ctc   6204
   Thr Glu Leu Leu Arg Asn Lys Gly Arg Tyr Phe Glu Leu Val Asn Leu
                   1315                1320                1325
   cag tcc ctt ggc agg gcc ggc taaatctttt gatttatttt catatccatg      6255
   Gln Ser Leu Gly Arg Ala Gly
               1330
   tatcataccc ccattttctt tcttgattct atctcacatt ccctgccatt tcccccatct 6315
   gttgcgtctc tttcaatttc tcccaccagg gtattcttgt gatagattat tttttcagca 6375
   gcaaagacgc ggaaagtcag ggtacattgt ctcccctcga taaagataaa aaattaaaaa 6435
   acatccacta gggatgtagg attagatgaa tgaatacaat gaaatctcaa ttcaacacca 6495
   cccggtttcg ctccaaactc catccgttca gttggatccc ttcaaaccat ttcaagtcct 6555
   ttcaattagt agacctcttg ccgtgtagcc cttcaatcct cctccgaaaa cccaaactcc 6615
   ctcaccacct cgacctgcct cacccccctg gcatcagcaa taagctccat caatccctgc 6675
   ccaacatccc tgatgtcatt gtactcatgc aagagctggg tatagcgtac gcatgtggta 6735
   ttgtaatcgt ccctgggata accaatggtt agagatgtaa gatgcaatgt ttcgatgttt 6795
   atgtagactg catgcaattg ggggagctga aggggtttta cttgagttgg gcggtcactg 6855
   aagctaagtc ggcttcgagc ctcgcatatt tagcttctag acttgcaatc gaagcgcgga 6915
   ggttgtccta cgtgggtgtt agtgcttaac atgatgaaca cgggtagtgg cagggtgggg 6975
   gatactacag acgtaccact cgttcctggt ctcggaagga cctggtttct ttggcttgat 7035
   ccatgtaggt gtgcaattag ctgcatatgc atatgtattg tggtacgtgg gatgcaagag 7095
   aggaagaagt tatccatgta cttacccaag taggtaggta cttcgtaccc cagaattata 7155
   tacttctgac caatcacagc cacgctgaat aatccactcc aagccaaggt ttctgcttgc 7215
   gcgtccttga ttttgctgtg atgttatttt ttaaaagttg tttaataagt aaatgataaa 7275
   atagctacct atatcacttg gctattcgag aatacagccc tggagatcaa aaagacggtt 7335
   ctttgactcc acgcgtcgtc ccgcctaatc ggatgccccg gattgggttt acctgttgtc 7395
   cgcatttgtt ctcttctccc ttgcaactcc ctacctcaac cacaccatgg catatcttcg 7455
   ccagtcctta tggactttgt ctgtattagt ggcctcggct ctcgcccatg gcggccacga 7515
   ggctgttcct gagggcgaat ctatctccca ggatccagga gaagaattcc tgcagcccgg 7575
   gggatccact agttctagag cggccgccac cgcggtggag ctccagcttt tgttcccttt 7635
   agtgagggtt aatttcgagc ttggcgtaat catggtcata gctgtttcct gtgtgaaatt 7695
   gttatccgct cacaattcca cacaacatac gagccggaag cataaagtgt aaagcctggg 7755
   gtgcctaatg agtgagctaa ctcacattaa ttgcgttgcg ctcactgccc gctttccagt 7815
   cgggaaacct gtcgtgccag ctgcattaat gaatcggcca acgcgcgggg agaggcggtt 7875
   tgcgtattgg gcgctcttcc gctttctcgc tcactgactc gctgcgctcg gtcgttcggc 7935
   tgcggcgagc ggtatcagct cactcaaagg cggtaatacg gttatccaca gaatcagggg 7995
   ataacgcagg aaagaacatg tgagcaaaag gccagcaaaa ggccaggaac cgtaaaaagg 8055
   ccgcgttgct ggcgtttttc cataggctcc gcccccctga cgagcatcac aaaaatcgac 8115
   gctcaagtca gaggtggcga aacccgacag gactataaag ataccaggcg tttccccctg 8175
   gaagctccct cgtgcgctct cctgttccga ccctgccgct taccggatac ctgtccgcct 8235
   ttctcccttc gggaagcgtg gcgctttctc atagctcacg ctgtaggtat ctcagttcgg 8295
   tgtaggtcgt tcgctccaag ctgggctgtg tgcacgaacc ccccgttcag cccgaccgct 8355
   gcgccttatc cggtaactat cgtcttgagt ccaacccggt aagacacgac ttatcgccac 8415
   tggcagcagc cactggtaac aggattagca gagcgaggta tgtaggcggt gctacagagt 8475
   tcttgaagtg gtggcctaac tacggctaca ctagaagaac agtatttggt atctgcgctc 8535
   tgctgaagcc agttaccttc ggaaaaagag ttggtagctc ttgatccggc aaacaaacca 8595
   ccgctggtag cggtggtttt tttgtttgca agcagcagat tacgcgcaga aaaaaaggat 8655
   ctcaagaaga tcctttgatc ttttctacgg ggtctgacgc tcagtggaac gaaaactcac 8715
   gttaagggat tttggtcatg agattatcaa aaaggatctt cacctagtgt gggcggacaa 8775
   aa 8777
<210> 36
   <211> 1333
   <212> PRT
   <213> Penicillium chrysogenum
   <223> Protein sequence of the Penicillium chrysogenum ABC transporter aa10 (derived from the DNA sequence as shown in SEQ ID NO. 34)
<400> 36
   Met Gln Gln Pro Gln Glu Gly Val Thr Ala Val Glu Lys Val Ala Asp
   1 5 10 15
   Gly Pro Gln Leu Asn Glu Lys Pro Val Asp Lys Arg Asp Ser Asp Ser
   20 25 30
   Ala Ser Thr Ser Thr Ala Lys Glu His Glu Ser Lys Thr Pro Val Ala
   35 40 45
   Ser Asp Glu Asp Ala Leu Tyr Ala His Leu Pro Glu His Glu Lys Asp
        50                  55                  60
   Ile Leu Lys Gln Gln Leu Asp Ala Pro Pro Val Asn Ile Ser Tyr Phe
   65                  70                  75                  80
   Gly Leu Tyr Arg Tyr Ala Ser Arg Ile Asp Ile Leu Ile Ile Val Ile
                   85                  90                  95
   Ser Thr Leu Cys Ala Ile Ala Ala Gly Ala Ala Leu Pro Leu Phe Thr
               100                 105                 110
   Ile Leu Phe Gly Ser Leu Ala Thr Ala Phe Gln Lys Ile Met Leu Arg
           115                 120                 125
   Thr Ile Pro Tyr Asp Glu Phe Tyr Asp Gln Leu Thr Thr Asn Val Leu
       130                 135                 140
   Tyr Phe Ile Tyr Leu Gly Ile Gly Glu Phe Val Thr Val Tyr Val Ser
   145                 150                 155                 160
   Thr Val Gly Phe Ile Tyr Thr Gly Glu His Val Thr Gln Lys Ile Arg
                   165                 170                 175
   Glu His Tyr Leu Glu Ala Ile Leu Arg Gln Asn Ile Ala Tyr Phe Asp
               180                 185                 190
   Lys Leu Gly Ala Gly Glu Val Thr Thr Arg Ile Thr Ala Asp Thr Asn
           195                 200                 205
   Leu Ile Gln Asp Gly Val Ser Glu Lys Val Gly Leu Thr Leu Thr Ala
       210                 215                 220
   Val Ala Thr Phe Val Thr Ala Phe Ile Val Ala Tyr Ile Lys Tyr Ala
   225                 230                 235                 240
   Pro Leu Ala Gly Ile Cys Thr Ser Thr Met Val Ala Leu Val Leu Ile
                   245                 250                 255
   Met Gly Gly Gly Ser Gln Leu Ile Ile Lys Phe Gly Lys Leu Ser Leu
               260                 265                 270
   Glu Ser Ala Gly Ala Gly Gly Thr Val Ala Glu Glu Val Ile Ser Ser
           275                 280                 285
   Ile Arg Asn Ala Thr Ala Phe Gly Thr Gln Asp Lys Leu Ala Lys Gln
       290                 295                 300
   Tyr Glu Ser His Leu Leu Arg Ala Glu Arg Trp Gly Met Arg Leu Gln
   305                 310                 315                 320
   Met Ser Leu Ala Val Met Val Gly Ile Met Phe Gly Leu Met Phe Met
                   325                 330                 335
   Asn Tyr Gly Leu Gly Phe Trp Met Gly Ser Gln Phe Leu Val Asp Gly
               340                 345                 350
   Lys Val Asp Val Gly Gln Val Leu Thr Ile Leu Met Ala Ile Leu Ile
           355                 360                 365
   Gly Ser Phe Ser Leu Gly Asn Val Ser Pro Asn Ala Ser Ala Phe Thr
   370 375 380
   Asn Ala Val Ala Ala Ala Thr Lys Ile Phe Ala Thr Ile Asp Arg Asp
   385 390 395 400
   Ser Pro Leu Asp Pro Thr Ser Asp Glu Gly Ile Ile Leu Asp His Val
   405 410 415
   Glu Gly His Ile Glu Phe Arg Asn Val Lys His Ile Tyr Pro Ser Arg
   420 425 430
   Pro Glu Val Thr Val Met Asn Asp Val Ser Leu Gly Ile Pro Ala Gly
   435 440 445
   Asn Thr Thr Ala Leu Val Gly Pro Ser Gly Ser Gly Lys Ser Thr Val
   450 455 460
   Val Gly Leu Val Glu Arg Phe Tyr Leu Pro Val Gly Gly Gln Val Phe
   465 470 475 480
   Leu Asp Gly His Asp Ile Gln Thr Leu Asn Leu Arg Trp Leu Arg Gln
   485 490 495
   Gln Ile Ser Leu Val Ser Gln Glu Pro Val Leu Phe Gly Thr Thr Ile
   500 505 510
   Tyr Gln Asn Ile Arg His Gly Leu Ile Gly Thr Arg Phe Glu His Glu
   515 520 525
   Ser Glu Glu Lys Ile Lys Glu Leu Ile Glu Asn Ala Ala Lys Met Ala
   530 535 540
   Asn Ala His Glu Phe Ile Thr Ala Leu Pro Glu Gly Tyr Glu Thr Asn
   545 550 555 560
   Val Gly Gln Arg Gly Phe Leu Leu Ser Gly Gly Gln Lys Gln Arg Ile
   565 570 575
   Ala Ile Ala Arg Ala Met Val Ser Asp Pro Lys Ile Leu Leu Leu Asp
   580 585 590
   Glu Ala Thr Ser Ala Leu Asp Thr Lys Ser Glu Gly Val Val Gln Ala
   595 600 605
   Ala Leu Asp Arg Ala Ala Glu Gly Arg Thr Thr Ile Val Ile Ala His
   610 615 620
   Arg Leu Ser Thr Ile Lys Ser Ala His Asn Ile Val Val Phe Val Gln
   625 630 635 640
   Gly Ser Ile Val Glu Gln Gly Thr His Ser Gln Leu Thr Glu His Asp
   645 650 655
   Gly Pro Tyr Phe Lys Leu Val Glu Ala Gln Arg Ile Asn Glu Glu Lys
   660 665 670
   Asp Ala Asp Ala Leu Asp Ala Asp Glu Asp Glu Asp Gly Leu Glu Glu
   675 680 685
   Met Thr Lys Ser His Ile Ala Arg Val Lys Ser Ile Ala Ser Gly Ser
   690 695 700
   Thr Cys Val Lys Asp Glu Ala Glu Thr Phe Gln Asp Ala Met His Arg
   705 710 715 720
   Gln Glu Ser Arg Lys Ser Val Ser Ser Val Ile Leu Ser Gln Lys Thr
   725 730 735
   Ala Glu Gly Gly Arg Lys His Ser Leu Leu Thr Leu Ile Lys Phe Ile
   740 745 750
   Gly Ser Phe Asn Lys Glu Glu Arg Trp Phe Met Ala Ile Gly Leu Cys
   755 760 765
   Phe Ser Ile Leu Ala Gly Cys Gly Gln Pro Thr Gln Ala Phe Leu Tyr
   770 775 780
   Ala Lys Ala Ile Ser Ala Leu Ser Leu Pro Lys Thr Gln Tyr Asp Lys
   785 790 795 800
   Leu Arg Ser Asp Ala Asn Phe Trp Ser Leu Met Phe Phe Ile Val Gly
   805 810 815
   Ile Val Gln Ile Ile Thr Phe Ser Val His Gly Ile Ala Phe Ala Phe
   820 825 830
   Ser Ser Glu Arg Leu Ile Arg Lys Ala Arg Gly Asn Ala Phe Arg Val
   835 840 845
   Met Leu Arg Gln Asp Ile Asn Phe Phe Asp Arg Glu Glu Asn Ser Thr
   850 855 860
   Gly Ala Leu Thr Ser Phe Leu Ser Thr Glu Thr Lys His Leu Ala Gly
   865 870 875 880
   Ile Ser Gly Gln Thr Leu Gly Thr Ile Leu Met Thr Ser Thr Thr Leu
   885 890 895
   Ile Ala Ser Ile Val Ile Ala Leu Ala Phe Gly Trp Lys Leu Ala Leu
   900 905 910
   Val Cys Met Ser Val Ile Pro Ile Leu Leu Gly Cys Gly Phe Tyr Arg
   915 920 925
   Phe Tyr Met Leu Ala Ala Phe Gln Ala Arg Ser Lys Ala Ala Tyr Glu
   930 935 940
   Gly Ser Ala Ser Tyr Ala Cys Glu Ala Thr Ser Ala Ile Arg Thr Val
   945 950 955 960
   Ala Ser Leu Thr Arg Glu Thr Asp Val Trp Ser Phe Tyr His Gly Gln
   965 970 975
   Leu Glu Arg Gln Gly Arg Thr Ser Leu Ile Ser Val Phe Lys Ser Ser
   980 985 990
   Leu Leu Tyr Ala Ala Ser Gln Ala Leu Val Phe Phe Cys Val Ala Leu
   995 1000 1005
   Gly Phe Trp Tyr Gly Gly Thr Leu Leu Gly His His Glu Tyr Asp Val
   1010 1015 1020
   Phe Arg Phe Phe Val Cys Phe Ser Glu Ile Leu Phe Gly Ala Gln Ser
   025 1030 1035 1040
   Ala Gly Thr Val Phe Ser Phe Ser Pro Asp Met Gly Lys Ala Lys Asn
   1045 1050 1055
   Ala Ala Ala Glu Phe Leu Arg Leu Phe Glu Arg Arg Pro Thr Ile Asp
   1060 1065 1070
   Thr Trp Ser Glu Glu Gly Glu Asn Leu Asp His Cys Glu Gly Thr Ile
   1075 1080 1085
   Glu Phe Lys Asp Val His Phe Arg Tyr Pro Thr Arg Pro Glu Gln Pro
   1090 1095 1100
   Val Leu Arg Gly Leu Asn Leu Thr Val Lys Pro Gly Gln Tyr Ile Ala
   105 1110 1115 1120
   Leu Val Gly Pro Ser Gly Cys Gly Lys Ser Thr Thr Ile Ala Leu Leu
   1125 1130 1135
   Glu Arg Phe Tyr Asp Ala Leu Ser Gly Gly Val Tyr Ile Asp Asp Lys
   1140 1145 1150
   Asn Ile Ala Asp Leu Asn Val Asn Ser Tyr Arg Ser His Leu Ala Leu
   1155 1160 1165
   Val Ser Gln Glu Pro Thr Leu Tyr Gln Gly Thr Ile Lys Glu Asn Ile
   1170 1175 1180
   Leu Leu Gly Ser Pro Asn Gln Asp Pro Thr Glu Glu Glu Leu Val Gln
   185 1190 1195 1200
   Val Cys Lys Asp Ala Asn Ile Tyr Asp Phe Ile Met Ser Leu Pro Glu
   1205 1210 1215
   Gly Phe Asn Thr Val Val Gly Ser Lys Gly Gly Met Leu Ser Gly Gly
   1220 1225 1230
   Gln Lys Gln Arg Val Ala Ile Ala Arg Ala Leu Leu Arg Asn Pro Lys
   1235 1240 1245
   Val Leu Leu Leu Asp Glu Ala Thr Ser Ala Leu Asp Ser Glu Ser Glu
   1250 1255 1260
   Lys Val Val Gln Ala Ala Leu Asp Ala Ala Ala Arg Gly Arg Thr Thr
   265 1270 1275 1280
   Ile Ala Val Ala His Arg Leu Ser Thr Ile Gln Lys Ala Asp Ile Ile
   1285 1290 1295
   Tyr Val Phe Asp Gln Gly Lys Ile Val Glu Ser Gly Thr His Thr Glu
   1300 1305 1310
   Leu Leu Arg Asn Lys Gly Arg Tyr Phe Glu Leu Val Asn Leu Gln Ser
   1315 1320 1325
   Leu Gly Arg Ala Gly
   1330
<210> 37
   <211> 396
   <212> DNA
   <213> Penicillium chrysogenum
<220>
   <223> Partial cDNA sequence of Penicillium chrysogenum ABC transporter aa20
<400> 37
   tcgggggcgg ggaagacgac tctgctcaat actttggccc agcgccagaa gatgggtgtc 60
   gtgactggtg atatgctcgt cgatggccac aagctgggtc ctgacttcca gcgtggcaca 120
   ggtttctgtg agcaaatgga tctgcacgac aataccgcta ctatccgcga agctttggaa 180
   ttctcagcca tcctccgcca gccgcgcgat gtctcgcgcc aggagaaact cgactacgtg 240
   gaccgcatca tcgaccttct cgaactcgag gatatccaag acgccatcat cggctgtctc 300
   accgtcgagc agaagaagcg cgtcaccatt ggcgtcgaac ttgcagccaa gcccagtctg 360
   cttctgttcc tggacgaacc caccagcgcc ttcgaa                           396
<210> 38
   <211> 396
   <212> DNA
   <213> Penicillium chrysogenum
<220>
   <221> CDS
   <222> (1).. (396)
<220>
   <223> Translation of the cDNA sequence of Penicillium chrysogenum ABC transporter aa20 from SEQ ID NO. 37
<400> 38
   tcg ggg gcg ggg aag acg act ctg ctc aat act ttg gcc cag cgc cag    48
   Ser Gly Ala Gly Lys Thr Thr Leu Leu Asn Thr Leu Ala Gln Arg Gln
    1               5                  10                  15
   aag atg ggt gtc gtg act ggt gat atg ctc gtc gat ggc cac aag ctg    96
   Lys Met Gly Val Val Thr Gly Asp Met Leu Val Asp Gly His Lys Leu
               20                  25                  30
   ggt cct gac ttc cag cgt ggc aca ggt ttc tgt gag caa atg gat ctg    144
   Gly Pro Asp Phe Gln Arg Gly Thr Gly Phe Cys Glu Gln Met Asp Leu
           35                  40                  45
   cac gac aat acc gct act atc cgc gaa gct ttg gaa ttc tca gcc atc    192
   His Asp Asn Thr Ala Thr Ile Arg Glu Ala Leu Glu Phe Ser Ala Ile
       50                  55                  60
   ctc cgc cag ccg cgc gat gtc tcg cgc cag gag aaa ctc gac tac gtg    240
   Leu Arg Gln Pro Arg Asp Val Ser Arg Gln Glu Lys Leu Asp Tyr Val
   65                  70                  75                  80
   gac cgc atc atc gac ctt ctc gaa ctc gag gat atc caa gac gcc atc    288
   Asp Arg Ile Ile Asp Leu Leu Glu Leu Glu Asp Ile Gln Asp Ala Ile
                   85                  90                  95
   atc ggc tgt ctc acc gtc gag cag aag aag cgc gtc acc att ggc gtc    336
   Ile Gly Cys Leu Thr Val Glu Gln Lys Lys Arg Val Thr Ile Gly Val
               100                 105                 110
   gaa ctt gca gcc aag ccc agt ctg ctt ctg ttc ctg gac gaa ccc acc    384
   Glu Leu Ala Ala Lys Pro Ser Leu Leu Leu Phe Leu Asp Glu Pro Thr
           115                 120                 125
   agc gcc ttc gaa                                                     396
   Ser Ala Phe Glu
       130
<210> 39
   <211> 132
   <212> PRT
   <213> Penicillium chrysogenum
   <223> Protein sequence of the Penicillium chrysogenum ABC transporter aa20 (derived from the DNA sequence as shown in SEQ ID NO. 37)
<400> 39
   Ser Gly Ala Gly Lys Thr Thr Leu Leu Asn Thr Leu Ala Gln Arg Gln
    1               5                  10                  15
   Lys Met Gly Val Val Thr Gly Asp Met Leu Val Asp Gly His Lys Leu
               20                  25                  30
   Gly Pro Asp Phe Gln Arg Gly Thr Gly Phe Cys Glu Gln Met Asp Leu
           35                  40                  45
   His Asp Asn Thr Ala Thr Ile Arg Glu Ala Leu Glu Phe Ser Ala Ile
       50                  55                  60
   Leu Arg Gln Pro Arg Asp Val Ser Arg Gln Glu Lys Leu Asp Tyr Val
   65                  70                  75                  80
   Asp Arg Ile Ile Asp Leu Leu Glu Leu Glu Asp Ile Gln Asp Ala Ile
                   85                  90                  95
   Ile Gly Cys Leu Thr Val Glu Gln Lys Lys Arg Val Thr Ile Gly Val
               100                 105                 110
   Glu Leu Ala Ala Lys Pro Ser Leu Leu Leu Phe Leu Asp Glu Pro Thr
           115                 120                 125
   Ser Ala Phe Glu
       130
<210> 40
   <211> 396
   <212> DNA
   <213> Penicillium chrysogenum
<220>
   <223> Partial cDNA sequence of Penicillium chrysogenum ABC transporter aa32
<400> 40
   tcgggggggg ggaagagtac gatgattagc atgctcgaac gcttttacga tccagtagcc 60
   ggtcacatta aaatcgacgc acttacattg gatagcttga atccgtggct gtatcgaggt 120
   caagtggcac ttgtgcagca ggaacccacc ctctatcccg gcaccattcg cgaaaatgtc 180
   gccatgggga caccgactga tgacggatca acggttcctg acagtgatat tgaagctgca 240
   tgtcgggctg ccaacgcgtg ggaatttatc tcttctctgc ccgatggcct tatgactctc 300
   tgtggggcga atgggacaca gctgtctggt gggcaacgtc agcgcatcgc cattgccagg 360
   gcgctactca gaaatccacg tttactccta cttgaa                           396
<210> 41
   <211> 396
   <212> DNA
   <213> Penicillium chrysogenum
<220>
   <221> CDS
   <222> (1) .. (396)
<220>
   <223> Translation of the cDNA sequence of Penicillium chrysogenum ABC transporter aa32 from SEQ ID NO. 40
<400> 41
   tcg ggg ggg ggg aag agt acg atg att agc atg ctc gaa cgc ttt tac      48
   Ser Gly Gly Gly Lys Ser Thr Met Ile Ser Met Leu Glu Arg Phe Tyr
    1               5                  10                  15
   gat cca gta gcc ggt cac att aaa atc gac gca ctt aca ttg gat agc      96
   Asp Pro Val Ala Gly His Ile Lys Ile Asp Ala Leu Thr Leu Asp Ser
               20                  25                  30
   ttg aat ccg tgg ctg tat cga ggt caa gtg gca ctt gtg cag cag gaa     144
   Leu Asn Pro Trp Leu Tyr Arg Gly Gln Val Ala Leu Val Gln Gln Glu
           35                  40                  45
   ccc acc ctc tat ccc ggc acc att cgc gaa aat gtc gcc atg ggg aca     192
   Pro Thr Leu Tyr Pro Gly Thr Ile Arg Glu Asn Val Ala Met Gly Thr
       50                  55                  60
   ccg act gat gac gga tca acg gtt cct gac agt gat att gaa gct gca     240
   Pro Thr Asp Asp Gly Ser Thr Val Pro Asp Ser Asp Ile Glu Ala Ala
   65                  70                  75                  80
   tgt cgg gct gcc aac gcg tgg gaa ttt atc tct tct ctg ccc gat ggc     288
   Cys Arg Ala Ala Asn Ala Trp Glu Phe Ile Ser Ser Leu Pro Asp Gly
                   85                  90                  95
   ctt atg act ctc tgt ggg gcg aat ggg aca cag ctg tct ggt ggg caa     336
   Leu Met Thr Leu Cys Gly Ala Asn Gly Thr Gln Leu Ser Gly Gly Gln
               100                 105                 110
   cgt cag cgc atc gcc att gcc agg gcg cta ctc aga aat cca cgt tta     384
   Arg Gln Arg Ile Ala Ile Ala Arg Ala Leu Leu Arg Asn Pro Arg Leu
           115                 120                 125
   ctc cta ctt gaa                                                     396
   Leu Leu Leu Glu
       130
<210> 42
   <211> 132
   <212> PRT
   <213> Penicillium chrysogenum
   <223> Protein sequence of the Penicillium chrysogenum ABC transporter aa32 (derived from the DNA sequence as shown in SEQ ID NO. 40)
<400> 42
   Ser Gly Gly Gly Lys Ser Thr Met Ile Ser Met Leu Glu Arg Phe Tyr
    1               5                  10                  15
   Asp Pro Val Ala Gly His Ile Lys Ile Asp Ala Leu Thr Leu Asp Ser
               20                  25                  30
   Leu Asn Pro Trp Leu Tyr Arg Gly Gln Val Ala Leu Val Gln Gln Glu
           35                  40                  45
   Pro Thr Leu Tyr Pro Gly Thr Ile Arg Glu Asn Val Ala Met Gly Thr
       50                  55                  60
   Pro Thr Asp Asp Gly Ser Thr Val Pro Asp Ser Asp Ile Glu Ala Ala
   65                  70                  75                  80
   Cys Arg Ala Ala Asn Ala Trp Glu Phe Ile Ser Ser Leu Pro Asp Gly
                   85                  90                  95
   Leu Met Thr Leu Cys Gly Ala Asn Gly Thr Gln Leu Ser Gly Gly Gln
               100                 105                 110
   Arg Gln Arg Ile Ala Ile Ala Arg Ala Leu Leu Arg Asn Pro Arg Leu
           115                 120                 125
   Leu Leu Leu Glu
       130
<210> 43
   <211> 184
   <212> DNA
   <213> Penicillium chrysogenum
<220>
   <223> Partial cDNA sequence of Penicillium chrysogenum ABC transporter dd034
<400> 43
   gatcgcggaa ctggaccgaa tgattgcgga tgctgctagc aagccccccg gaactgtcga 60
   tgatggattc gaattctcca tgcctctttg ggagcagacc aaaatcgtca ctcatcggat 120
   gaatgtcgcg cttttccgaa acaccaacta tgtcaacaac aagttctccc ttcacattat 180
   ctaa                                                              184
<210> 44
   <211> 184
   <212> DNA
   <213> Penicillium chrysogenum
<220>
   <221> CDS
   <222> (2)..(181)
   <223> Translation of the cDNA sequence of Penicillium chrysogenum ABC transporter dd034 from SEQ ID NO. 43
<400> 44
   g atc gcg gaa ctg gac cga atg att gcg gat gct gct agc aag ccc ccc 49
     Ile Ala Glu Leu Asp Arg Met Ile Ala Asp Ala Ala Ser Lys Pro Pro
       1               5                   10                  15
   gga act gtc gat gat gga ttc gaa ttc tcc atg cct ctt tgg gag cag   97
   Gly Thr Val Asp Asp Gly Phe Glu Phe Ser Met Pro Leu Trp Glu Gln
                20                  25                  30
   acc aaa atc gtc act cat cgg atg aat gtc gcg ctt ttc cga aac acc   145
   Thr Lys Ile Val Thr His Arg Met Asn Val Ala Leu Phe Arg Asn Thr
            35                  40                  45
   aac tat gtc aac aac aag ttc tcc ctt cac att atc taa               184
   Asn Tyr Val Asn Asn Lys Phe Ser Leu His Ile Ile
        50                  55                  60
<210> 45
   <211> 60
   <212> PRT
   <213> Penicillium chrysogenum
   <223> Protein sequence of the Penicillium chrysogenum ABC transporter dd034 (derived from the DNA sequence as shown in SEQ ID NO. 43)
<400> 45
   Ile Ala Glu Leu Asp Arg Met Ile Ala Asp Ala Ala Ser Lys Pro Pro
    1               5                   10                  15
   Gly Thr Val Asp Asp Gly Phe Glu Phe Ser Met Pro Leu Trp Glu Gln
                20                  25                  30
   Thr Lys Ile Val Thr His Arg Met Asn Val Ala Leu Phe Arg Asn Thr
            35                  40                  45
   Asn Tyr Val Asn Asn Lys Phe Ser Leu His Ile Ile
        50                  55                  60
<210> 46
   <211> 459
   <212> DNA
   <213> Penicillium chrysogenum
<220>
   <223> Partial cDNA sequence of Penicillium chrysogenum ABC transporter dd062
<400> 46
   ttagcgcgag gttgctacct ggagagcgta tgtgaatatc gccatgacac ttggacgcag 60
   tcttggaggc ccgctaggtg gttggttgag cgataccatc ggatggagat ggctgttcat 120
   cctacaggcg ccgttcctcg ctctggctgc agttctcgtg attgcgaagt tgaacgtcga 180
   gagaccacac tccaaggaaa acaaactcgc caaagtcgat ttcctcggaa cagcactgct 240
   aggctcatcc attgtcgcaa tgaccactct cctcgatcaa ggtgggaagt ccttcccatg 300
   gagctcatgg tggaccgtcc tcctagcaag caatggcgta tcactcctgg tcgctttcgt 360
   gctcgtggaa gcatacgttg cccgcgaacc catcttcaac ctccgcattc tccggcgcac 420
   aaacgtgtca atggcataca tcatcgggca ctccagatc                        459
<210> 47
   <211> 459
   <212> DNA
   <213> Penicillium chrysogenum
<220>
   <221> CDS
   <222> (5)..(457)
<220>
   <223> Translation of the cDNA sequence of Penicillium chrysogenum ABC transporter dd062 from SEQ ID No. 46
<400> 47
   ttag cgc gag gtt gct acc tgg aga gcg tat gtg aat atc gcc atg aca   49
        Arg Glu Val Ala Thr Trp Arg Ala Tyr Val Asn Ile Ala Met Thr
        1               5                   10                  15
   ctt gga cgc agt ctt gga ggc ccg cta ggt ggt tgg ttg agc gat acc   97
   Leu Gly Arg Ser Leu Gly Gly Pro Leu Gly Gly Trp Leu Ser Asp Thr
                   20                  25                  30
   atc gga tgg aga tgg ctg ttc atc cta cag gcg ccg ttc ctc gct ctg   145
   Ile Gly Trp Arg Trp Leu Phe Ile Leu Gln Ala Pro Phe Leu Ala Leu
               35                  40                  45
   gct gca gtt ctc gtg att gcg aag ttg aac gtc gag aga cca cac tcc   193
   Ala Ala Val Leu Val Ile Ala Lys Leu Asn Val Glu Arg Pro His Ser
           50                  55                  60
   aag gaa aac aaa ctc gcc aaa gtc gat ttc ctc gga aca gca ctg cta   241
   Lys Glu Asn Lys Leu Ala Lys Val Asp Phe Leu Gly Thr Ala Leu Leu
       65                  70                  75
   ggc tca tcc att gtc gca atg acc act ctc ctc gat caa ggt ggg aag   289
   Gly Ser Ser Ile Val Ala Met Thr Thr Leu Leu Asp Gln Gly Gly Lys
   80                  85                  90                  95
   tcc ttc cca tgg agc tca tgg tgg acc gtc ctc cta gca agc aat ggc   337
   Ser Phe Pro Trp Ser Ser Trp Trp Thr Val Leu Leu Ala Ser Asn Gly
                   100                 105                 110
   gta tca ctc ctg gtc gct ttc gtg ctc gtg gaa gca tac gtt gcc cgc   385
   Val Ser Leu Leu Val Ala Phe Val Leu Val Glu Ala Tyr Val Ala Arg
               115                 120                 125
   gaa ccc atc ttc aac ctc cgc att ctc cgg cgc aca aac gtg tca atg   433
   Glu Pro Ile Phe Asn Leu Arg Ile Leu Arg Arg Thr Asn Val Ser Met
           130                 135                 140
   gca tac atc atc ggg cac tcc aga tc                                459
   Ala Tyr Ile Ile Gly His Ser Arg
       145                 150
<210> 48
   <211> 151
   <212> PRT
   <213> Penicillium chrysogenum
   <223> Protein sequence of the Penicillium chrysogenum ABC transporter dd062 (derived from the DNA sequence as shown in SEQ ID No. 46)
<400> 48
   Arg Glu Val Ala Thr Trp Arg Ala Tyr Val Asn Ile Ala Met Thr Leu
   1               5                   10                  15
   Gly Arg Ser Leu Gly Gly Pro Leu Gly Gly Trp Leu Ser Asp Thr Ile
               20                  25                  30
   Gly Trp Arg Trp Leu Phe Ile Leu Gln Ala Pro Phe Leu Ala Leu Ala
           35                  40                  45
   Ala Val Leu Val Ile Ala Lys Leu Asn Val Glu Arg Pro His Ser Lys
       50                  55                  60
   Glu Asn Lys Leu Ala Lys Val Asp Phe Leu Gly Thr Ala Leu Leu Gly
   65                  70                  75                  80
   Ser Ser Ile Val Ala Met Thr Thr Leu Leu Asp Gln Gly Gly Lys Ser
                   85                  90                  95
   Phe Pro Trp Ser Ser Trp Trp Thr Val Leu Leu Ala Ser Asn Gly Val
               100                 105                 110
   Ser Leu Leu Val Ala Phe Val Leu Val Glu Ala Tyr Val Ala Arg Glu
           115                 120                 125
   Pro Ile Phe Asn Leu Arg Ile Leu Arg Arg Thr Asn Val Ser Met Ala
       130                 135                 140
   Tyr Ile Ile Gly His Ser Arg
   145                 150
<210> 49
   <211> 24
   <212> DNA
   <213> Artificial Sequence
   <220>
   <223> aa7 forward primer
<400> 49
   attggctgag acagcaaatc tcgc 24
<210> 50
   <211> 20
   <212> DNA
   <213> Artificial Sequence
<220>
   <223> aa7 reverse primer
<400> 50
   aggatcggaa atgagggcgc 20
<210> 51
   <211> 25
   <212> DNA
   <213> Artificial Sequence
<220>
   <223> aa10 forward primer
<400> 51
   agcggttcta tctcccagtc ggagg 25
<210> 52
   <211> 23
   <212> DNA
   <213> Artificial Sequence
<220>
   <223> aa10 reverse primer
<400> 52
   cacgagcgat ggcaatacgt tgc 23
<210> 53
   <211> 21
   <212> DNA
   <213> Artificial Sequence
<220>
   <223> dd062 forward primer
<400> 53
   tggagtgccc gatgatgtat g 21
<210> 54
   <211> 22
   <212> DNA
   <213> Artificial Sequence
<220>
   <223> dd062 reverse primer
<400> 54
   cgcgaggttg ctacctggag ag 22

## Claims

1. Method for enhancing the secretion of a β-lactam compound from a micro-organism, capable of producing β-lactam compounds comprising the step of enhancing an ABC transporter activity of said micro-organism, which comprises the step of transforming said micro-organism with at least one expression cassette comprising a polynucleotide selected from the group consisting of SEQ ID NO 7. 8, 9, 11, 12, 14, 15, 18, 19, 21, 22, 24, 25, 26, 27, 29, 30, 34, 35, 37, 38, 46 and 47 or a polynucleotide which shows a percentage of similarity of least 60% with these polynucleotide sequences, encoding a homologous or heterologous ABC transporter.

2. Method according to claim 1, wherein said polynucleotide encodes an ABC transporter which comprises an amino acid sequence selected from the group consisting of SEQ ID NO. 10, 13, 16, 20, 23, 28, 31, 36, 39, and 48 or a polypeptide which shows a percentage of similarity of least 60% with these polypeptide sequences.

3. Method according to claim 1 or 2, wherein said micro-organism is a fungus.

4. Method according to claim 3, wherein said fungus is selected from the group consisting of *Penicillium chrysogenum, Acremonium chrysogenum, Aspergillus nidulans, Saccharomyces cerevisiae, Hansenula polymorpha* and *Pichia pastoris.*

5. Method according to claim 1 or 2, wherein said micro-organism is a bacterium, preferably an actinomycete, even more preferably an actinomycete selected from the group consisting of *Streptomyces clavuligerus, Nocardia lactamdurans, Flavobacterium sp.*

6. An isolated polynucleotide encoding an amino acid sequence selected from the group consisting of SEQ ID NO. 10, 13, 16, 20, 23, 28, 31, 36, 39, and 48, or a sequence which shows a percentage of similarity of at least 95% with these amino acid sequences.

7. An isolated polynucleotide comprising a nucleotide sequence selected from the group consisting of SEQ ID NO. 7, 8, 9, 11, 12, 14, 15, 18, 19, 21, 22, 24, 25, 26, 27, 29, 30, 32, 33, 34, 35, 37, 38, 46 and 47, or a polynucleotide which shows a similarity of at least 95% with these polynucleotide sequences

8. A vector comprising a polynucleotide according to claim 7.

9. A vector according to claim 8 wherein the polynucleotide sequence encodes an ABC transporter and wherein said polynucleotide is operably linked to regulatory sequences suitable for expression of the ABC transporter in a suitable host cell.

10. A transformed host cell comprising the vector of claim 8 or 9.

11. Use of an isolated polynucleotide according to claim 6 or 7 and a vector according to claim 8 or 9 in a method for enhancing the production of β-lactam compounds in a micro-organism capable of producing β-lactam compounds.

12. Method for enhancing the production of β-lactam compounds in a micro-organism capable of producing β-lactam compounds comprising the step of enhancing ABC transporter activity of said micro-organism, which comprises the step of transforming said micro-organism with at least one expression cassette comprising a polynucleotide selected from the group consisting of SEQ ID NO 7, 8, 9, 11, 12, 14, 15, 18, 19, 21, 22, 24, 25, 26, 27, 29, 30, 34, 35, 37, 38, 46 and 47 or a polynucleotide which shows a percentage of similarity of least 60% with these polynucleotide sequences, encoding a homologous or heterologous ABC transporter, and subsequently culturing said micro-organism under conditions allowing the production of said β-lactam compounds.

13. Isolated ABC transporter encoded by an ABC transporter gene comprising a nucleotide sequence according to a SEQ ID NO selected from the group consisting of SEQ ID NO. 7, 8, 9, 11, 12, 14, 15, 18, 19, 21, 22, 24, 25, 26, 29, 30, 32, 33, 34, 37, 38, 46 and 47

## Patentansprüche

1. Verfahren zum Verstärken der Sekretion einer β-Lactam-Verbindung aus einem Mikroorganismus, der in der Lage ist, β-Lactam-Verbindungen zu produzieren, umfassend den Schritt des Verstärkens einer ABC-Transporteraktivität des Mikroorganismus, welches den Schritt des Transformierens des Mikroorganismus mit mindestens einer Expressionskassette umfasst, die ein Polynukleotid umfasst, das aus der Gruppe ausgewählt ist, die aus SEQ ID Nr. 7, 8, 9, 11, 12, 14, 15, 18, 19, 21, 22, 24, 25, 26, 27, 29, 30, 34, 35, 37, 38, 46 und 47 oder einem Polynukleotid besteht, das eine prozentuale Ähnlichkeit von mindestens 60% mit diesen Polynukleotidsequenzen zeigt, das einen homologen oder heterologen ABC-Transporter kodiert.

2. Verfahren nach Anspruch 1, wobei das Polynukleotid einen ABC-Transporter kodiert, welcher eine Aminosäuresequenz aufweist, die aus der Gruppe ausgewählt ist, welche aus SEQ ID Nr. 10, 13, 16, 20, 23, 28, 31, 36, 39 und 48 oder einem Polypeptid besteht, das eine prozentuale Ähnlichkeit von mindestens 60% mit diesen Polynukleotidsequenzen zeigt.

3. Verfahren nach Anspruch 1 oder 2, wobei der Mikroorganismus ein Pilz ist.

4. Verfahren nach Anspruch 3, wobei der Pilz aus der Gruppe ausgewählt ist, die aus *Penicillium chrysogenum, Acremonium chrysogenum, Aspergillus nidulans, Saccharomyces cerevisiae, Hansenula polymorpha* und *Pichia pastoris* besteht.

5. Verfahren nach Anspruch 1 oder 2, wobei der Mikroorganismus ein Bakterium ist, vorzugsweise ein Actinomycet, noch bevorzugter ein Actinomycet, der aus der Gruppe ausgewählt ist, welche aus *Streptomyces cla vuligerus, Nocardia lactamdurans, Flavobacterium sp.* besteht.

6. Isoliertes Polynukleotid, das eine Aminosäuresequenz kodiert, die aus der Gruppe ausgewählt ist, welche aus SEQ ID Nr. 10, 13, 16, 20, 23, 28, 31, 36, 39 und 48 besteht, oder eine Sequenz, die eine prozentuale Ähnlichkeit von mindestens 95% mit diesen Aminosäuresequenzen zeigt.

7. Isoliertes Polynukleotid, das eine Nukleotidsequenz umfasst, die aus der Gruppe ausgewählt ist, welche aus SEQ ID Nr. 7, 8, 9, 11, 12, 14, 15, 18, 19, 21, 22, 24, 25, 26, 27, 29, 30, 32, 33, 34, 35, 37, 38, 46 und 47 besteht, oder ein Polynukleotid, das eine Ähnlichkeit von mindestens 95% mit diesen Polynukleotidsequenzen zeigt.

8. Vektor, welcher ein Polynukleotid nach Anspruch 7 aufweist.

9. Vektor nach Anspruch 8, wobei die Polynukleotidsequenz einen ABC-Transporter kodiert und wobei das Polynukleotid funktionell mit regulatorischen Sequenzen verknüpft ist, die für die Expression des ABC-Transporters in einer geeigneten Wirtszelle geeignet sind.

10. Transformierte Wirtszelle, welche den Vektor von Anspruch 8 oder 9 aufweist.

11. Verwendung eines isolierten Polynukleotids nach Anspruch 6 oder 7 und eines Vektors nach Anspruch 8 oder 9 in einem Verfahren zum Verstärken der Produktion von β-Lactamverbindungen in einem Mikroorganismus, der in der Lage ist, β-Lactamverbindungen zu produzieren.

12. Verfahren zum Verstärken der Produktion von β-Lactam-Verbindungen in einem Mikroorganismus, der in der Lage ist, β-Lactam-Verbindungen zu produzieren, umfassend den Schritt des Verstärkens einer ABC-Transporteraktivität des Mikroorganismus, welches den Schritt des Transformierens des Mikroorganismus mit mindestens einer Expressionskassette umfasst, die ein Polynukleotid umfasst, das aus der Gruppe ausgewählt ist, die aus SEQ ID Nr. 7, 8, 9, 11, 12, 14, 15, 18, 19, 21, 22, 24, 25, 26, 27, 29, 30, 34, 35, 37, 38, 46 und 47 oder einem Polynukleotid besteht, das eine prozentuale Ähnlichkeit von mindestens 60% mit diesen Polynukleotidsequenzen zeigt, das einen homologen oder heterologen ABC-Transporter kodiert, und anschließendes Kultivieren des Mikroorganismus unter Bedingungen, welche die Produktion der β-Lactamverbindungen zulassen.

13. Isolierter ABC-Transporter, der von einem ABC-Transportergen kodiert wird, welches eine Nukleotidsequenz nach einer SEQ ID Nr. aufweist, die aus der Gruppe ausgewählt ist, welche aus SEQ ID Nr. 7, 8, 9, 11, 12, 14, 15, 18, 19, 21, 22, 24, 25, 26, 29, 30, 32, 33, 34, 37, 38, 46 und 47 besteht.

## Revendications

1. Procédé d'amplification de la sécrétion d'un composé de β-lactame provenant d'un micro-organisme, capable de produire des composés de β-lactame comprenant les étapes d'amplification d'une activité transporteur ABC dudit micro-organisme, qui comprend les étapes de transformation dudit micro-organisme avec au moins une cassette d'expression comprenant un polynucléotide choisi dans le groupe consistant en SEQ ID NO. 7, 8, 9, 11, 12, 14, 15, 18, 19, 21, 22, 24, 25, 26, 27, 29, 30, 34, 35, 37, 38, 46 et 47, ou un polynucléotide qui présente un pourcentage de similitude d'au moins 60 % avec ces séquences polynucléotidiques, codant pour un transporteur ABC homologue ou hétérologue.

2. Procédé selon la revendication 1, dans lequel ledit polynucléotide code pour un transporteur ABC qui comprend une séquence d'acides aminés choisie dans le groupe consistant en SEQ ID N0. 10, 13, 16, 20, 23, 28, 31, 36, 39 et 48, ou un polypeptide qui présente un pourcentage de similitude d'au moins 60 % avec ces séquences polypeptidiques.

3. Procédé selon la revendication 1 ou 2, dans lequel ledit micro-organisme est un champignon.

4. Procédé selon la revendication 3, dans lequel ledit champignon est choisi dans le groupe constitué de *Penicillium chrysogenum, Acremonium chrysogenum, Aspergillus nidulans, Saccharomyces cerevisiae, Hansenula polymorpha* et *Pichia pastoris.*

5. Procédé selon la revendication 1 ou 2, dans lequel ledit micro-organisme est une bactérie, de préférence un actinomycète, de manière même plus préférentielle, un actinomycète choisi dans le groupe constitué de *Streptomyces clavuligerus, Nocardia lactamdurans, Flavobacterium sp.*

6. Polynucléotide isolé codant pour une séquence d'acides aminés choisie dans le groupe consistant en SEQ ID NO. 10, 13, 16, 20, 23, 28, 31, 36, 39 et 48, ou une séquence qui présente un pourcentage de similitude d'au moins 95 % avec ces séquences d'acides aminés.

7. Polynucléotide isolé comprenant une séquence nucléotidique choisie dans le groupe consistant en SEQ ID NO. 7, 8, 9, 11, 12, 14, 15, 18, 19, 21, 22, 24, 25, 26, 27, 29, 30, 32, 33, 34, 35, 37, 38, 46 et 47, ou un polynucléotide qui présente une similitude d'au moins 95 % avec ces séquences polynucléotidiques.

8. Vecteur comprenant un polynucléotide selon la revendication 7.

9. Vecteur selon la revendication 8, dans lequel la séquence polynucléotidique code pour un transporteur ABC et dans lequel ledit polynucléotide est lié de manière fonctionnelle à des séquences de régulation appropriées pour l'expression du transporteur ABC dans une cellule hôte adéquate.

10. Cellule hôte transformée comprenant le vecteur selon la revendication 8 ou 9.

11. Utilisation d'un polynucléotide isolé selon la revendication 6 ou 7 et d'un vecteur selon la revendication 8 ou 9 dans un procédé d'amplification de la production de composés de β-lactame dans un micro-organisme capable de produire des composés de β-lactame.

12. Procédé d'amplification de la production de composés de β-lactame dans un micro-organisme capable de produire des composés de β-lactame comprenant les étapes d'amplification d'une activité d'un transporteur ABC dudit micro-organisme, qui comprend les étapes de transformation dudit micro-organisme avec au moins une cassette d'expression comprenant un polynucléotide choisi dans le groupe consistant en SEQ ID NO. 7, 8, 9, 11, 12, 14, 15, 18, 19, 21, 22, 24, 25, 26, 27, 29, 30, 34, 35, 37, 38, 46 et 47, ou un polynucléotide qui présente un pourcentage de similitude d'au moins 60 % avec ces séquences polynucléotidiques, codant pour un transporteur ABC homologue ou hétérologue, et ultérieurement une culture dudit micro-organisme dans des conditions permettant la production desdits composés de β-lactame.

13. Transporteur ABC isolé codé par un gène d'un transporteur ABC comprenant une séquence nucléotidique selon une SEQ ID NO. choisie dans le groupe consistant en SEQ ID NO. 7, 8, 9, 11, 12, 14, 15, 18, 19, 21, 22, 24, 25, 26, 29, 30, 32, 33, 34, 37, 38, 46 et 47.
